# EUROPEAN PATENT APPLICATION

(11) **EP 4 636 395 A1**
(43) Date of publication of application: **22.10.2025**
(21) Application number: 23903046.3
(22) Date of filing: 04.09.2023
(51) Int. Cl.: G01N 27/62, B03C 1/00, B03C 1/01, B03C 1/28

(54) **METHOD FOR PREPARING PEPTIDE-CONTAINING SAMPLE FOR MASS SPECTROMETRY, AND METHOD FOR SEPARATING NON-MAGNETIC SOLID CARRIER**

(30) Priority: 13.12.2022 JP 2022199020
(71) Applicant: Kazusa DNA Research Institute, Kisarazu-shi, Chiba 292-0818 (JP)
(72) Inventor: NAKAJIMA Daisuke, Kisarazu-shi Chiba 292-0818 (JP); KAWASHIMA Yusuke, Kisarazu-shi Chiba 292-0818 (JP); OHARA Osamu, Kisarazu-shi Chiba 292-0818 (JP)
(74) Representative: J A Kemp LLP
(86) International application number: PCT/JP2023/032297
(87) International publication number: WO 2024/127742

(57) **Abstract**

The present invention aims to provide a method for preparing a peptide-containing sample that enables identifying a greater number of trace proteins in proteome analysis of biological samples such as dried blood spots (DBS), and that allows for the automated processing of a large number of samples. By washing a fiber aggregate having a biological sample adsorbed thereon with an aqueous solvent, hydrophilic proteins contained in the biological sample are removed from the fiber aggregate. Residual proteins bound to the fiber aggregate are digested with a protease, and the fiber aggregate is removed from the digestion reaction mixture to obtain a peptide mixture. By allowing magnetic particles such as iron powder to be adhered to the fiber aggregate and magnetically adsorbing the fiber aggregate, operations such as recovery, washing, and transfer of the fiber aggregate having the biological sample adsorbed thereon are carried out.

The present invention also aims to provide a technique for easily and reliably separating non-magnetic solid-phase carriers dispersed and suspended in a liquid from the liquid. By agitating a liquid containing magnetic particles and a non-magnetic solid-phase carrier, the magnetic particles are allowed to be adhered to the non-magnetic solid-phase carrier. The non-magnetic solid-phase carrier having the magnetic particles adhered thereto is then magnetically adsorbed and separated from the liquid. When a non-magnetic solid-phase carrier having a porous structure is used, magnetic particles can enter the voids within the porous structure through agitation, thereby allowing the non-magnetic solid-phase carrier to be more reliably magnetically adsorbed. When a thickening agent is added to the liquid, the sedimentation rates of the non-magnetic solid-phase carrier and the magnetic particles become equally small, allowing the two to mix well in the liquid and the magnetic particles to be uniformly incorporated into the non-magnetic solid phase carriers, thereby ensuring more reliable adsorption to the magnet.

## Description

### TECHNICAL FIELD

The present invention relates to a method for preparing a peptide-containing sample for identifying proteins contained in a biological sample by mass spectrometry. The present invention also relates to a method for separating non-magnetic solid-phase carriers dispersed and suspended in a liquid from the liquid.

### BACKGROUND ART

Early detection of hereditary diseases is important for the prevention of onset and the alleviation of symptoms of the diseases. Newborn screening is widely performed to detect diseases in newborns, and dried blood spots (DBS), which are low-invasive and excellent in storability and transportability, are widely used therein. Conventional newborn screening targeting specific proteins such as metabolites and enzymes in DBS involves different detection methods for each protein, and thus has a problem in that an increase in the number of test items leads to an increase in labor, cost and time. On the other hand, non-targeted DBS proteome analysis enables the simultaneous and collective detection of a large number of disease-causing proteins by a single analysis, and is considered capable of realizing rapid and low-cost newborn screening. Meanwhile, screening by genome-wide sequencing has recently attracted attention; however, it remains expensive, and DNA sequence information easily identifies individuals, raising ethical issues. In contrast, newborn screening by comprehensive protein detection holds the potential to realize low-cost, rapid, and comprehensive disease screening. Furthermore, proteome information does not directly lead to personal identification as genome information does, and therefore, is considered to pose fewer ethical problems.

In DBS proteome analysis, proteins are generally first extracted directly from DBS, subjected to reductive alkylation treatment as necessary, enzymatic digestion, and then analyzed using a liquid chromatography-mass spectrometry (LC-MS). However, there is a problem in that highly abundant hydrophilic proteins such as hemoglobin, albumin, and globulin contained in large quantities in DBS hinder the detection of trace proteins. Therefore, in the pretreatment step, it is necessary to remove highly abundant proteins as much as possible and increase the relative proportion of trace proteins. However, there is a dilemma in that the removal of highly abundant proteins may instead result in the loss of coexisting trace proteins. Generally, these highly abundant proteins are specifically adsorbed and removed using antibody columns in which antibodies against those proteins are immobilized. However, commercially available antibody columns are very expensive, costing 6000 to 7000 yen per column, and are not suitable for newborn screening, which requires cost reduction. By using the present invention, it is possible to remove highly abundant proteins simply using commonly-used inexpensive reagents, and to detect trace proteins.

To date, many groups have attempted to increase the number of proteins and peptides identified in non-targeted proteome analysis of DBS. In Non-Patent Document 1, proteins in DBS were extracted by treating the surface of DBS with 50 mM aqueous ammonium bicarbonate solution, and the extracted product was subjected to trypsin treatment followed by LC-MS/MS analysis with data-dependent acquisition (DDA), whereby 120 proteins were identified.

In Non-Patent Document 2, proteins were extracted from DBS with 25 mM ammonium bicarbonate, 1% sodium deoxycholate, and 5 mM tris(2-carboxyethyl)phosphine hydrochloride, and the resulting protein extract solution was alkylated with iodoacetamide, treated with trypsin, and subjected to LC-MS/MS analysis with DDA to identify 253 proteins.

In Non-Patent Document 3, proteins were extracted from DBS with 50 mM ammonium bicarbonate buffer (ABC buffer), and the extract was treated with trypsin and analyzed by LC-MS/MS using DDA combined with field asymmetric ion mobility spectrometry (FAIMS), whereby approximately 350 proteins were identified.

In Non-Patent Document 4, solid-liquid phase extraction was performed on DBS discs with 50 mM ammonium bicarbonate buffer and 2% sodium deoxycholate, and the liquid phase was recovered, alkylated with iodoacetamide, treated with trypsin, and analyzed by LC-MS/MS with DDA, whereby 295 proteins were identified.

On the other hand, Nieman et al. treated DBS samples with 6 M urea, 50 mM ammonium bicarbonate, and 0.1 mM dithiothreitol to redissolve proteins, added 0.1 mM iodoacetamide for alkylation, treated the resulting solution with trypsin, and analyzed it by LC-MS/MS using data-independent acquisition (DIA), successfully identifying 712 proteins (Non-Patent Document 5).

To overcome the effects of heterogeneity of hematocrit values and samples, van den Broek et al. utilized volumetric absorptive microsampling (VAMS) instead of DBS and extracted proteins from the samples with 2% w/v octyl-β-glucopyranoside, 8.3 mM Tris(2-carboxyethyl)phosphine, and 73.3 mM Tris, 2.9 mM calcium chloride buffer (pH 8.5), alkylated the extract with 200 mmol/L MMTS, treated it with trypsin, and analyzed it by LC-MS/MS with data-independent acquisition (DIA), whereby 423 proteins were identified (Non-Patent Document 6).

The present inventors succeeded in increasing the number of identified proteins to more than 2000 at once, including 500 disease-causing proteins, in a single-shot LC-MS analysis by introducing DIA-MS in combination with simple enrichment of hydrophobic proteins from DBS by sodium carbonate precipitation (SCP) (Non-Patent Document 7). In this method, DBS is crushed in 100 mM aqueous sodium carbonate solution to extract proteins adsorbed on the filter paper into the aqueous sodium carbonate solution. The mixture is centrifuged at 3000 × g to precipitate the insoluble filter paper, and the supernatant is recovered while the filter paper is removed. Next, the obtained supernatant is centrifuged at 17,400 × g to precipitate poorly soluble proteins. The precipitate is washed with 100 mM aqueous sodium carbonate solution, centrifuged at 17,400 × g to precipitate again, and the supernatant is removed to isolate SCP. This SCP is subjected to reductive alkylation treatment, trypsin digestion, and LC-MS/MS analysis. However, this method has the drawback that proteins extracted in the extraction step are difficult to visually recognize, and there is a high possibility that the small and slippery precipitates of hydrophobic proteins may be lost. Therefore, this series of operations must be performed with great care by well-trained operators. As such, this method has low compatibility with high-throughput treatment and is difficult to apply to newborn screening, which requires rapid processing of large numbers of samples.

On the other hand, when washing a solid-phase carrier containing an analytical sample to remove unwanted substances such as contaminants, or when removing the solid-phase carrier after extracting target analytes such as proteins or metabolites from the solid-phase carrier, it is generally necessary to add a desired solution to the solid-phase carrier, agitate the mixture, centrifuge it to precipitate the solid-phase carrier, and then remove or recover the supernatant. When centrifugation is used to recover the solid-phase carrier in this manner, care must be taken to proceed the operations during the removal or recovery of the supernatant because the precipitated solid-phase carrier may be unintentionally aspirated or may resuspend due to vibrations during the recovery operation. Therefore, operations become complicated when processing a large number of samples, and automated processing is also difficult. Although it is possible to directly pick up the solid-phase carrier in the vessel with tweezers or the like, this involves the risk of sample contamination by the tweezers and is also troublesome, making it unsuitable for processing a large number of samples.

When magnetic beads are used as the solid-phase carrier, operations such as recovery, washing, and separation of the solid-phase carrier can be automated by combining them with an automated device equipped with magnetic rods, such as Maelstrom 8 Autostage (MS8) (manufactured by Taiwan Advanced Nanotech). However, the samples that can be analyzed using commercially available magnetic beads are limited, and when using non-magnetic solid-phase carriers such as filter paper, gel pieces, or cotton, troublesome operations are still required.

Patent Documents 1 and 2 disclose a method for removing non-magnetic suspended products (colloidal product) in a liquid by magnetically aggregating it together with magnetic suspended product. In this method, a magnetic field is formed by placing the magnetic poles of multiple magnets in an opposing pole configuration on two rotating disks provided in a magnetic aggregation processing tank, and a liquid containing impurities is passed through this field. As a result, the impurities are magnetically aggregated with the magnetic suspended products serving as a nucleus, in a state where the non-magnetic suspended products are captured, and the resulting magnetic aggregates are adsorbed onto the magnets. This technique is used, for example, to remove fine impurities (colloidal products), such as magnetic suspended products including chips and metal powders, and non-magnetic suspended products such as carbon, sludge, and oil, contained in oily or aqueous working fluids used during electrical discharge machining.

### PRIOR ART DOCUMENTS

### PATENT DOCUMENTS

Patent Document 1: JPH06-71195A
Patent document 2: JPH09-248483A

### NON-PATENT DOCUMENTS

Non-Patent Document 1: Martin et al., Journal of the American Society for Mass Spectrometry, 24(8), 1242-1249, 2013
Non-Patent Document 2: Chambers et al., Journal of the American Society for Mass Spectrometry, 24(9), 1338-1345, 2013
Non-Patent Document 3: Rosting et al, Journal of Proteome Research, 17(6), 1997-2004, 2018
Non-Patent Document 4: Eshghi et al, Molecular and Cellular Proteomics, 19(3), 540-553, 2020
Non-Patent Document 5: Nieman et al, Proteomes, 8(1), 4, 2020
Non-Patent Document 6: Van Den Broek et al., Clinical Mass Spectrometry, 4-5, 25-33, 2017
Non-Patent Document 7: Nakajima et al., Journal of Proteome Research, 19(7), 2821-2827

### SUMMARY OF THE INVENTION

### PROBLEMS TO BE SOLVED BY THE INVENTION

The present invention aims to provide a method for preparing a peptide-containing sample that enables identifying a greater number of trace proteins by inexpensively and rapidly removing highly abundant proteins from blood in proteome analysis, and that allows automated processing of a large number of samples. The present invention also aims to provide a technique for easily and reliably separating non-magnetic solid-phase carriers dispersed and suspended in a liquid from the liquid.

### MEANS TO SOLVE THE PROBLEMS

The present inventors have conducted intensive studies to solve the above problems, and found that in all conventional DBS proteome analyses, proteins adsorbed on the filter paper were extracted into an aqueous solution, the proteins in the obtained extract solution were treated with enzymes or the like, and analyzed by LC-MS/MS, while the filter paper was discarded. (Non-Patent Documents 1 to 7). The present inventors focused on the filter paper residue that had conventionally been discarded after protein extraction, and analyzed the residual proteins contained therein. Surprisingly, they found that despite having undergone protein extraction, the filter paper residue still retained many proteins including trace proteins in an adsorbed state. After washing the filter paper residue, the proteins adsorbed on the filter paper were subjected to trypsin digestion while remaining in the adsorbed state, and the resulting peptide fragments were analyzed by LC-MS/MS, whereby more than 4000 species of proteins were successfully identified. This number greatly exceeded the number of proteins identified by the conventional methods in which the protein extract solution from filter paper is analyzed by LC-MS. Through the washing operation of the filter paper, hydrophilic proteins such as albumin, which are abundantly contained in DBS and interfere with the detection of trace proteins, were appropriately washed away, whereas many of the trace proteins remained adsorbed on the filter paper and were retained, leading to the identification of a large number of proteins.

**In** addition, it was found that when magnetic particles such as iron powder were suspended and agitated in a liquid containing non-magnetic solid-phase carriers, and a magnet was then brought close, the magnetic particles adhered to the non-magnetic solid-phase carriers, and the non-magnetic solid-phase carriers with the adhered magnetic particles was adsorbed onto the magnet, thereby successfully separating the non-magnetic solid-phase carrier from the liquid in a magnetism-dependent manner. As a result, it became possible to wash and recover or remove the non-magnetic solid-phase carriers without using centrifugation or the like. When non-magnetic solid-phase carriers having a porous structure were used, the magnetic particles entered the voids within the porous structure upon agitation, thereby allowing the non-magnetic solid-phase carriers to tightly capture the magnetic particles and enabling more reliable adsorption and recovery of the non-magnetic solid-phase carriers by the magnet. In particular, when a fiber aggregate such as filter paper was used as the non-magnetic solid-phase carrier, the fibers loosened and the voids between the fibers expanded upon agitation, allowing a large number of magnetic particles to be incorporated into the non-magnetic solid-phase carrier, thereby enhancing adsorption to the magnet. When a thickening agent was added to the liquid, the sedimentation rate of the non-magnetic solid-phase carrier and the magnetic particles became equally small, allowing the two to mix well in the liquid and the magnetic particles to be uniformly incorporated into the non-magnetic solid phase carriers, thereby ensuring more reliable adsorption to the magnet. As a result, it became possible to minimize the risk of recovery errors of the non-magnetic solid-phase carrier during the operation and to perform automated processing of a large number of non-magnetic solid-phase carrier samples.

Accordingly, the present inventors applied the above-described method for separating non-magnetic solid-phase carriers using magnetic particles to the method for extracting filter paper-bound proteins, with the aim of adapting this method to disease screening, in which it is required to process a large number of samples as quickly and accurately as possible. To this end, the present inventors aimed to establish an automated extraction method for the filter paper-bound proteins using an automatic nucleic acid extraction device. When iron powder was added during the crush of the filter paper in solution, the iron powder entered the space between the fibers of the swollen, pulp-like filter paper, forming a "composite" of the filter paper and the iron powder, which could then be easily recovered using a magnet. This method successfully enabled the automation of DBS crush, composite formation, recovery, washing, and buffer replacement. The finally obtained filter paper was suspended in a buffer for trypsin digestion, and the proteins bound to the filter paper were digested with trypsin to generate peptides, which were then analyzed by DIA-MS for proteome analysis. As a result, a total of 5817 proteins were identified, which greatly exceeded the numbers identified by conventional methods. Furthermore, comparison with the OMIM database revealed that 1895 disease-related proteins were included, indicating that the method has the potential to be used in disease screening far more extensively than conventional methods. In addition, investigation of the reproducibility across days and between analytical instruments showed a high Pearson correlation coefficient (r) of 0.96 or higher, demonstrating that the method is sufficiently applicable to the testing of daily-delivered samples. Based on these findings, the present inventors conducted further studies and completed the present invention.

Accordingly, the present invention relates to the following:
[1] A method for preparing a peptide-containing sample for identifying, by mass spectrometry, proteins contained in a biological sample containing proteins from a fiber aggregate having the biological sample containing proteins adsorbed thereon, the method comprising the following steps:
   1) washing the fiber aggregate having the biological sample containing proteins adsorbed thereon with an aqueous solvent to remove hydrophilic proteins contained in the biological sample from the fiber aggregate, and recovering the fiber aggregate having residual proteins bound thereto;
   2) treating the recovered fiber aggregate having residual proteins bound thereto with a protease to digest the residual proteins bound to the fiber aggregate with the protease; and
   3) removing the fiber aggregate from the digestion reaction mixture to recover a peptide mixture as a digestion product.
[2] The preparation method of [1], wherein the fiber aggregate is a cellulose fiber aggregate.
[3] The preparation method of [2], wherein the cellulose fiber aggregate is a filter paper.
[4] The preparation method of any of [1] to [3], wherein the fiber aggregate having the biological sample containing proteins adsorbed thereon is a dried blood spot.
[5] The preparation method of any of [1] to [4], further comprising subjecting the fiber aggregate having residual proteins bound thereto to a reductive alkylation treatment.
[6] A method for separating non-magnetic solid-phase carriers from a liquid containing the non-magnetic solid-phase carriers, the method comprising the following steps:
   1) providing a liquid containing magnetic particles and a non-magnetic solid-phase carrier;
   2) stirring the liquid containing the magnetic particles and the non-magnetic solid-phase carriers to allow the magnetic particles to adhere to the non-magnetic solid-phase carrier;
   3) bringing a magnet close to the liquid containing the magnetic particles and the non-magnetic solid-phase carriers to magnetically adsorb the non-magnetic solid-phase carrier to which the magnetic particles have adhered; and
   4) separating the magnetically adsorbed non-magnetic solid-phase carrier from the liquid.
[7] The method of [6], wherein the non-magnetic solid-phase carrier has a porous structure and the magnetic particles enter the voids in the porous structure by agitation.
[8] The method of [7], wherein the non-magnetic solid-phase carrier comprises a fiber aggregate.
[9] The method of any of [6] to [8], wherein the non-magnetic solid phase carrier is sheet-shaped.
[10] The method of any of [6] to [9], wherein the non-magnetic solid phase carrier is any one selected from the group consisting of paper, gel, fabric, resin, cotton and swab.
[11] The method of any of [6] to [10], wherein the liquid contains a thickening agent.
[12] The method of any of [6] to [11], wherein the magnetic particles are ferromagnetic metal particles or ferromagnetic metal oxide particles.
[13] The method of [12], wherein the magnetic particles are an iron powder.
[14] The method of any of [6] to [13], wherein the average particle diameter of the magnetic particles is 150 µm or less.
[15] The method of any of [6] to [14], wherein 0.3 mg or more of magnetic particles are dispersed per 1 ml of liquid.
[16] The method of any of [6] to [15], wherein 0.2 mg or more of magnetic particles are dispersed in a liquid for 1 mg of non-magnetic solid phase carriers.
[17] The preparation method of [1], further comprising agitating the magnetic particles and the fiber aggregate having the biological sample containing protein adsorbed thereon in an aqueous solvent to allow the magnetic particles to adhere to the fibrous aggregate.
[18] The preparation method of [17], wherein the aqueous solvent used in Step 1 comprises magnetic particles, and Step 1 is performed by agitating, in the aqueous solvent containing the magnetic particles, the fiber aggregate having the biological sample containing proteins adsorbed thereon to wash the fiber aggregate with the aqueous solvent to remove hydrophilic proteins contained in the biological sample from the fiber aggregate and allow the magnetic particles to adhere to the fiber aggregate, thereby recovering a fiber aggregate having residual proteins bound thereto and the magnetic particles adhered thereto.
[19] The preparation method of [17] or [18], wherein the fiber aggregate having residual proteins bound thereto is recovered by magnetic adsorption in Step 1.
[20] The preparation method of any of [17] to [19], wherein the fiber aggregate is removed from the digestion reaction mixture by magnetic adsorption in Step 3.
[21] The preparation method of any of [17] to [20], wherein the magnetic particles are iron powder.
[22] The preparation method of any of [17] to [21], wherein the aqueous solvent comprises a thickening agent.
[23] The preparation method of [22], wherein the thickening agent is glycerol.

### EFFECTS OF THE INVENTION

According to the present invention, a peptide-containing sample in which the content of peptides derived from hydrophilic proteins such as albumin that are abundantly present in the biological sample is suppressed, and which is enriched in peptides derived from trace proteins can be prepared from a fiber aggregate having the biological sample adsorbed thereon in a simple manner by using commonly-used inexpensive reagents. By subjecting the peptide-containing sample obtained by the preparation method of the present invention to LC-MS analysis, it becomes possible to identify a large number of proteins including trace proteins.

According to the present invention, it becomes possible to separate a non-magnetic solid-phase carrier from a liquid in a magnetism-dependent manner. Therefore, the non-magnetic solid-phase carrier can be washed and recovered or removed without the use of centrifugation or the like. Conventionally, when performing washing, extraction, or other processing serially on a non-magnetic solid-phase carrier using a liquid, the non-magnetic solid-phase carrier is typically left inside a vessel and precipitated by centrifugation, followed by addition and removal of the liquid for washing or extraction. In such procedures, the precipitated non-magnetic solid-phase carrier may be unintentionally aspirated when the liquid is removed or recovered from the container, or the precipitate may float, resulting in loss of the non-magnetic solid-phase carrier. In contrast, in the method of the present invention, the non-magnetic solid-phase carrier is removed from the vessel while leaving the liquid for washing or extraction, rather than the non-magnetic solid-phase carrier, in the vessel. Therefore, the non-magnetic solid-phase carrier can be subjected to serial processing with a liquid such as washing or extraction while minimizing the loss of the non-magnetic solid-phase carrier. Since automated devices for handling magnetic solid-phase carriers such as magnetic beads are already widely available, combining the present invention with such devices enables automated processing of large amount of non-magnetic solid-phase carriers, which has been difficult to achieve by conventional means.

Furthermore, by applying this method for separating the non-magnetic solid-phase carrier using magnetic particles to the preparation of a peptide-containing sample from a fiber aggregate having the biological sample adsorbed thereon and allowing the magnetic particles to adhere to the fiber aggregate, operations such as washing, recovery, and buffer replacement of the fiber aggregate having the biological sample adsorbed thereon can be conveniently performed by magnetic adsorption. In addition, by employing automated processing using an automated nucleic acid extraction device and the like, a large number of samples can be processed in a short period of time, and the reproducibility of the results can be improved, thereby enabling application to mass screening for congenital diseases in newborns using dried blood spots.

### BRIEF DESCRIPTION OF THE DRAWINGS

[Figure 1] Figure 1 shows flowcharts illustrating the preparation of insoluble proteins from sodium carbonate precipitates (A) and the preparation of filter paper-bound proteins from DBS (B).
[Figure 2] Figure 2 shows the optimization of the m/z range in DIA-MS for FPBP proteome analysis. The bar graph indicates the number of proteins (left) and peptides (right) detected in each m/z range. The left y-axis represents the number of proteins, and the right y-axis represents the number of peptides.
[Figure 3] Figure 3 shows a Venn diagram of the number of proteins isolated by the SCP method and FPBP method. "SCP" refers to sodium carbonate precipitation and "FPBP" refers to filter paper-bound proteins.
[Figure 4] A schematic diagram illustrating the automated isolation process of FPBP using the Maelstrom 8 Autostage / Maelstrom 9610.
[Figure 5A] Figure 5A shows a Venn diagram illustrating the number of proteins identified by two different preparation methods. "FPBP" refers to the manual protein preparation method using filter paper-bound proteins, and "Auto-FPBP" refers to the automated protein preparation method using filter paper-bound proteins.
[Figure. 5B] Figure 5B is a bar graph showing the total intensities of highly abundant proteins in blood prepared by the manual or automated FPBP method. The highly abundant proteins included HBB, HBA2, IGHG1, APOA1, and ALB.
[Figure 5C] Figure 5C is a graph showing violin plots of the coefficients of variation of protein quantification values obtained by the FPBP and Auto-FPBP methods. The y-axis represents CV (%).
[Figure 6A] Figure 6A is a graph comparing the number of proteins identified by three different preparation methods. "SCP" refers to the protein preparation method using sodium carbonate precipitates, "FPBP" refers to the manual protein preparation method using filter paper-bound proteins, and "Auto-FPBP" refers to the automated protein preparation method using filter paper-bound proteins. From top to bottom, the numbers of plasma-derived proteins, blood cell-derived proteins, and other proteins are shown.
[Figure 6B] Figure 6B is a graph showing the number of membrane proteins identified by three different preparation methods.
[Figure 6C] Figure 6C is a graph showing the number of proteins hit in OMIM contained in the proteins identified by the three different preparation methods.
[Figure 7] Figure 7 is a graph showing the evaluation of inter-LC-MS instrument and inter-day reproducibility in automated protein preparation using filter paper-bound proteins. Protein intensities of samples 1-12, 13-24, and 25-36 prepared by the Auto-FPBP method on days 0, 32, and 60, respectively, were measured by LC-MS instruments A and B, and Pearson correlation analysis was performed.
[Figure 8] Figure 8 is a schematic diagram comparing the workflow of the pretreatment of filter paper-bound proteins for conducting proteome analysis between a conventional method and the separation method of the present invention (improved method).
[Figure 9] Figure 9 shows the magnetic adsorption of filter paper and gel pieces in TBST, mediated by the magnetic force between iron powder suspended in TBST and a magnet.
[Figure 10] Figure 10 shows the state in which DBS and iron powder are added to TBST and agitated with MS8.

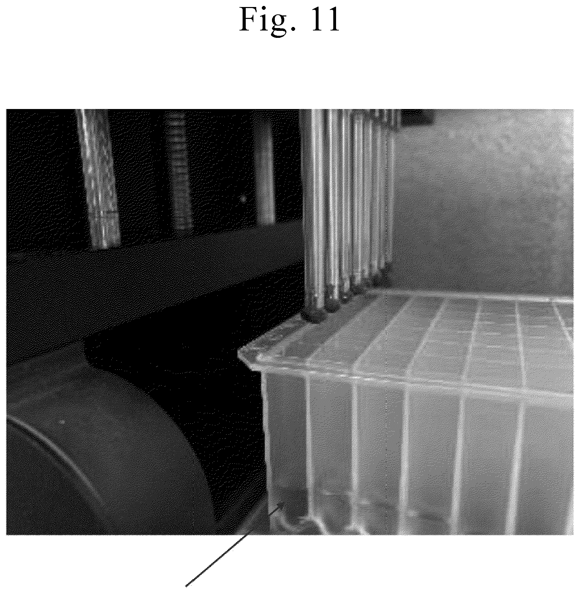

[Figure 11] Figure 11 shows the recovery of DBS in the liquid phase by magnetic force between iron powder suspended in the liquid phase and a magnet equipped in MS8.

### MODE FOR CARRYING OUT THE INVENTION

### 1. Preparation method of a peptide-containing sample

The present invention provides a method for preparing a peptide-containing sample (hereinafter referred to as "the preparation method of the present invention") for identifying, by mass spectrometry, proteins contained in a biological sample containing proteins from a fiber aggregate having the biological sample containing proteins adsorbed thereon, the method comprising the following steps:
1) washing the fiber aggregate having the biological sample containing proteins adsorbed thereon with an aqueous solvent to remove hydrophilic proteins contained in the biological sample from the fiber aggregate, and recovering the fiber aggregate having residual proteins bound thereto;
2) treating the recovered fiber aggregate having residual proteins bound thereto with a protease to digest the residual proteins bound to the fiber aggregate with the protease; and
3) removing the fiber aggregate from the digestion reaction mixture to recover a peptide mixture as a digestion product.

In conventional preparation methods, proteins adsorbed onto a fiber aggregate, including abundant hydrophilic proteins, were extracted into an aqueous solution, and the extracted proteins were then subjected to enzymatic treatment or the like and analyzed by LC-MS/MS, and the fiber aggregate was discarded (Non-Patent Documents 1 to 7). The present inventors focused on the fiber aggregate residue that had been discarded, and upon analyzing the residual proteins therein, unexpectedly found that, despite the protein extraction operation, a large number of proteins, including trace proteins, remained adsorbed on the fiber aggregate residue. The present invention was completed based on these findings.

Examples of the biological sample used in the present invention include, but are not limited to, body fluids such as blood (whole blood, serum, plasma), saliva, urine, sweat, lymph fluid, sputum, tear fluid, nasal mucous, semen, cerebrospinal fluid, pleural fluid, ascites, synovial fluid, pericardial fluid, and tissue fluid; cells; tissues or portions thereof; cell or tissue lysates; cell or tissue extracts; biopsy samples; swab samples; feces; cell culture products; bacteria; viruses; and fungi. Biological samples may be extracts or concentrates of a specific biological component fraction. The biological sample typically contains proteins.

Fiber aggregate refers to a structure in which fibers are overlaid in three dimensions, regardless of whether the fibers are entangled with each other. Examples of fiber aggregates include, but are not limited to, cotton-like, paper-like, fabric-like, or felt-like structures. The types of fibers constituting the fiber aggregate are not particularly limited, and examples thereof include natural fibers such as cellulose, keratin, silk, chitin, and hemicellulose, and synthetic fibers such as nylon, polyester, acrylic, and rayon. The fibers constituting the fiber aggregate are preferably cellulose. That is, the fiber aggregate is preferably a cellulose fiber aggregate. The cellulose may be either modified or unmodified. Examples of modifications include, but are not limited to, nitration, esterification (e.g., nitrate esterification, sulfate esterification), amination, acetylation, and methoxylation. The cellulose is preferably an unmodified cellulose. Cellulose is composed of hydrophilic β-glucose units linearly polymerized via β-1,4 glycosidic linkages and exhibits hydrophobic properties, thereby becoming insoluble. Although not intended to be bound by theory, cellulose fibers, having both hydrophilic and hydrophobic regions, are capable of adsorbing proteins with a wide range of properties. Examples of cellulose fiber aggregates include, but are not limited to, paper (e.g., filter paper), cotton, cotton thread, and cotton fabric. The cellulose fiber aggregate is preferably filter paper.

The fiber aggregate may be contained in an aqueous solvent in a suspended, dispersed, or floated state. The size (maximum diameter) of the fiber aggregate is not particularly limited as long as the fiber aggregate can be suspended, dispersed, or floated in the aqueous solvent in the vessel in which the preparation method of the present invention is carried out. Preferably, the size (maximum diameter) of the fiber aggregate is smaller than the inner diameter of the opening of the vessel (well) used in the preparation method of the present invention, so as to facilitate the separation of the fiber aggregate from the aqueous solvent. The size (maximum diameter) of the fiber aggregate is typically 100 µm or more (e.g., 500 µm or more, 1 mm or more, 3 mm or more, or 5 mm or more).

In one embodiment, the fiber aggregate having a biological sample adsorbed thereon is a dried blood spot (DBS). A dried blood spot refers to a sample obtained by allowing collected blood to be absorbed into filter paper and drying it, and is used, for example, in newborn screening or doping tests for athletes. A DBS is typically prepared by dropping one to two drops (approximately 20 to 40 microliters) of human blood onto filter paper to form a circular spot approximately 1 cm in diameter, followed by natural drying at room temperature. In one embodiment, a punched-out disk with a diameter of 3.2 mm (corresponding to approximately 3 microliters of blood) from a DBS is used in the preparation method of the present invention. As used herein, a dried blood spot may also be referred to as dried filter paper blood.

In the preparation method of the present invention, the fiber aggregate having the biological sample adsorbed thereon is first washed with an aqueous solvent. By this washing operation, hydrophilic proteins contained in the biological sample are removed from the fiber aggregate. Despite the washing operation, various proteins, including trace proteins present in the biological sample, remain bound to the fiber aggregate. Therefore, the fiber aggregate having residual proteins bound thereto is recovered and used for sample preparation for mass spectrometry. Many biological samples contain abundant hydrophilic proteins such as albumin, γ-globulin, β-globin, hemoglobin A2, immunoglobulin, and apolipoprotein A. When such a biological sample is directly analyzed by LC-MS, the signals derived from these highly abundant hydrophilic proteins may mask the signals of trace proteins, making it difficult to identify the trace proteins. In the preparation method of the present invention, the hydrophilic proteins abundantly present in the biological sample are eluted and removed into the aqueous solvent during the washing operation. Therefore, by subjecting the residual proteins bound to the fiber aggregate to LC-MS analysis, masking of trace-protein signals by the hydrophilic proteins is suppressed, enabling trace proteins to be detected and identified with high sensitivity.

In the preparation method of the present invention, it is preferable to add an appropriate buffering agent to the aqueous solvent used for washing in order to maintain the pH. Examples of buffering agents include, but are not limited to, Tris-HCl, phosphate salts, citrate salts, acetate salts, HEPES, borate salts, tartrate salts and the like. An inorganic salt may also be added to the aqueous solvent used for washing for purposes such as suppressing the effects of salt concentration in the biological sample. Examples of inorganic salts include, but are not limited to, sodium chloride, potassium chloride, calcium chloride, magnesium sulfate, ammonium sulfate and the like. To promote the dissolution of hydrophilic proteins, a surfactant may also be added to the aqueous solvent used for washing. Examples of surfactants include, but are not limited to, nonionic surfactants such as Tween-20, Tween-80, Triton X-100, Nonidet P-40 and the like; ionic surfactants such as sodium dodecyl sulfate, sodium deoxycholate and the like; and zwitterionic surfactants such as CHAPS and the like. A chelating agent may also be added to the aqueous solvent used for washing. Examples of chelating agents include, but are not limited to, EDTA, EGTA, DTPA, HEDTA and the like. Only one species may be used alone for each of the buffering agents, inorganic salts, surfactants, and chelating agents or several species may be used in combination. The pH of the aqueous solvent used for washing is typically from 5.5 to 9.5, preferably from 6.5 to 9.0, and more preferably from 7.0 to 8.5. Examples of preferred aqueous solvents for washing include, but are not limited to, physiological saline added with a buffering agent and a nonionic surfactant, such as TBS-Tween (physiological saline containing Tris-HCl and Tween-20) at pH 8.0.

The washing of a fiber aggregate having a biological sample adsorbed thereon with an aqueous solvent can be performed, for example, by placing the fiber aggregate having a biological sample adsorbed thereon and an aqueous solvent into a suitable tube, agitating the mixture, and then centrifuging it to remove the supernatant containing hydrophilic proteins dissolved from the biological sample, thereby recovering the precipitate of the fiber aggregate having residual proteins bound thereto. The washing operation may be repeated multiple times, and different types of aqueous solvents may also be used for washing.

To cleave inter- and intramolecular disulfide bonds between cysteine residues in proteins and prevent disulfide bond formation, the fiber aggregate having residual proteins bound thereto may be subjected to a reductive alkylation treatment. The reduction of the residual proteins may be carried out by heating the fiber aggregate having residual proteins bound thereto with a reducing agent such as 1,4-dithiothreitol (DTT), 2-mercaptoethanol, or tris(2-carboxyethyl)phosphine (TCEP). The alkylation of the residual proteins may be carried out by reacting the reduced fiber aggregate having residual proteins bound thereto with an alkylating agent such as iodoacetamide, iodoacetic acid, acrylamide, chloroacetamide and the like. To promote the reductive alkylation, a chaotropic agent may be added during the reduction step and/or the alkylation step. Examples of chaotropic agents include, but are not limited to, urea, thiourea, guanidine hydrochloride, thiocyanate, sarcosine and the like. After the reductive alkylation reaction, the fiber aggregate having residual proteins bound thereto may be washed with an aqueous solvent to remove unreacted reducing agents and alkylating agents. The aqueous solvent used for this washing may be the same as that described for removing hydrophilic proteins from the fiber aggregate. It is noted that the reductive alkylation treatment is an optional process and may be omitted when rapidness and labor-saving operation are prioritized.

Next, the fiber aggregate having residual proteins (including residual proteins subjected to reductive alkylation) bound thereto is treated with a protease to digest the residual proteins bound to the fiber aggregate. Specifically, the fiber aggregate having residual proteins bound thereto is incubated in an aqueous solvent containing a protease. Examples of proteases include, but are not limited to, endoproteases such as trypsin, Glu-C, Lys-N, Lys-C, Asp-N, chymotrypsin and the like. Several proteases may be used in combination. The aqueous solvent used may be the same as that described above for removing hydrophilic proteins from the fiber aggregate. A preferred aqueous solvent includes, but are not particularly limited to, an aqueous buffer solution, such as Tris-HCl buffer at pH 8.0.

By the treatment with the protease, the residual proteins bound to the fiber aggregate are digested, and the resulting peptide digestion products are released into the aqueous solvent from the fiber aggregate.

Then, the fiber aggregate is removed from the digestion reaction mixture to recover the peptide mixture as the digestion product, which is used as a peptide-containing sample for identifying proteins contained in the biological sample by mass spectrometry. Specifically, for example, the digestion reaction mixture comprising the aqueous solvent containing the peptide mixture as the digestion product and the fiber aggregate is centrifuged to precipitate the fiber aggregate, and the aqueous solvent containing the peptide mixture, the digestion product of the supernatant, is recovered.

To analyze the recovered aqueous solvent containing the peptide mixture by LC-MS, the sample is typically subjected to desalting to remove low-molecular-weight substances such as salts, buffering agents, and chaotropic agents, thereby purifying the peptide mixture. The desalting treatment can be carried out by applying the aqueous solvent containing the peptide mixture to a reversed-phase column. Examples of reversed-phase columns include, but are not limited to, columns in which hydrophobic groups such as octadecyl group, (C18) octyl group (C8), butyl group (C3), phenyl group, cyanopropyl group and the like are bonded to a solid-phase carrier (e.g., silica gel), and columns composed of styrenedivinylbenzene (SDB) copolymers. Preferably, the reversed-phase column is a C18 column, in which an octadecyl group (C18) is bonded to a solid-phase carrier (e.g., silica gel), or an SDB copolymer column. Commercially available reversed-phase columns may be used. Examples include commercially available kits such as EVOSEP ONE, SDB-STAGE tips and the like.

When the aqueous solvent containing the peptide mixture is applied to a reversed-phase column, the peptide mixture is adsorbed onto the reversed-phase column, while low-molecular-weight substances such as salts, buffering agents, and chaotropic agents do not bind to the column and are eluted and removed. Subsequently, the peptide mixture adsorbed onto the reversed-phase column can be eluted to obtain a purified peptide mixture. The solvent used for elution of peptides is typically a mixed solvent of an organic solvent and water. Examples of organic solvents include acetonitrile, methanol, tetrahydrofuran, isopropanol, acetone and the like. To promote protonation, it is preferable to add an acid such as trifluoroacetic acid, formic acid, acetic acid, hydrochloric acid and the like to the water.

The eluted peptide mixture is dried to remove the solvent. The drying of the peptide mixture can be carried out, for example, using a centrifugal evaporator.

The resulting dried peptide mixture is redissolved in water. To promote protonation, an acid such as formic acid, trifluoroacetic acid, acetic acid, hydrochloric acid and the like may be added to the water. An organic solvent such as acetonitrile, methanol, tetrahydrofuran, isopropanol, acetone and the like may also be added to the water. For example, the dried peptide mixture may be dissolved in water containing 0.1% (v/v) formic acid to obtain an aqueous solution of the peptide mixture.

The resulting aqueous solution of the peptide mixture is then subjected to mass spectrometry. Based on the obtained mass spectrometry data, the peptides contained in the peptide mixture are identified, and based on the information on the identified peptides, the proteins present in the biological sample from which the peptides are derived are determined. The mass spectrometric analysis of the peptide mixture is typically carried out using liquid chromatography-mass spectrometry (LC-MS), and preferably using tandem mass spectrometry (LC-MS/MS) coupled online with liquid chromatography. The measurement mode may be appropriately selected depending on the analytical purpose. For comprehensive identification of proteins contained in the biological sample by non-targeted proteomics, measurement modes such as data dependent acquisition (DDA), data independent acquisition (DIA) or the like may be selected. For the identification and quantification of specific proteins by targeted proteomics, measurement modes such as selected or multiple reaction monitoring (S/MRM) or the like may be selected.

In DDA, multiple number of precursor ions with strong signal intensities are selected in descending order of intensity, and product ion spectra are automatically acquired. From the obtained data, a file (peak list) linking the product ion spectra to the peaks on the MS spectrum is generated and subjected to a database search. In the database search, possible peptide sequences obtained by enzymatic digestion of all protein sequences registered in an arbitrary sequence database are theoretically predicted, and the peptide sequence candidates with masses matching those of the precursor ions are selected from them. Next, the theoretical product ion spectrum obtained by calculating the m/z values of all N-terminal ions (b-ions) and C-terminal ions (y-ions) generated upon cleavage at respective peptide bonds in a candidate peptide sequence is compared with the experimentally obtained product ion spectrum to identify the corresponding peptide sequences.

In DIA, mixed product ion spectra of all precursor ions that fall within the Q1 window with a predetermined m/z width are repeatedly acquired while shifting the window without selecting specific precursor ions. In DIA, chromatograms of fragment ions derived from the peptide sequence of interest are extracted by using an MS/MS spectral library generated from previously acquired DDA data, and chromatographic peak of the target peptide is then identified based on the co-elution pattern of the fragment ions.

In S/MRM, combinations of the m/z values of the precursor ions of target peptides identified by DDA and the m/z values of fragment ions generated by CID are specified in the measurement method. The ions of the target peptides in the sample (fragment ion signals) are selectively monitored to obtain chromatograms.

In DDA, when a large number of analytes are simultaneously eluted and their abundance levels differ greatly, there is a relatively high risk that analytes present at low abundance may not be detected in the initial MS spectrum or that the speed of the mass spectrometer may not be sufficient to acquire MS/MS spectra for all peaks detected in MS mode (i.e., too slow relative to the complexity of the sample). In contrast, in DIA, all MS/MS spectra of all detectable analytes passing through each Q1 window are collected, and the entire mass range is analyzed within the time frame of LC analysis. Accordingly, complete MS and MS/MS spectra of all detectable peaks in the sample can be acquired substantially, making DIA advantageous for the detection and identification of low-abundance analytes. In the preparation method of the present invention, since hydrophilic proteins such as albumin, which are abundantly present in the biological sample, are removed in the pretreatment stage while maintaining trace proteins, a peptide-containing sample with a high content of peptides derived from trace proteins can be prepared. Accordingly, by subjecting the peptide-containing sample prepared by the preparation method of the present invention to LC-MS/MS analysis in a DIA measurement mode, trace proteins and peptides derived therefrom, which are present in the biological sample, can be detected and identified with higher sensitivity, thereby enabling more sensitive and deeper proteome analysis.

Accordingly, the preparation method of the present invention ca n also be regarded as a method for identifying proteins contained in a biolog ical sample by mass spectrometry, the method comprising the following steps: 0) providing a fiber aggregate having a biological sample containing proteins adsorbed thereon;
1) washing the fiber aggregate having the biological sample containing proteins adsorbed thereon with an aqueous solvent to remove hydrophilic proteins contained in the biological sample from the fiber aggregate, and recovering the fiber aggregate having residual proteins bound thereto;
2) treating the recovered fiber aggregate having residual proteins bound thereto with a protease to digest the residual proteins bound to the fiber aggregate with the protease;
3) removing the fiber aggregate from the digestion reaction mixture to recover a peptide mixture as a digestion product;
4) subjecting the recovered peptide mixture to desalting treatment to obtain a purified peptide mixture;
5) subjecting the purified peptide mixture to mass spectrometry and identifying peptides contained in the peptide mixture based on the resulting mass spectrometry data; and
6) identifying proteins present in the biological sample from which the identified peptides are derived, based on the information of the identified peptides.

As described in detail in the Examples, by subjecting a peptide-containing sample prepared from a DBS (preferably a newborn DBS) using the preparation method of the present invention to mass spectrometry, it was possible to identify the proteins listed in Tables 1-1 to 1-20, which could not be detected by the conventional SCP method. These proteins included OMIM-hit proteins listed in Tables 2-1 to 2-7. Accordingly, in one embodiment, by subjecting a peptide-containing sample prepared from a DBS (preferably a newborn DBS) using the preparation method of the present invention to mass spectrometry, it is possible to identify at least one protein, for example at least 5, 10, 20, 50, 100, 200, 300, 400, 500, or 1000 proteins, selected from among the proteins listed in Tables 1-1 to 1-20 present in the DBS. In another embodiment, by subjecting a peptide-containing sample prepared from a DBS (preferably a newborn DBS) using the preparation method of the present invention to mass spectrometry, it is possible to identify at least one protein, for example at least 5, 10, 20, 50, 100, 200, 300, or 400 proteins, selected from among the OMIM-hit proteins listed in Tables 2-1 to 2-7 present in the DBS.

Table 3 shows a part of the proteins that were identified by subjecting a peptide-containing sample prepared from a DBS (preferably a newborn DBS) using the preparation method of the present invention to mass spectrometry, and which are expected to be applicable to newborn screening. Accordingly, in one embodiment, by subjecting a peptide-containing sample prepared from a DBS (preferably a newborn DBS) using the preparation method of the present invention to mass spectrometry, at least one, preferably 2, 3, 4, 5, 6, 7, 8, 9, 10, or 11 proteins selected from among the proteins listed in Table 3 present in the DBS may be identified. Among the proteins listed in Table 3, Coagulation factor VIII and Collagen alpha-1(I) chain were not detected by the SCP method, but could be detected from DBS for the first time using the preparation method of the present invention. Accordingly, in one embodiment, by subjecting a peptide-containing sample prepared from a DBS (preferably a newborn DBS) using the preparation method of the present invention to mass spectrometry, at least one, preferably both, of Coagulation factor VIII and Collagen alpha-1(I) chain present in the DBS may be identified.

### 2. Separation method of a non-magnetic solid-phase carrier

The present invention provides a method for separating a non-magnetic solid-phase carrier from a liquid containing the non-magnetic solid-phase carrier (hereinafter referred to as "the separation method of the present invention"), the method comprising the following steps:
1) providing a liquid containing magnetic particles and a non-magnetic solid-phase carrier;
2) agitating the liquid containing the magnetic particles and the non-magnetic solid-phase carriers to allow the magnetic particles to adhere to the non-magnetic solid-phase carrier;
3) bringing a magnet close to the liquid containing the magnetic particles and the non-magnetic solid-phase carriers to magnetically adsorb the non-magnetic solid-phase carrier to which the magnetic particles have adhered; and
4) separating the magnetically adsorbed non-magnetic solid-phase carrier from the liquid.

In the separation method of the present invention, a liquid containing magnetic particles and a non-magnetic solid-phase carrier is first provided. Specifically, such a liquid can be obtained by adding magnetic particles to a liquid containing a non-magnetic solid-phase carrier, by adding a non-magnetic solid-phase carrier to a liquid containing magnetic particles, or by adding both a non-magnetic solid-phase carrier and magnetic particles to a liquid.

As used herein, the term "carrier" refers to a substance that serves as a base material for adsorbing or immobilizing other substances. The carrier used in the separation method of the present invention is a non-magnetic solid-phase carrier. As used herein, the term "solid phase" includes gels. In one embodiment, the non-magnetic solid-phase carrier used in the separation method of the present invention contains an analytical sample (e.g., a biological sample), and the separation method of the present invention is carried out for the purpose of removing unnecessary substances from the non-magnetic solid-phase carrier, extracting necessary substances from the non-magnetic solid-phase carrier, modifying the sample contained in the non-magnetic solid-phase carrier or the like as a pretreatment process for intended analysis. The non-magnetic solid-phase carrier used in the separation method of the present invention preferably has a porous structure and contains voids therein. The voids are large enough to allow magnetic particles to enter. When a non-magnetic solid-phase carrier having a porous structure is used in the separation method of the present invention, magnetic particles enter the voids within the porous structure through agitation, strongly adhere to the non-magnetic solid-phase carrier and cannot be easily released from the non-magnetic solid-phase carrier. As a result, more reliable magnetic adsorption can be expected when a magnet is brought close. Examples of porous structures include fiber aggregates, foams, mesh structures and the like. A "fiber aggregate" refers to a structure in which fibers are overlaid three-dimensionally, regardless of whether the fibers are entangled. Examples of fiber aggregates include, but are not limited to, cotton-like, paper-like, fabric-like, and felt-like structures. A "foam" refers to a structure in which numerous bubbles are dispersed within a matrix, and includes, for example, sponge-like structures. A "mesh structure" refers to a structure in which polymers are three-dimensionally crosslinked in a network.

Examples of materials constituting the non-magnetic solid-phase carrier used in the separation method of the present invention include, but are not limited to, paper (e.g., filter paper), gels (e.g., agarose gel, polyacrylamide gel), fabrics (e.g., nonwoven fabrics), resins, cotton, swabs, glass, non-magnetic metals, and ceramics. In one embodiment, the non-magnetic solid-phase carrier used in the separation method of the present invention comprises a fiber aggregate composed of a material such as paper (e.g., filter paper), fabrics (e.g., nonwoven fabrics), resins, cotton, swabs, glass, non-magnetic metals, or ceramics.

In one embodiment, the non-magnetic solid-phase carrier used in the separation method of the present invention is sheet-shaped. The term "sheet-shaped" refers to a shape having two-dimensional planar extension and comprising two opposing surfaces (i.e., a front surface and a back surface) separated by a distance corresponding to the thickness. The thickness of the sheet is not particularly limited, but is, for example, 5 mm or less (e.g., 1 nm to 5 mm), and preferably 1 mm or less (e.g., 1 nm to 1 mm). When a non-magnetic solid-phase carrier in the form of a sheet is used, magnetic particles spread over the surface of the sheet opposite to the magnet and adhere thereto when a magnet is brought close, thereby magnetically attracting the non-magnetic solid-phase carrier in the direction of the magnet (i.e., in the direction of the magnetic force). Moreover, since the magnetic particles on the opposite surface are in close proximity to the magnet due to the sheet shape, more reliable magnetic adsorption can be expected.

In a preferred embodiment, the non-magnetic solid-phase carrier used in the separation method of the present invention has a porous structure (e.g., a fiber aggregate) and is sheet-shaped. When a sheet-shaped non-magnetic solid-phase carrier having a porous structure is used in the separation method of the present invention, magnetic particles can enter the voids within the porous structure and strongly adhere to the non-magnetic solid-phase carrier. Furthermore, the magnetic particles spread over the surface of the sheet opposite to the magnet and adhere thereto, thereby attracting the non-magnetic solid-phase carrier in the direction of the magnet (i.e., in the direction of the magnetic force). In addition, because the magnetic particles that have spread on the opposite surface are positioned at a distance corresponding to the thickness from the magnet, more reliable magnetic adsorption can be expected. Examples of sheet-shaped non-magnetic solid-phase carriers having a porous structure include paper (e.g., filter paper), fabrics (e.g., nonwoven fabrics), and the like.

The non-magnetic solid-phase carrier may be contained in a liquid in a suspended, dispersed, or floating state. The size (maximum diameter) of the non-magnetic solid-phase carrier is not particularly limited as long as it can remain suspended, dispersed, or floating in the liquid within the vessel used to carry out the separation method of the present invention. To facilitate separation of the non-magnetic solid-phase carrier from the liquid, the size (maximum diameter) of the non-magnetic solid-phase carrier is preferably smaller than the inner diameter of the opening of the vessel (e.g., a well) used in the separation method of the present invention. The size (maximum diameter) of the non-magnetic solid-phase carrier is typically 100 µm or more (e.g., 500 µm or more, 1 mm or more, 3 mm or more, or 5 mm or more).

The amount of the non-magnetic solid-phase carrier to be used is not particularly limited as long as the non-magnetic solid-phase carrier having magnetic particles adhered thereto can be magnetically adsorbed when a magnet is brought close to the liquid containing the dispersed magnetic particles and the non-magnetic solid-phase carrier. The specific amount of the non-magnetic solid-phase carrier to be added may be appropriately adjusted in consideration of factors such as the volume of the liquid. If the amount of the non-magnetic solid-phase carrier contained in the liquid is too large, it may become difficult to achieve sufficient mixing of the magnetic particles and the non-magnetic solid-phase carrier. Therefore, the volume of the non-magnetic solid-phase carrier is preferably 0.6 ml or less, more preferably 0.4 ml or less, and even more preferably 0.2 ml or less, per 1 ml of liquid.

If the specific gravity of the non-magnetic solid-phase carrier is too high, the non-magnetic solid-phase carrier will rapidly sediment in the liquid, making it difficult to achieve magnetic adsorption while the magnetic particles are adhered thereto. Accordingly, the apparent specific gravity of the non-magnetic solid-phase carrier in a state immersed in the liquid (hereinafter simply referred to as "the apparent specific gravity of the non-magnetic solid-phase carrier") is preferably 3.0 or less, more preferably 2.0 or less, still more preferably 1.5 or less, and even more preferably 1.2 or less. Even when the apparent specific gravity of the non-magnetic solid-phase carrier is lower than that of the liquid the separation method of the present invention can still be carried out while the non-magnetic solid-phase carrier remains floating on the surface of the liquid. However, the apparent specific gravity of the non-magnetic solid-phase carrier is preferably equal to or less than the specific gravity of the liquid used.

The magnetic particles used in the separation method of the present invention are not particularly limited as long as they contain a metal that is attracted to a magnet and have a property of being attracted to a magnet. Such magnetic particles may be composed of single-metal particles, alloy particles, metal oxide particles, or particles coated with a metal that is attracted to a magnet. Preferably, magnetic particles are particles of ferromagnetic metals such as iron, nickel, or cobalt, or particles of ferromagnetic metal oxides such as iron oxide or chromium oxide. More preferably, the magnetic particles are iron particles (iron powder) or iron oxide particles, and still more preferably, iron particles. The purity of iron in the iron particles is typically 90% (w/w) or more, and preferably 95% (w/w) or more. To prevent modification (e.g., oxidation) in the liquid, the magnetic particles may be coated with a polymer or the like, or may be uncoated.

If the particle diameter of the magnetic particles is too large, the sedimentation rate increases, causing the particles to accumulate at the bottom of the vessel. As a result, even when the liquid containing the magnetic particles and the non-magnetic solid-phase carrier is agitated, the magnetic particles may not properly adhere to the non-magnetic solid-phase carrier. Therefore, the average particle diameter of the magnetic particles is preferably 150 µm or less, more preferably 45 µm or less, and even more preferably 5 µm or less. The average particle diameter of the magnetic particles is typically 1 µm or more. As used herein, the average particle diameter of the magnetic particles refers to the volume-based average particle diameter. The average particle diameter of the magnetic particles can be measured using laser diffraction scattering methods.

The amount of magnetic particles to be added is an amount sufficient to enable magnetic adsorption of the non-magnetic solid-phase carrier having magnetic particles adhered thereto when a magnet is brought close to the liquid containing the magnetic particles and the non-magnetic solid-phase carrier. The specific amount of magnetic particles to be added may be appropriately adjusted in consideration of factors such as the volume of the liquid and the weight of the non-magnetic solid-phase carrier. If the amount of magnetic particles per unit volume of liquid is too small, magnetic adsorption of the non-magnetic solid-phase carrier having magnetic particles adhered thereto becomes difficult even when a magnet is brought close. Therefore, it is preferable to add magnetic particles in an amount of 0.3 mg or more, more preferably 0.6 mg or more, still more preferably 1.0 mg or more, even more preferably 3.0 mg or more, and further preferably 5.0 mg or more per 1 ml of liquid. On the other hand, if the volume of magnetic particles per unit volume of liquid is too large, magnetic adsorption of the non-magnetic solid-phase carrier having magnetic particles adhered thereto may be hindered due to the excessive magnetic particles. Therefore, the volume of magnetic particles to be added per 1 ml of liquid is preferably 0.5 ml or less, more preferably 0.25 ml or less, and still more preferably 0.1 ml or less. In addition, if the amount of magnetic particles per unit weight of the non-magnetic solid-phase carrier is too small, magnetic adsorption of the non-magnetic solid-phase carrier having magnetic particles adhered thereto becomes difficult. Therefore, magnetic particles are preferably added in an amount of 0.2 mg or more, more preferably 0.4 mg or more, still more preferably 0.66 mg or more, even more preferably 0.83 mg or more, yet more preferably 1.66 mg or more, further preferably 2.0 mg or more, and still further preferably 3.3 mg or more per 1 mg of the non-magnetic solid-phase carrier.

The specific gravity of the magnetic particles (referring to the apparent specific gravity in a state immersed in the liquid for dispersion, in the case where the magnetic particles have voids) is preferably higher than the specific gravity of the liquid in which they are dispersed.

If there is a large difference between the specific gravity of the magnetic particles and the apparent specific gravity of the non-magnetic solid-phase carrier, one of them may sediment more quickly when mixed, making it difficult for the magnetic particles to adhere uniformly to the non-magnetic solid-phase carrier. This may result in a decrease in the recovery rate by magnetic adsorption. Accordingly, it is preferable that the difference between the specific gravity of the magnetic particles and the apparent specific gravity of the non-magnetic solid-phase carrier be as small as possible, so that their sedimentation rates are as similar as possible. For example, assuming that the smaller of the two values (i.e., the specific gravity of the magnetic particles and the apparent specific gravity of the non-magnetic solid-phase carrier) is 1, it is preferable that the other value be 10.0 or less, 9.0 or less, 8.0 or less, 7.0 or less, 6.0 or less, 5.0 or less, 4.0 or less, 3.0 or less, 2.0 or less, 1.5 or less, or 1.2 or less.

The liquid used in the separation method of the present invention may be either aqueous or oily, but is preferably aqueous. The type of liquid is not particularly limited, and examples include a washing solution for removing an intended substance from the non-magnetic solid-phase carrier, an extraction solution for extracting an intended substance from the non-magnetic solid-phase carrier, and a reaction solution for modifying a sample contained in the non-magnetic solid-phase carrier.

In one embodiment, the liquid used in the separation method of the present invention contains a thickening agent. When magnetic particles having a specific gravity higher than that of the liquid are used, accurate dispensing operations become difficult due to sedimentation of the magnetic particles. However, by adding a thickening agent to the liquid, the sedimentation rate of the magnetic particles may be reduced, thereby allowing accurate dispensing operations. Examples of thickening agents include, but are not limited to: polyhydric alcohols having 3 to 6 carbon atoms such as glycerol, propylene glycol, diglycerol and the like; natural polysaccharides such as carrageenan, xanthan gum, dextrin, hyaluronic acid and the like; semi-synthetic polysaccharides such as methylcellulose, ethylcellulose, hydroxyethylcellulose, hydroxypropylcellulose and the like; and synthetic polymers such as polyvinyl alcohol, polyacrylic acid, polymethacrylic acid, carboxyvinyl polymer, copolymers of acrylic acid/alkyl acrylate (C10-C30) and the like. A thickening agent is added such that the viscosity of the liquid exceeds that of pure water (1 mPa·s), (for example, 2 mPa·s or more, 4 mPa·s or more, or 6 mPa·s or more). However, if the viscosity is too high, although sedimentation of the magnetic particles can be suppressed, the resulting excessive viscosity may hinder dispensing operations. Therefore, the viscosity of the liquid is typically 50 mPa·s or less (for example, 20 mPa·s or less or 10 mPa·s or less).

Next, the liquid containing magnetic particles and a non-magnetic solid-phase carrier is agitated to allow the magnetic particles to adhere to the non-magnetic solid-phase carrier. The agitation operation can be carried out using known methods, such as a vortex mixer, a plate mixer, or a spin chip provided in devices such as Maelstrom 8. The magnetic particles may adhere to the outside of the non-magnetic solid-phase carrier, or may penetrate into the inside of the non-magnetic solid-phase carrier and adhere internally. When a non-magnetic solid-phase carrier having a porous structure is used, the magnetic particles can enter the voids in the porous structure during agitation, thereby adhering tightly to the non-magnetic solid-phase carrier. In particular, when a fiber aggregate such as filter paper is used as the non-magnetic solid-phase carrier, agitation loosens the fibers and expands the voids between the fibers, allowing a large amount of magnetic particles to be incorporated into the interior of the non-magnetic solid-phase carrier. Further, when a thickening agent is added to the liquid, the sedimentation rates of the non-magnetic solid-phase carrier and the magnetic particles become equally low, allowing the two to be well mixed in the liquid, and allowing the magnetic particles to be uniformly incorporated into the non-magnetic solid-phase carriers.

Once the magnetic particles have adhered to the non-magnetic solid-phase carrier, a magnet is brought close to the liquid containing the magnetic particles and the non-magnetic solid-phase carrier. As the magnetic particles are attracted to the magnet, the non-magnetic solid-phase carrier having the magnetic particles adhered thereto is attracted toward the magnet and is magnetically adsorbed onto the magnet or the side surface of the vessel. The magnetic force (surface magnetic flux density) of the magnet used should be sufficient to magnetically adsorb the non-magnetic solid-phase carrier having the magnetic particles adhered thereto, and is typically 1000 gauss or more, preferably 2000 gauss or more, and more preferably 3000 gauss or more. The magnet may be either a permanent magnet or an electromagnet. Since an electromagnet allows magnetic force to be switched on and off electrically, it offers excellent operability. To prevent the magnetic particles and the non-magnetic solid-phase carrier from being directly adsorbed onto the magnet, the magnet may be covered with a resin cap or the like.

Then, the magnetically adsorbed non-magnetic solid-phase carrier is separated from the liquid. The separation may be carried out by moving the magnetically adsorbed non-magnetic solid-phase carrier out of the liquid through movement of the magnet, or by removing the liquid from the vessel while keeping the non-magnetic solid-phase carrier in a magnetically adsorbed state, thereby separating the non-magnetic solid-phase carrier from the liquid.

In one embodiment, a magnet is brought close to the side surface of a vessel containing the liquid containing the magnetic particles and the non-magnetic solid-phase carrier, thereby allowing the non-magnetic solid-phase carrier having the magnetic particles adhered thereto to be magnetically adsorbed onto the inner wall surface of the vessel near the magnet. For example, an appropriate magnetic separator (magnet stand) corresponding to the shape of the vessel may be used as the magnet. Then, while holding the non-magnetic solid-phase carrier on the inner wall surface of the vessel, the liquid may be removed from the vessel, or the magnet may be moved to separate the non-magnetic solid-phase carrier, which has been magnetically adsorbed onto the wall surface, out of the liquid along the wall.

In another embodiment, the magnet is inserted into the liquid containing the magnetic particles and the non-magnetic solid-phase carrier along the direction of gravity, thereby magnetically adsorbing the non-magnetic solid-phase carrier having magnetic particles adhered thereto onto the magnet. Preferably, the magnet is a rod-shaped magnet (magnetic rod) having a magnetic pole at its tip, and the magnetic pole is inserted into the liquid along the direction of gravity, whereby the non-magnetic solid-phase carrier having magnetic particles adhered thereto is magnetically adsorbed onto the magnetic pole together with the magnetic particles. Preferably, magnetic particles having a specific gravity higher than that of the liquid are used. When the magnet is inserted along the direction of gravity, the magnetic particles abundantly adhered to the lower surface (in the direction of gravity) of the non-magnetic solid-phase carrier are pulled upward, against gravity, together with the non-magnetic solid-phase carrier and are more reliably adsorbed onto the magnet. Accordingly, the risk of losing the non-magnetic solid-phase carrier during operation or the non-magnetic solid-phase carrier falling from the magnet is suppressed. In particular, when a sheet-shaped non-magnetic solid-phase carrier, such as paper, fabric and the like, is used, the magnetic particles spread and adhered to the lower (bottom) surface of the sheet-shaped non-magnetic solid-phase carrier in the direction of gravity pull the non-magnetic solid-phase carrier upward against gravity. Furthermore, because the carrier is sheet-shaped, the distance between the magnetic particles and the magnet is reduced to only the thickness of the sheet, thereby allowing the non-magnetic solid-phase carrier to be more reliably retained in a magnetically adsorbed state on the magnet.

According to the separation method of the present invention, it becomes possible to separate a non-magnetic solid-phase carrier from a liquid in a magnetism-dependent manner. Therefore, by combining the method with an automated device for handling magnetic solid-phase carriers including magnetic beads (e.g., Maelstrom 8 Autostage (MS8), manufactured by Taiwan Advanced Nanotech Inc.), a large number of non-magnetic solid-phase carriers can be automatically processed. For example, in a vessel with a plurality of wells arranged in an array, such as a 96-well plate, a non-magnetic solid-phase carrier, magnetic particles and liquid 1 are added to a first well, and liquid 2 is added to a second well. By agitating the mixture of the non-magnetic solid-phase carrier, magnetic particles and liquid 1 in the first well, the non-magnetic solid-phase carrier is treated with liquid 1 and magnetic particles are caused to adhere to the non-magnetic solid-phase carrier. Subsequently, a rod-shaped magnet (magnetic rod) having a magnetic pole at its tip is inserted into liquid 1 containing the non-magnetic solid-phase carrier having magnetic beads adsorbed thereto, along the direction of gravity, thereby allowing the non-magnetic solid-phase carrier having magnetic particles adhered thereto to be magnetically adsorbed onto the magnet. Preferably, the magnet is an electromagnet that allows switching on and off of the magnetic field. In order to prevent direct contact of the liquid or magnetic particles with the magnet, the magnet may be covered with a resin cap or the like. For example, a tubular cover is fitted over the rod-shaped magnet, and the rod-shaped magnet with the cover attached is inserted into liquid 1 containing the non-magnetic solid-phase carrier having magnetic particles adhered thereto along the direction of gravity. Alternatively, the tubular cover may first be inserted into liquid 1 containing the non-magnetic solid-phase carrier having magnetic particles adhered thereto, and then the rod-shaped magnet may be inserted into the tubular cover along the direction of gravity. By then pulling the magnet upward, in a direction opposite to gravity, the non-magnetic solid-phase carrier having magnetic particles adhered thereto and magnetically adsorbed onto the magnet can be separated from liquid 1. While keeping the separated non-magnetic solid-phase carrier having magnetic particles adhered thereto adsorbed on the magnet, it is transferred into liquid 2 in the second well. By turning off the switch of the electromagnet or pulling the rod-shaped magnet out from the tubular cover, the non-magnetic solid-phase carrier having magnetic particles adhered thereto is released into liquid 2. Then, by agitating the second well, the non-magnetic solid-phase carrier is treated with liquid 2, thereby obtaining the non-magnetic solid-phase carrier having magnetic particles adhered thereto and dispersed in liquid 2. By sequentially performing these series of operations, the non-magnetic solid-phase carrier can be serially treated with various types of liquids. Furthermore, by using a plurality of rod-shaped magnets arranged to correspond to the layout of the wells (for example, 8-channel or 12-channel magnetic rods for use with a 96-well plate), and automating the operations including magnet insertion, movement, well agitation and the like, a large number of non-magnetic solid-phase carriers can be automatically and serially treated with various types of liquids.

Conventionally, when performing washing, extraction, or other processing serially on a non-magnetic solid-phase carrier using a liquid, the non-magnetic solid-phase carrier is typically left inside a vessel and precipitated by centrifugation, followed by addition and recovery of the liquid for washing or extraction. In such procedures, the precipitated non-magnetic solid-phase carrier may be unintentionally aspirated when the liquid is removed or recovered from the container, or the precipitate may float, resulting in loss of the non-magnetic solid-phase carrier. In contrast, in the method of the present invention, the non-magnetic solid-phase carrier is removed from the vessel while leaving the liquid for washing or extraction, rather than the non-magnetic solid-phase carrier, in the vessel. Therefore, the non-magnetic solid-phase carrier can be subjected to serial processing with a liquid such as washing or extraction while minimizing the loss of the non-magnetic solid-phase carrier. Accordingly, the separation method of the present invention is useful, for example, when the non-magnetic solid-phase carrier is to be serially washed with liquids or a substance retained in the non-magnetic solid-phase carrier is to be treated stepwise with various reaction solutions, while retaining the intended substance (e.g., an analytical sample) in the non-magnetic solid-phase carrier.

### 3. Preparation of peptide-containing sample using magnetic particles

In one embodiment, magnetic particles are employed in the preparation method of the present invention as detailed in Chapter 1, by applying the separation method of the present invention as detailed in Chapter 2. Magnetic particles are allowed to adhere to the fiber aggregate, and operations such as recovery and removal of the fiber aggregate are performed by magnetically adsorbing the fiber aggregate having the magnetic particles adhered thereto. By handling the fiber aggregate through magnetic adsorption, operations such as washing, recovery, and buffer exchange of the fiber aggregate having a biological sample adsorbed thereon can be carried out more easily. In addition, automated processing using an automated nucleic acid extraction device or the like becomes feasible, allowing a significantly large number of samples to be processed, and thereby improving the reproducibility of the results.

In one embodiment, the preparation method of the present invention further comprises agitating, in an aqueous solvent, magnetic particles and a fiber aggregate having a biological sample containing proteins adsorbed thereon, to allow the magnetic particles to adhere to the fiber aggregate.

As the magnetic particles, those described in Chapter 2 above as usable in the separation method of the present invention can be employed. The magnetic particles are preferably iron particles (iron powder) or iron oxide particles, and more preferably, iron particles. The magnetic particles may or may not be coated with a polymer or the like in order to prevent modification (such as oxidation) in the liquid.

The particle diameter of the magnetic particles can be appropriately set in accordance with the description in Chapter 2 above.

The amount of magnetic particles to be added is sufficient to allow magnetic adsorption of the fiber aggregate having the magnetic particles adhered thereto when a magnet is brought close to the aqueous solvent containing the magnetic particles and the fiber aggregate, and can be set as described in Chapter 2 above.

The specific gravity of the magnetic particles (referring to the apparent specific gravity in a state immersed in the liquid for dispersion, in the case where the magnetic particles have voids) is preferably higher than the specific gravity of the liquid in which they are dispersed and can be appropriately set in accordance with the description in Chapter 2 above.

As for the structure of the fiber aggregate, examples include cotton-like, paper-like, fabric-like, or felt-like forms, but are not limited thereto. In this embodiment where magnetic particles are used, the fiber aggregate employed in the preparation method of the present invention preferably contains voids inside (i.e., between fibers), and such voids are of a size sufficient to allow the magnetic particles to enter. When magnetic particles and a fiber aggregate having a biological sample containing proteins adsorbed thereon is agitated in an aqueous solvent, the magnetic particles enter the voids within the fiber aggregate due to the agitation and tightly adhere to the fiber aggregate, making it difficult for the magnetic particles to release therefrom. Accordingly, more reliable magnetic adsorption can be expected when a magnet is brought close. Preferably, the fiber aggregate, when agitated in an aqueous solvent, does not disperse into individual fibers in the solvent, but rather the fibers remain entangled with each other, thereby maintaining the aggregated form and retaining magnetic particles between the fibers. When cellulose fiber aggregate such as paper (e.g., filter paper), cotton, cotton yarn, or cotton fabric are used as the fiber aggregate, it is preferable to select those that, upon agitation in an aqueous solvent, form voids of sufficient size to allow the magnetic particles to enter between the fibers. For example, in the case of paper, it is preferable to use one that becomes swollen and pulpy when agitated in an aqueous solvent, thereby forming voids of a size sufficient to allow the magnetic particles to enter. The term "pulpy" refers to a state in which filament units or fibers close to filament units are entangled in a disordered manner. When filter paper is agitated together with magnetic particles in an aqueous solvent, it becomes swollen and pulpy and firmly retains the magnetic particles that have entered the voids between the fibers. Therefore, filter paper is preferably used in combination with magnetic particles in the preparation method of the present invention.

In this embodiment, the fiber aggregate to be used may be sheet-shaped. The thickness thereof is not particularly limited, but is, for example, 5 mm or less (e.g., 1 nm to 5 mm), and preferably 1 mm or less (e.g., 1 nm to 1 mm). Examples of sheet-shaped fiber aggregates include paper (e.g., filter paper) and fabric (e.g., nonwoven fabric).

The amount and specific gravity of the fiber aggregate to be used can be appropriately set in accordance with the description in Chapter 2 above.

As the aqueous solvent used for agitating the magnetic particles and the fiber aggregate having a biological sample containing proteins adsorbed thereon, the same aqueous solvent described above for removing hydrophilic proteins from the fiber aggregate in Step 1 of the preparation method of the present invention may be used.

A thickening agent may be added to the aqueous solvent. When magnetic particles having a higher specific gravity than the aqueous solvent are used, sedimentation of the particles may hinder accurate dispensing operations. However, by adding a thickening agent to the aqueous solvent, the sedimentation rate may be reduced, enabling accurate dispensing. As the thickening agent, those described in Chapter 2 above may be used. A thickening agent is added such that the viscosity of the aqueous solvent exceeds that of pure water (1 mPa·s), (for example, 2 mPa·s or more, 4 mPa·s or more, or 6 mPa·s or more). However, if the viscosity is too high, although sedimentation of the magnetic particles can be suppressed, the resulting excessive viscosity may hinder dispensing operations. Therefore, the viscosity of the aqueous solvent is typically 50 mPa·s or less (for example, 20 mPa·s or less or 10 mPa·s or less).

The agitation operation can be carried out in accordance with the description in Chapter 2. By agitation, the magnetic particles enter the voids between the fibers constituting the fiber aggregate and may be tightly adhered to the fiber aggregate. In particular, when filter paper is used as the fiber aggregate, agitation loosens the fibers and expands the voids between the fibers, allowing a large amount of magnetic particles to be incorporated into the interior of the non-magnetic solid-phase carrier. In addition, when a thickening agent is added to the aqueous solvent, the sedimentation rates of the fiber aggregate and the magnetic particles become equally low, allowing the two to be well mixed in the liquid, and allowing the magnetic particles to be uniformly incorporated into the fiber aggregate.

The fiber aggregate having a biological sample containing proteins adsorbed thereon and magnetic particles adhered thereto, obtained in this manner, can be subjected to the subsequent operations starting from Step 1.

The adhesion of magnetic particles to the fiber aggregate may be performed prior to Step 1 or concurrently with Step 1. In the case where the step of adhering magnetic particles is carried out prior to Step 1, the resulting fiber aggregate having a biological sample containing proteins adsorbed thereon and magnetic particles adhered thereto is subjected to the subsequent operations starting from Step 1. On the other hand, when the adhesion of magnetic particles to the fiber aggregate is carried out concurrently with Step 1, magnetic particles are included in the aqueous solvent for washing the fiber aggregate having a biological sample containing proteins adsorbed thereon. Then, by agitating the fiber aggregate having a biological sample containing proteins adsorbed thereon in the aqueous solvent containing magnetic particles, the fiber aggregate is washed with the aqueous solvent to remove hydrophilic proteins contained in the biological sample from the fiber aggregate, and the magnetic particles are adhered to the fiber aggregate. As a result, a fiber aggregate having residual proteins bound thereto and magnetic particles adhered thereto is recovered.

The operations such as the recovery of the fiber aggregate after magnetic particles are adhered thereto can be carried out by magnetically adsorbing the fiber aggregate having the magnetic particles adhered thereto. For example, at least one, preferably both, of the operation of recovering the fiber aggregate having residual proteins bound thereto in Step 1 and the operation of removing the fiber aggregate from the digestion reaction mixture in Step 3 can be carried out by magnetically adsorbing the fiber aggregate having magnetic particles adhered thereto. In addition, the recovery or washing of the fiber aggregate having reduced residual proteins bound thereto, or the recovery or washing of the fiber aggregate having reductively alkylated residual proteins bound thereto, in the case where reductive alkylation is carried out, as well as the protease treatment of the fiber aggregate having residual proteins bound thereto in Step 2 (i.e., the addition of the fiber aggregate having residual proteins bound thereto to an aqueous solvent containing a protease), can also be carried out by magnetically adsorbing the fiber aggregate having magnetic particles adhered thereto.

The magnetic adsorption of the fiber aggregate having magnetic particles adhered thereto can be carried out, in accordance with the description in Chapter 2, by bringing a magnet close to the aqueous solvent containing the fiber aggregate having magnetic particles adhered thereto. When the magnetic particles are attracted to the approaching magnet, the fiber aggregate having the magnetic particles adhered thereto is also attracted to the magnet along with the magnetic particles and is magnetically adsorbed onto the magnet or the side surface of the vessel. The magnetic force (surface magnetic flux density) of the magnet to be used is sufficient to magnetically adsorb the fiber aggregate having magnetic particles adhered thereto, and is typically 1000 gauss or more, preferably 2000 gauss or more, and more preferably 3000 gauss or more. The magnet may be either a permanent magnet or an electromagnet. Since an electromagnet allows magnetic force to be switched on and off electrically, it offers excellent operability. To prevent the magnetic particles and the non-magnetic solid-phase carrier from being directly adsorbed onto the magnet, the magnet may be covered with a resin cap or the like.

Then, in accordance with the description in Chapter 2, the fiber aggregate magnetically adsorbed is separated from the aqueous solvent. The separation of the fiber aggregate from the aqueous solvent may be carried out by moving the magnet to transfer the magnetically adsorbed fiber aggregate out of the aqueous solvent, or alternatively, by removing the aqueous solvent from the vessel while maintaining the magnetic adsorption of the fiber aggregate, thereby separating the fiber aggregate from the aqueous solvent.

In one embodiment, a magnet is brought close to the side surface of a vessel containing an aqueous solvent containing a fiber aggregate having magnetic particles adhered thereto is suspended, so as to allow the fiber aggregate having magnetic particles adhered thereto to be magnetically adsorbed onto the inner wall surface of the vessel near the magnet. For example, a suitable magnetic separator (magnet stand) corresponding to the shape of the vessel to be used may be employed as the magnet. Thereafter, the aqueous solvent may be removed from the vessel while holding the fiber aggregate on the inner wall surface of the vessel, or alternatively, the magnet may be moved to transfer the magnetically adsorbed fiber aggregate along the wall surface and separate it out of the aqueous solvent.

In another embodiment, a magnet is inserted, along the direction of gravity, into an aqueous solvent containing a fiber aggregate having magnetic particles adhered thereto, so that the fiber aggregate having magnetic particles adhered thereto is magnetically adsorbed onto the magnet. Preferably, the magnet is a rod-shaped magnet (magnetic rod) having a magnetic pole at its tip, and the magnetic pole is inserted into the aqueous solvent containing the fiber aggregate having magnetic particles adhered thereto along the direction of gravity, whereby the fiber aggregate having magnetic particles adhered thereto is magnetically adsorbed onto the magnetic pole together with the magnetic particles. Preferably, magnetic particles having a specific gravity higher than that of the liquid are used. When the magnet is inserted along the direction of gravity, the magnetic particles abundantly adhered to the lower surface (in the direction of gravity) or inside of the fiber aggregate are pulled upward, against gravity, together with the fiber aggregate, thereby allowing the fiber aggregate to be more reliably adsorbed onto the magnet. Accordingly, the risk of losing the fiber aggregate or the fiber aggregate falling from the magnet during operation is suppressed. In particular, when a sheet-shaped fiber aggregate, such as paper, fabric and the like, is used, the magnetic particles spread and adhered to the lower (bottom) surface of the sheet-shaped fiber aggregate in the direction of gravity pull the fiber aggregate upward against gravity. Furthermore, because the fiber aggregate is sheet-shaped, the distance between the magnetic particles and the magnet is reduced to only the thickness of the sheet, thereby allowing the fiber aggregate to be more reliably retained in a magnetically adsorbed state on the magnet.

By using magnetic particles, the fiber aggregate can be separated from the aqueous solvent in a magnetism-dependent manner. Therefore, by combining with an automated device for handling magnetic solid-phase carriers including magnetic beads (e.g., Maelstrom 8 Autostage (MS8), manufactured by Taiwan Advanced Nanotech Inc.), it becomes possible to prepare a peptide-containing sample from a fiber aggregate having a biological sample containing proteins adsorbed thereon, for identifying proteins contained in the biological sample by mass spectrometry, in an automated manner. For example, in a vessel with a plurality of wells arranged in an array, such as a 96-well plate, aqueous solvents for processing the fiber aggregate having a biological sample containing proteins adsorbed thereon are added to the respective wells in accordance with each step of the preparation method of the present invention and arranged in the order of the steps. Then, by sequentially transferring the fiber aggregate having the biological sample adsorbed thereon to each well containing the aqueous solvent for processing in accordance with each step, the preparation method of the present invention can be carried out. In this case, the transfer of the fiber aggregate between the wells is carried out by magnetically adsorbing the fiber aggregate having magnetic particles adhered thereto.

Specifically, in a vessel with a plurality of wells arranged in an array, such as a 96-well plate, a fiber aggregate having biological sample absorbed thereto, magnetic particles and an aqueous solvent for washing for use in Step 1 are added to a first well, and an aqueous solvent containing protease for use in Step 2 is added to a second well. By agitating the mixture of the fiber aggregate having biological sample absorbed thereto, magnetic particles and the aqueous solvent for washing in the first well, hydrophilic proteins contained in the biological sample are removed from the fiber aggregate into the aqueous solvent for washing, and the magnetic particles are adhered to the fiber aggregate, whereby the fiber aggregate having residual proteins bound thereto is obtained. Subsequently, a rod-shaped magnet (magnetic rod) having a magnetic pole at its tip is inserted into the first well containing the fiber aggregate having residual proteins bound thereto and magnetic beads adsorbed thereto, along the direction of gravity, thereby allowing the fiber aggregate having residual proteins bound thereto and magnetic beads adsorbed thereto to be magnetically adsorbed onto the magnet. Preferably, the magnet is an electromagnet that allows switching on and off of the magnetic field. **In** order to prevent direct contact of the aqueous solvent for washing or magnetic particles with the magnet, the magnet may be covered with a resin cap or the like. For example, a tubular cover is fitted over the rod-shaped magnet, and the rod-shaped magnet with the cover attached is inserted into the first well containing the fiber aggregate having residual proteins bound thereto and magnetic beads adsorbed thereto along the direction of gravity. Alternatively, the tubular cover may first be inserted into the first well containing the fiber aggregate having residual proteins bound thereto and magnetic beads adsorbed thereto, and then the rod-shaped magnet may be inserted into the tubular cover along the direction of gravity. By then pulling the magnet upward, in a direction opposite to gravity, from the first well, the fiber aggregate having residual proteins bound thereto and magnetic beads adsorbed thereto magnetically adsorbed onto the magnet can be separated from the aqueous solvent for washing in the first well. While keeping the fiber aggregate having residual proteins bound thereto and magnetic beads adsorbed thereto adsorbed on the magnet, it is transferred into an aqueous solution of a protease solution in the second well. By turning off the switch of the electromagnet or pulling the rod-shaped magnet out from the tubular cover, the fiber aggregate having residual proteins bound thereto and magnetic beads adsorbed thereto is released into the aqueous solution of the protease. Then, by agitating the second well, the fiber aggregate having residual proteins bound thereto and magnetic beads adsorbed thereto is treated with the protease, thereby digesting the residual proteins bound to the fiber aggregate with the protease. The peptide mixture as a digestion product is released from the fiber aggregate into the aqueous solution of the protease. A rod-shaped magnet is inserted into the second well containing the digestion reaction mixture along the direction of gravity. Alternatively, the tubular cover may first be inserted into the second well, and then the rod-shaped magnet may be inserted into the tubular cover along the direction of gravity. By then pulling the magnet upward, in a direction opposite to gravity, from the second well, the fiber aggregate having magnetic beads adsorbed thereto magnetically adsorbed onto the magnet is removed from the digestion reaction mixture, whereby the peptide mixture as a digestion product is recovered in the second well.

In the above example, the aqueous solvent for washing to be used in Step 1 is added to the first well, and the aqueous solution of the protease to be used in Step 2 is added to the second well. However, the present invention is not limited thereto, and other wells containing processing solutions appropriate for desired treatments may be provided, in which the fiber aggregate having residual proteins bound thereto and magnetic particles adhered thereto may be processed. The fiber aggregate having residual proteins bound thereto and magnetic particles adhered thereto can be serially treated with various types of liquids by transferring the fiber aggregate between the wells by inserting, moving, and removing the magnet as described above to perform a series of operations. For example, a plurality of wells containing an aqueous solvent for washing may be provided, and the fiber aggregate having residual proteins bound thereto and magnetic particles adhered thereto may be sequentially transferred into those wells for repeated washing in Step 1. Alternatively, 1) a well containing a reducing agent, 2) a well containing an alkylating agent, and 3) a well containing a aqueous solvent for washing may be provided. The fiber aggregate having residual proteins bound thereto and magnetic particles adhered thereto and magnetic obtained in Step 1, may be sequentially transferred into the wells containing 1) a well containing a reducing agent → 2) a well containing an alkylating agent- 3) a well containing a aqueous solvent for washing thereby subjecting the fiber aggregate to reductive alkylation of the proteins to obtain a fiber aggregate having alkylated residual proteins bound thereto and the magnetic particles adhered thereto. The obtained fiber aggregate having reduced and alkylated residual proteins bound thereto and magnetic particles adhered thereto may subsequently be transferred into a well containing an aqueous solution of the protease to carry out Step 2 by protease treatment.

By using a plurality of rod-shaped magnets arranged to correspond to the layout of the wells (for example, 8-channel or 12-channel magnetic rods for use with a 96-well plate), and automating the operations including magnet insertion, movement, well agitation and the like, it becomes possible to automatically and serially prepare a peptide-containing sample for identifying the proteins contained in the biological sample by mass spectrometry, from a large number of fiber aggregates having a biological sample containing proteins adsorbed thereon.

The definitions of terms in this chapter follow those given in Chapters 1 and 2, unless otherwise specified.

All references cited in the present specification, including publication, patent document and the like, are hereby incorporated individually and specifically by reference, to the extent that the entireties thereof have been specifically disclosed herein.

The present invention is explained in more detail in the following by referring to Examples, which are not to be construed as limitative.

### EXAMPLES

### [Example 1]

### I. Materials and Methods

### 1. Reagents

Dried filter paper blood was prepared by soaking blood from the fingertips of healthy volunteers on filter paper for blood collection (ADVANTEC (Tokyo, Japan)) and then drying. Water, acetonitrile, 0.1% (v/v) formic acid-water, and 0.1% (v/v) formic acid-acetonitrile for mass spectrometry were purchased from Thermo Fisher Scientific (Waltham, MA). Trypsin/Lys-C Mix for mass spectrometry was obtained from Promega (Madison, WI). Iron powder (3-5 µm particle diameter, 99.9%) was purchased from Kojundo Chemical Laboratory Co. Ltd. (Saitama, Japan). 5 mm zirconia beads (Tommy Seiko Corporation, Tokyo, Japan), TBST: Tris buffered saline with Tween 20 (10x), liquid was purchased from Nakalai Tesque Corporation (Kyoto, Japan). Sodium dodecanoate, 1M Tris-HCl (pH 8.0): Trisma (registered trademark) hydrochloride solution pH 8.0, 1 Molar was purchased from Sigma-Aldrich Co. LLC (Saint Louis, MO). n-dodecyl-β-D-maltoside (DDM) was purchased from Fujifilm Wako Pure Chemicals Corporation (Osaka, Japan). BCA, Pierce ^{™}BCA Protein Assay Kit was purchased from Thermo Fisher Scientific (Waltham, MA). GL-Tip SDB was purchased from G.L. Science Corporation (Tokyo, Japan). Other reagents were purchased from Fujifilm Wako Pure Chemicals Corporation (Osaka, Japan).

### 2. Instruments

The following instruments were used in this experiment:
Stirring centrifuge: NSD-12 (Nisshin Rika, Tokyo, Japan)
Automated extraction instrument: Maelstrom 8 Autostage, Maelstrom 9610 (Taiwan Advanced Nanotech Inc., Taoyuan City 333, Taiwan (R.O.C.))
Sonicator: BIORUPTOR, UCW-201 (Sonic Bio Corporation, Kanagawa, Japan) Tissue Lyser (Qiagen, Hildenm, Germany)

### 3. Extraction of hydrophobic proteins by sodium carbonate precipitation (SCP)

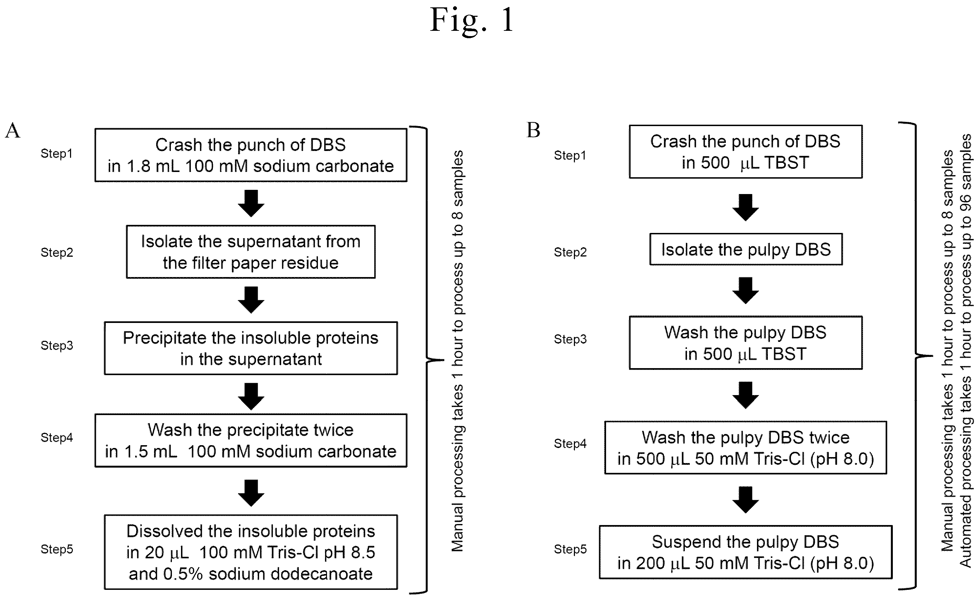

An overview is shown in Fig. 1A.
Step 1: Two 3.2 mm-diameter DBS discs punched out from DBS and one zirconia bead were added to 900 µL 100 mM sodium carbonate aqueous solution. The mixture was vigorously agitated using a TissueLyser at 25 Hz for 5 minutes to crush the DBS. 900 µL 100 mM sodium carbonate aqueous solution was added and the resulting mixture was sonicated (BIORUPTOR, UCW-201, "High", 5 minutes).
Step 2: The mixture was centrifuged (3,000g, 4 °C, 3 minutes), after which the supernatant was transferred to a fresh 1.5 mL Eppendorf tube and the precipitated filter paper residue was discarded.
Step 3: The supernatant was centrifuged (17,400g, 4 °C, 15 minutes), after which the supernatant was completely removed to isolate the precipitate.
Step 4: 1.5 mL 100 mM sodium carbonate aqueous solution was added and the resulting mixture was sonicated (BIORUPTOR, UCW-201, "High", 5 minutes). After centrifugation (17,400g, 4 °C, 15 minutes), the supernatant was completely removed to wash the precipitate.
Step 5: The precipitate was dissolved by adding 20 µL 100 mM Tris-Cl pH 8.5, 0.5% sodium dodecanoate, followed by vigorous agitation (stirring centrifuge, max, 1 minute) and ultrasonication (BIORUPTOR, UCW-201, "High", 5 minutes). The proteins were subjected to reductive alkylation and then digested with trypsin. The resulting peptides were desalted using an SDB tip, dried, and dissolved in 3% CAN, 0.1% TFA, followed by LC-MS analysis. For further details, see Nakajima et al., Journal of Proteome Research, 2020, 19, 2821-2827.

### 4. Extraction of filter paper-binding proteins (FPBP method)

An overview is shown in Fig. 1B.
Steps 1 and 2: One 3.2 mm-diameter DBS disc was added to 500 µL TBST, vigorously agitated (stirring centrifuge, max, 30 minutes) and then centrifuged (15,000g, rt, 5 minutes). The supernatant was removed to isolate pulpy DBS.
Step 3: Pulpy DBS was washed by adding 500 µL TBST, agitated vigorously (stirring centrifuge, max, 10 minutes), and centrifuged (15,000g, rt, 5 minutes), after which the supernatant was removed.
Step 4: 500 µL 50 mM Tris-HCl pH 8.0 was added, and the mixture was agitated vigorously (stirring centrifuge, max, 10 minutes) and centrifuged (15,000g, rt, 2 minutes), followed by supernatant removal. Afterward, another 500 µL 50 mM Tris-HCl pH 8.0 was added, and the mixture was agitated vigorously (valerina, max, 2 minutes) and centrifuged (15,000g, rt, 5 minutes), after which the supernatant was removed to wash the pulpy DBS.
Step 5: The pulpy DBS was suspended by adding 200 µL 50 mM Tris-HCl pH 8.0 and agitating vigorously (stirring centrifuge, max, 3 minutes).

4 µL of 500 ng/µL Trypsin/Lys-C Mix was added to the DBS suspension, and the proteins were digested overnight at 37°C. The mixture was placed on a magnet to allow magnetic adsorption of the complex of filter paper and iron powder onto the magnet, and the supernatant was subsequently transferred to a fresh tube. 50 µL of 5% TFA was added to acidify the sample, and the mixture was desalted using GL-Tip SDB. GL-Tip SDB was used for desalting treatment. The GL-Tip SDB was equilibrated with 25 µL 80% acetonitrile, 0.1% TFA and 50 µL of 3% acetonitrile, 0.1% TFA, and then the acidified sample was loaded. After washing with 80 µL 3% acetonitrile, 0.1% TFA, peptides were eluted with 30 µL 50% acetonitrile, 0.1% TFA. The eluate was dried and dissolved in 12.5 µL 0.02% DDM aqueous solution. Protein concentration was measured using the BCA method and adjusted to 200 ng/µL, and 500 ng was subjected to LC-MS analysis. In the SCP method, reductive alkylation was performed; however, in the FPBP method, preliminary experiments showed that omitting reductive alkylation did not significantly reduce the number of disease-causing proteins detected. Therefore, in consideration of speed and labor savings, reductive alkylation was not performed in this experiment.

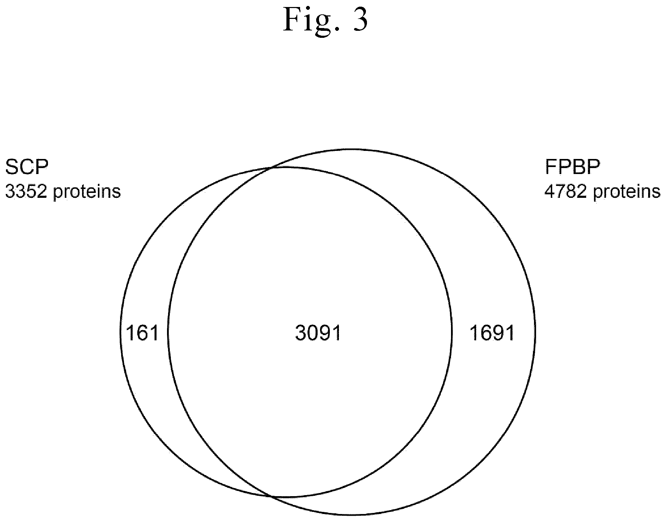

### 5. Automated extraction of filter paper binding proteins (FPBP) (Fig. 3)

### (1) Preparation of plates for MaelStrom 8 Autostage/MaelStrom 9610 processing

500 µL 1× TBST, 5 mg iron powder solution (3-5 µm particle diameter, 5 mg/25 µL in 50% glycerol), and one 3.2 mm diameter disk punched out from DBS were added to the first row/first 96-well plate. 500 µL 1× TBST was added to the second row/second 96-well plate. 500 µL 50 mM Tris-HCl pH 8.0 was added to the third row/third 96-well plate. 500 µL 50 mM Tris-HCl pH 8.0 was added to the fourth row/fourth 96-well plate. 200 µL of 50 mM Tris-HCl pH 8.0 was added to the fifth row/fifth 96-well plate.

### (2) Details of automated extraction using MaelStrom 8 Autostage/MaelStrom 9610

### (2-1) Crushing of filter paper in TBST

A spin tip was inserted into the first row/first plate, and the DBS was crushed by agitating in TBST for 30 min at 3,500 rpm with inverting every 10 sec. This formed a complex of pulpy DBS and iron powder. Next, a magnetic rod was inserted into the spin tip, and the complex was adsorbed and collected on the spin tip over 60 sec and then moved to the second row/second plate.

### (2-2) Washing of complexes using TBST and 50 mM Tris-Cl pH 8.0

After removing the magnet rod from the spin tip in TBST, the complex was washed by agitation for 10 min at 3,500 rpm with inverting every 10 sec. A magnetic rod was inserted into the spin tip, and the complex was adsorbed and collected on the spin tip over 60 sec and then moved to the third row/third plate. After removing the magnet rod from the spin tip in 50 mM Tris-HCl pH 8.0, the complex was washed by agitation for 2 min at 3,500 rpm with inverting every 10 sec. A magnetic rod was inserted into the spin tip, and the complex was adsorbed and collected on the spin tip over 60 sec and then moved to the fourth row/fourth plate. After removing the magnet rod from the spin tip in 50 mM Tris-HCl pH 8.0, the complex was washed by agitation for 2 min at 3,500 rpm with inverting every 10 sec. A spin tip was inserted into the fourth row/fourth plate, and the complex was washed by agitating for 2 min at 3,500 rpm with inverting every 10 sec. A magnetic rod was inserted into the spin tip, and the complex was adsorbed and collected on the spin tip over 60 sec and then moved to the fifth row/fifth plate.

### (2-3) Washing of complexes using TBST and 50 mM Tris-Cl pH 8.0

After removing the magnet rod from the spin tip, the complex was washed in 50 mM Tris-Cl pH 8.0 by agitation for 2 min at 3,500 rpm with inverting every 10 sec and released and suspended in a solution. Thereafter, the same procedures as the manual extraction method for filter paper-bound proteins as described above were carried out until LC-MS analysis.

### II. Results and Discussion

Figure 1 shows a schematic diagram of protein extraction by the SCP method and the preparation method for filter paper-bound proteins. As described in detail in Nakajima et al., Journal of Proteome Research, 2020, 19, 2821-2827, the SCP method enables efficient removal of hydrophilic proteins such as hemoglobin, albumin, and immunoglobulins, which account for the majority of proteins in blood, by precipitating hydrophobic proteins in 100 mM sodium carbonate aqueous solution, thereby significantly increasing the number of identified proteins. This operation involves the precipitation of hydrophobic proteins; however, since the resulting precipitate is small and difficult to visually recognize, and also slippery, there is a risk that it may be inadvertently aspirated and discarded along with the supernatant during its removal. Therefore, this procedure needs to be handled by well-trained operators with great care (Figure 1A). In this study, aiming to establish a simpler method for extracting proteins from DBS, extraction of filter paper-bound proteins was attempted. The filter paper-bound proteins were successfully isolated by washing the pulpy filter paper through agitation and centrifugation in a solution using TBST or Tris buffer, conventionally used in biochemical experiments (Fig. 1B). Since the precipitate of the pulpy filter paper was large and easy to visually recognize, there was a low risk of it being inadvertently aspirated and discarded, and even untrained operators were able to handle it successfully.

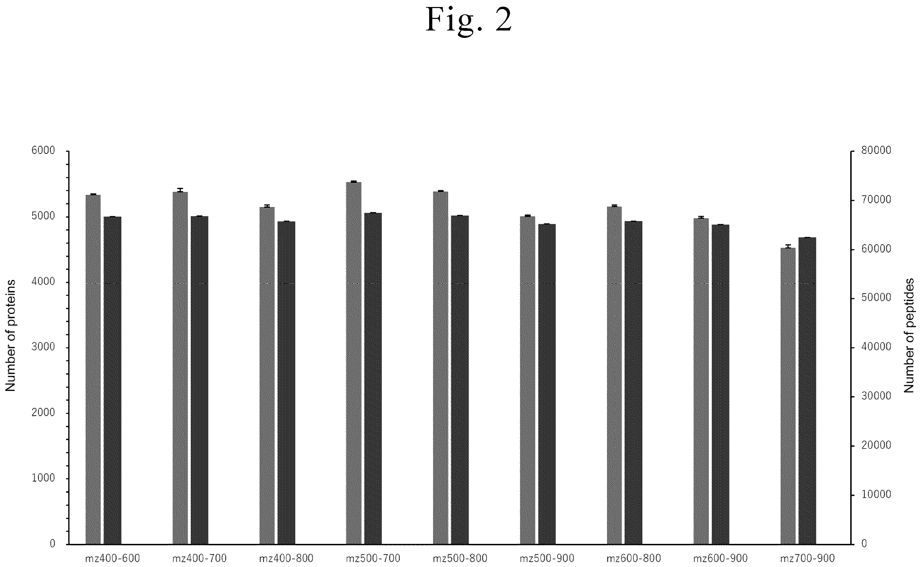

For performing DIA-MS analysis, the window width was optimized for the identification of FPBP. As a result, both the number of identified proteins and the number of identified peptides were the highest in the m/z range of 500-700 (Fig. 2). In the following experiments, DIA-MS analysis was carried out under this condition.

Fig. 3 shows a comparison of the number of proteins identified by proteomic analysis between the SCP method and the filter paper-bound protein (FPBP) extraction method. The number of identified proteins was 3352 by the SCP method and 4782 by the FPBP method, indicating a 1.4-fold increase with the FPBP method. Proteins that were not detected by the SCP method but were detected by the FPBP method (either manual or automated, or both) are listed in Table 1. The number of proteins identified in proteomic analyses of DBS without prior fractionation conducted by other groups remained below 1000 (Eshghi et al., Molecular and Cellular Proteomics, 19(3), 540-553, 2020; Nieman et al., Proteomes, 8(1), 4, 2020), which demonstrates the superiority of the method of the present invention. While 3091 proteins were commonly identified by both the SCP method and the filter paper-bound protein method, 1691 proteins were unique to the FPBP method, and 161 proteins were unique to the SCP method. Therefore, the FPBP method was considered more advantageous as a method capable of detecting a wide variety of proteins using a simplified procedure.

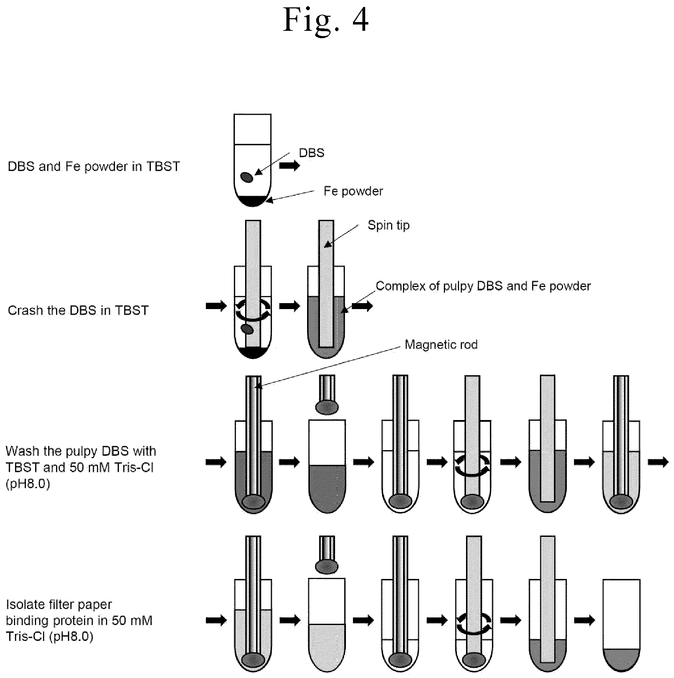

Manual preparation is labor-intensive and makes it difficult to process a large number of samples. Moreover, due to variability in handling by operators, it may be challenging to achieve consistent preparation. Therefore, to further stabilize and expedite the FPBP method and enable processing of a large number of samples, the present inventors attempted to automate the procedure by applying a nucleic acid automated extraction system. A schematic of the automated extraction method is shown in Figure 4. By adding iron powder during the crushing of DBS in TBST, a complex of iron powder and filter paper was formed during the crushing of DBS via the rotational movement of the spin chip. It was confirmed that, by inserting a magnetic rod into the spin chip, this complex could be magnetically bound to the spin chip and easily and completely recovered. The recovered complex was transferred to a fresh solution, and washing steps composed of suspension and recovery were repeated until the reddish-brown color derived from hemoglobin became colorless and transparent. Furthermore, in the latter stage of the washing steps, the buffer was replaced with 50 mM Tris-Cl pH 8.0, which is compatible with trypsin digestion, allowing the final product to be directly digested by trypsin. Once reagents were set in the 96-well plate, a series of processes including DBS pulping, formation of the complex of pulpy DBS and iron powder, and washing and isolation of the complex could be performed by the automated extraction instrument in approximately 30 minutes. Up to eight samples could be processed at once using the Maelstrom 8 Autostage, and up to 96 samples could be processed at once using the Maelstrom 9610. By performing automated processing with the instrument, more stable handling with fewer errors was achieved compared to manual operations. This is important for stably and rapidly processing a large number of samples in applications such as newborn mass screening and cohort studies.

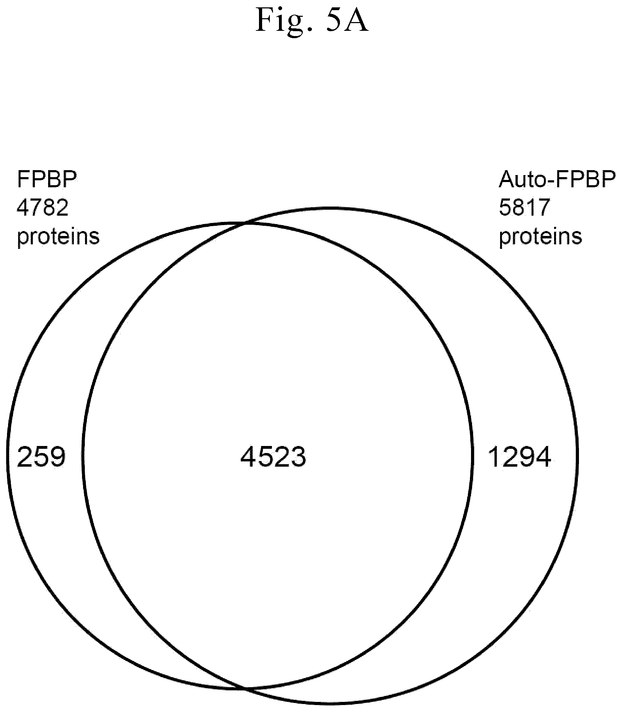

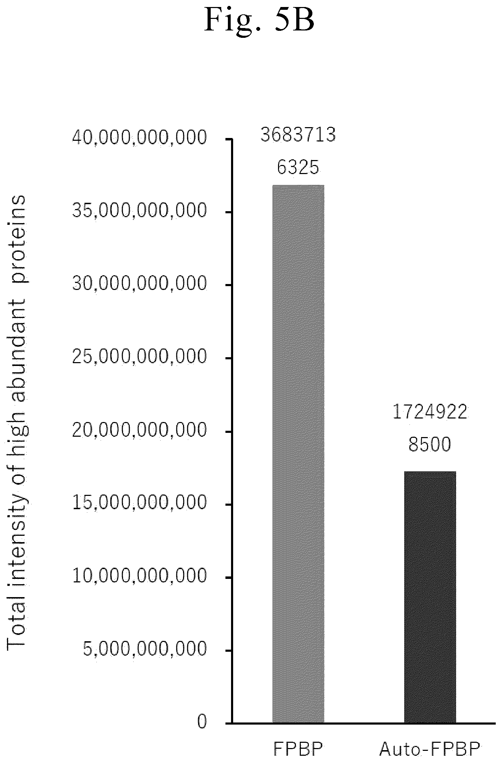

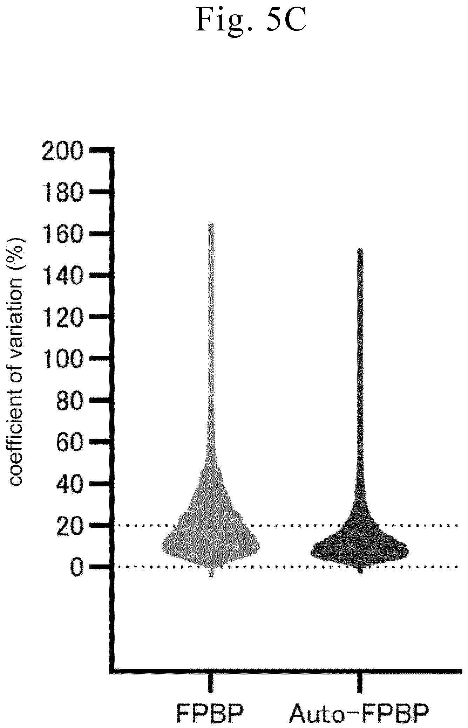

Figure 5 shows a comparison of the number of proteins identified using the FPBP method between manual and automated processing. In the manual method, 4782 proteins were identified, whereas in the automated method, 5817 proteins were identified, indicating a 1.2-fold increase in the number of identified proteins by the automated method (Figure 5A). To explore the reason for this increase, the total ion intensities of representative highly abundant blood proteins that interfere with proteomic depth (HBB, HBA2, IGHG1, APOA1, and ALB) were compared. The results showed that in the automated method, the total ion intensity of these proteins was reduced to less than half compared to the manual method, suggesting that abundant hydrophilic blood proteins were more effectively removed through washing operations (Figure 5B). Furthermore, to assess the stability of protein detection and quantification between the manual and automated methods, CV values of ion intensities were examined for individual proteins (proteins detected in 3 or more out of n=4 replicates were included: 4376/4782 in the manual method and 5463/5817 in the automated method). As a result, 57% (2,495/4,376) of the proteins identified by the manual method had CV values <20%, whereas this proportion significantly increased to 81% (4,438/5,463) in the automated method, indicating more stable detection and quantification of proteins using the automated method (Figure 5C).

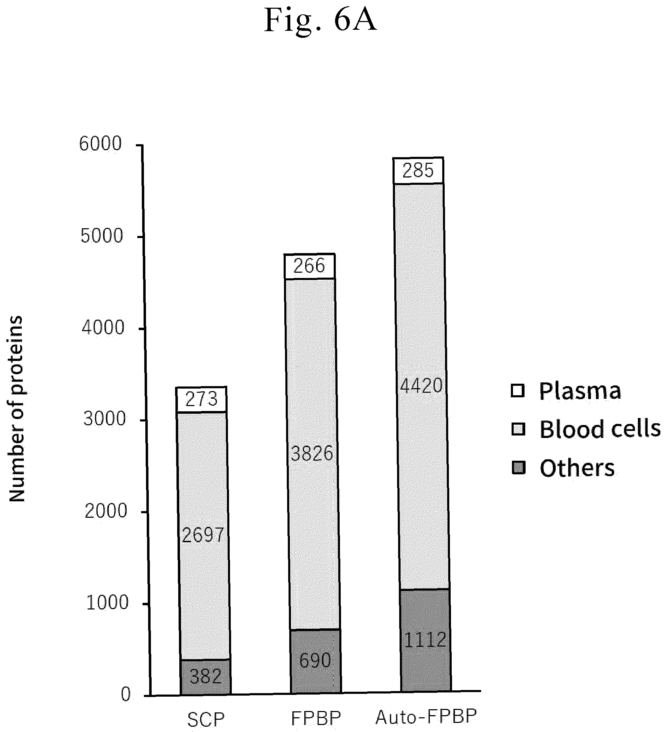

The characteristics of proteins identified by the SCP method, the FPBP method (manual), and the FPBP method (automated) were compared. The results are shown in Figures 6A to 6C. First, the origin of the proteins identified by each method in the blood was examined (Figure 6A). The number of plasma-derived proteins identified was 273, 266, and 285, respectively, showing nearly the same numbers across all methods. However, regarding the number of blood cell-derived proteins identified was 2697, 3826, and 4420, respectively, showing that the FPBP method (manual) and FPBP method (automated) identified approximately 1.4-fold and 1.6-fold more proteins, respectively, than SCP method. These results suggest that filter paper has a higher capacity to absorb blood cell-derived proteins.

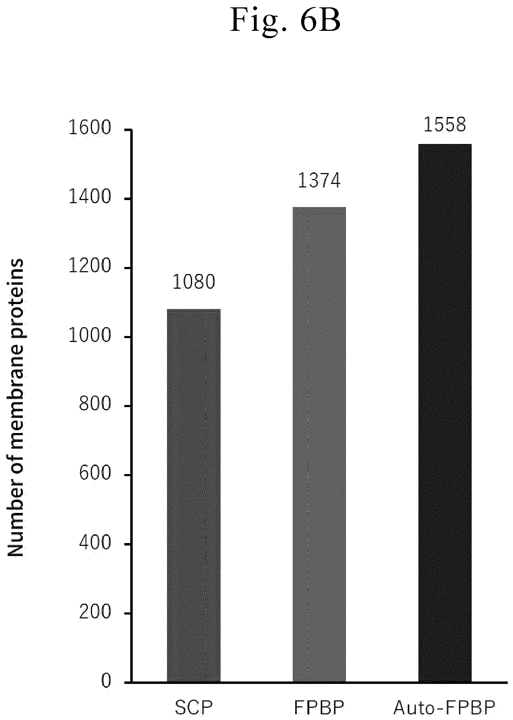

The number of membrane proteins identified was 1080 for the SCP method, 1374 for the FPBP method (manual), and 1558 for the FPBP method (automated), indicating that the FPBP method (manual) and FPBP method (automated) identified approximately 1.3 and 1.4 times more membrane proteins, respectively, than the SCP method (Figure 6B). The ratio of membrane proteins among all identified proteins was highest in the SCP method at 32% (1080/3352), compared to 28% (1374/4782) in the FPBP method (manual) and 20% (1558/5817) in the FPBP method (automated), suggesting that the SCP method has a greater enrichment effect for membrane proteins than the FPBP method. The number of membrane proteins identified was highest with the FPBP method (automated). The main component of filter paper is cellulose. Cellulose acquires hydrophobic properties and becomes insoluble as a result of the linear polymerization of hydrophilic β-glucose units via β-1,4 glycosidic linkages. Filter paper is considered to be capable of adsorbing proteins with various properties due to its combination of hydrophilic and hydrophobic characteristics.

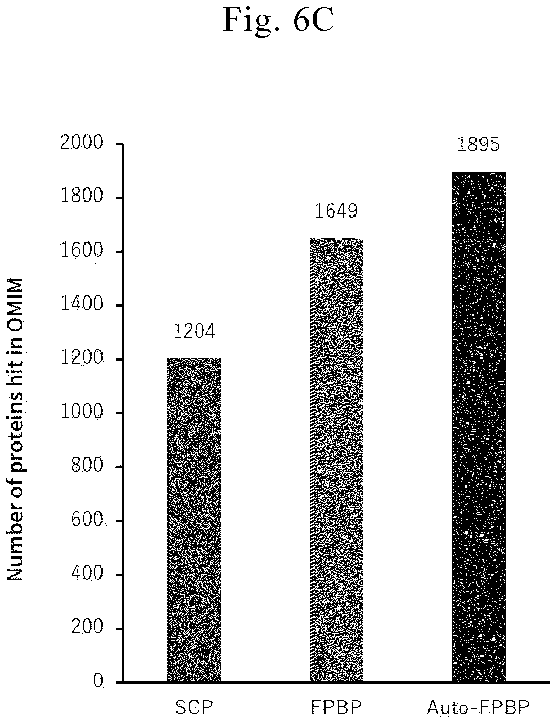

To investigate whether disease-causing proteins could be detected, a cross-reference with the Online Mendelian Inheritance in Man (OMIM) database was performed. As a result, the number of OMIM-hit proteins identified significantly increased from 1204 with the SCP method to 1649 with the FPBP method (manual), and further to 1895 with the FPBP method (automated) (Figure 6C). OMIM-hit proteins that were not detected by the SCP method but were detected by either or both of the manual and automated FPBP methods are listed in Table 2-1 through Table 2-7. Additionally, among the proteins detected by the FPBP method (including those also identified by the SCP method), a subset that is expected to be applicable to newborn screening is summarized in Table 3. These findings suggest that the FPBP method has the potential to be applied to the screening of a broader range of diseases.

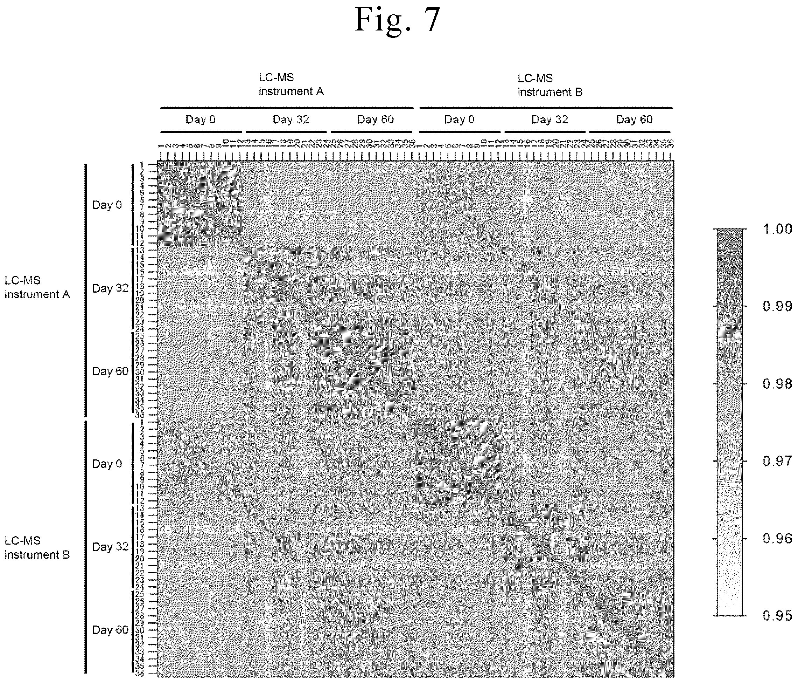

Reproducibility is crucial for disease screening. Therefore, the reproducibility of the automated FPBP method was evaluated across different days and instruments (Figure 7). Samples were independently prepared using the automated FPBP method on three separate occasions more than four weeks apart, with 12 samples each time. These samples were then subjected to proteome analysis using DIA-MS on two LC-MS instruments of the same specifications. Pearson's r values across all combinations was confirmed for the obtained data. Samples prepared on the same day tended to show high correlation, with the Pearson's r values across all combinations being an average of 0.98, a median of 0.98, a maximum of 0.99, and a minimum of 0.96. Considering the variability in the conditions including amount of blood retained in each DBS, subsequent protein extraction, peptidation, desalting, and LC-MS, inter-day and inter-device reproducibility was considered to be high. In newborn screening, it is necessary to analyze DBS samples received daily and report results within a limited time frame. Thus, the high reproducibility of the automated FPBP method is considered to be of great importance.

### [Experimental Example 1] Separation of filter paper and gel fragments by magnetism

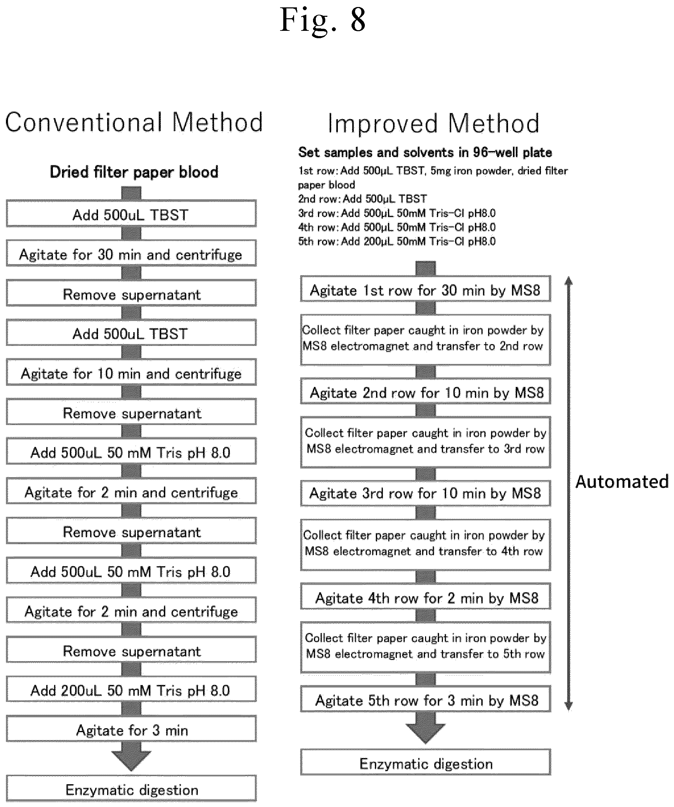

To the test tube, 1 ml water, iron powder (particle diameter 3-5 µm, 5 mg), and filter paper (3.2 mm diameter, 1.5 mg) or a gel fragment (1 mm x 5 mm x 1 mm, 5 mg) were added and agitated. When a magnet (cylindrical neodymium magnet (5 mm outer diameter, 5 mm thickness), surface magnetic flux density (T) = 0.4, and attractive force (N) = 9.29) was brought close to the side of the tube, the swelled filter paper having iron powder incorporated inside, and the gel fragment having iron powder attached to its surface, were absorbed by the magnet (Fig. 8).

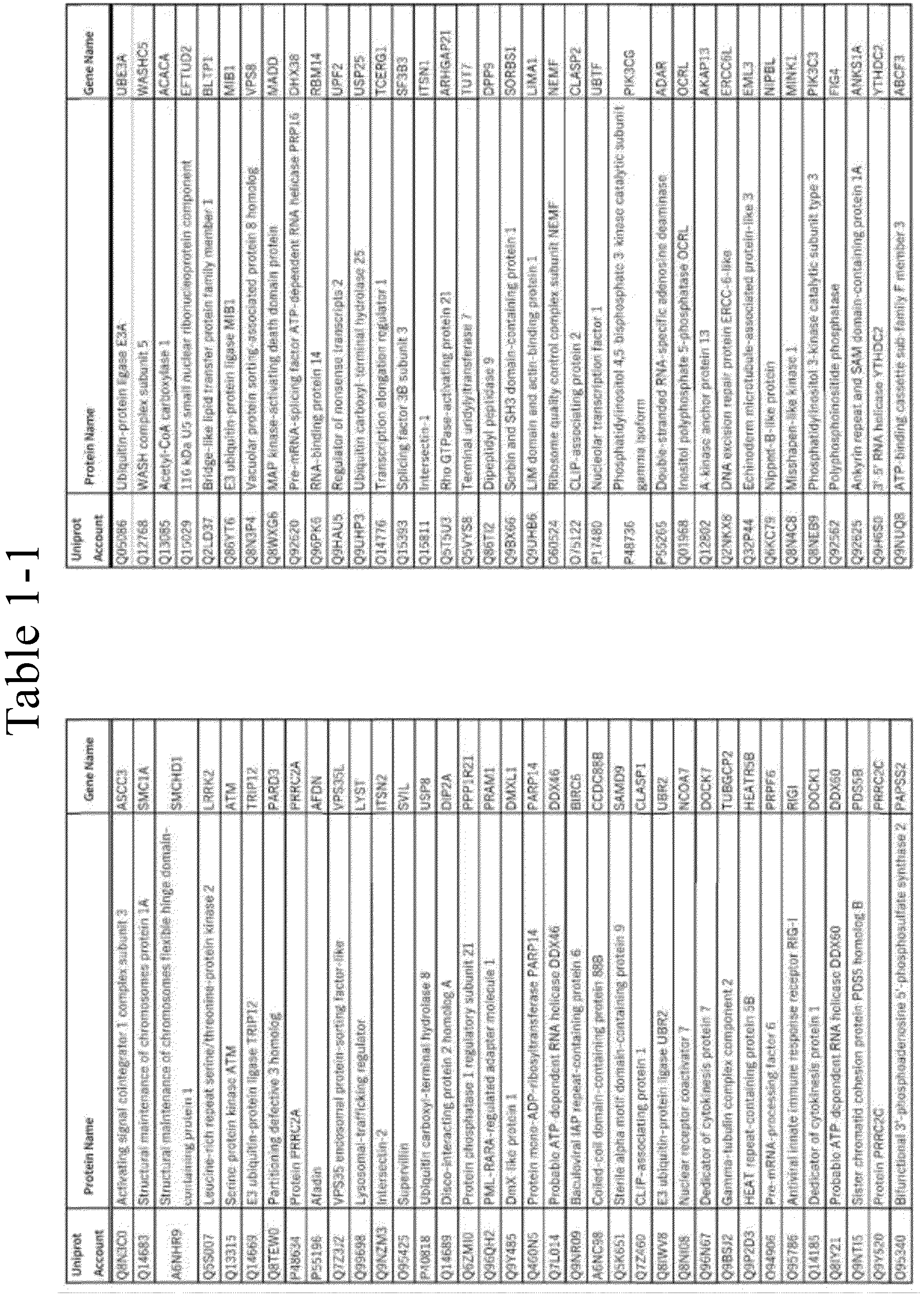

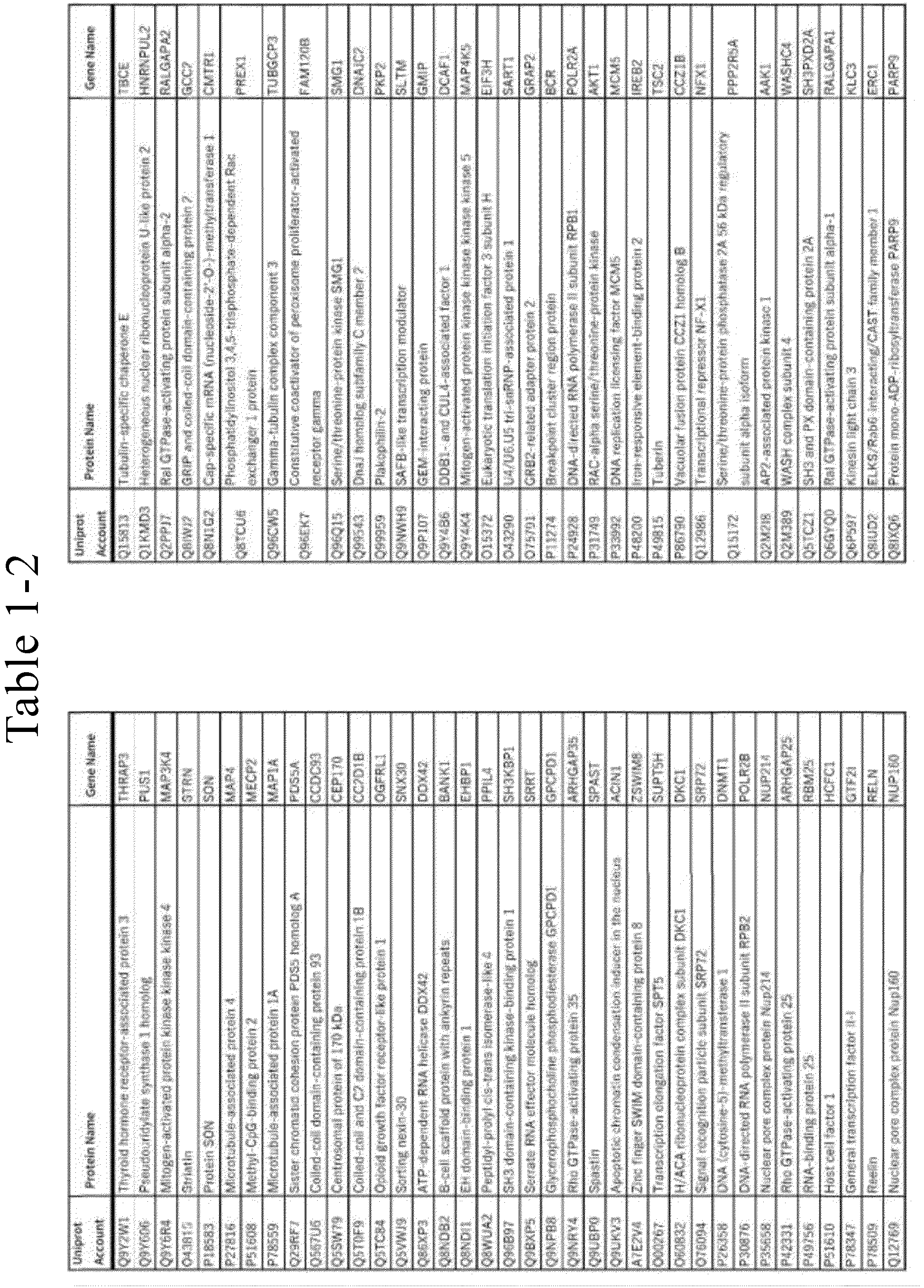

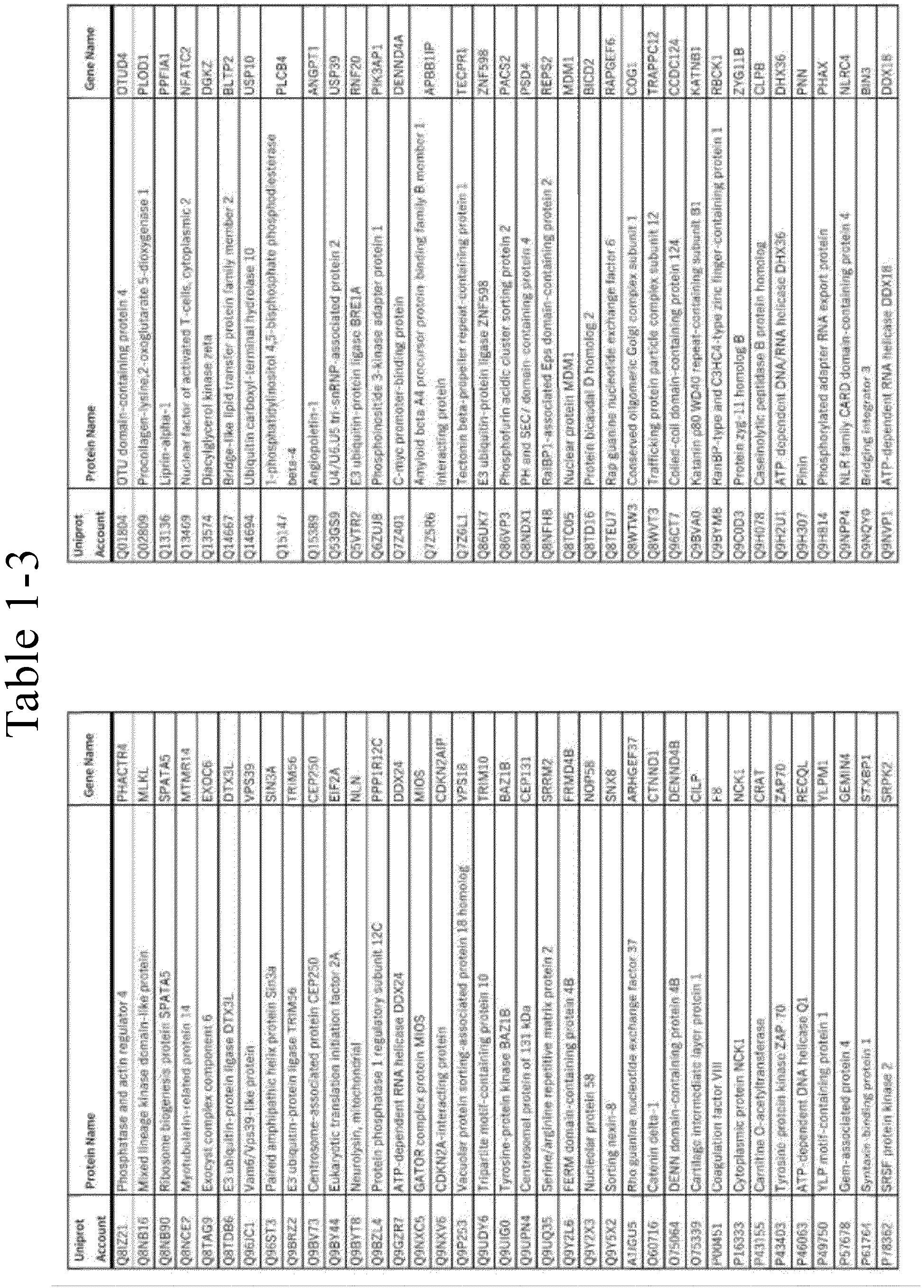

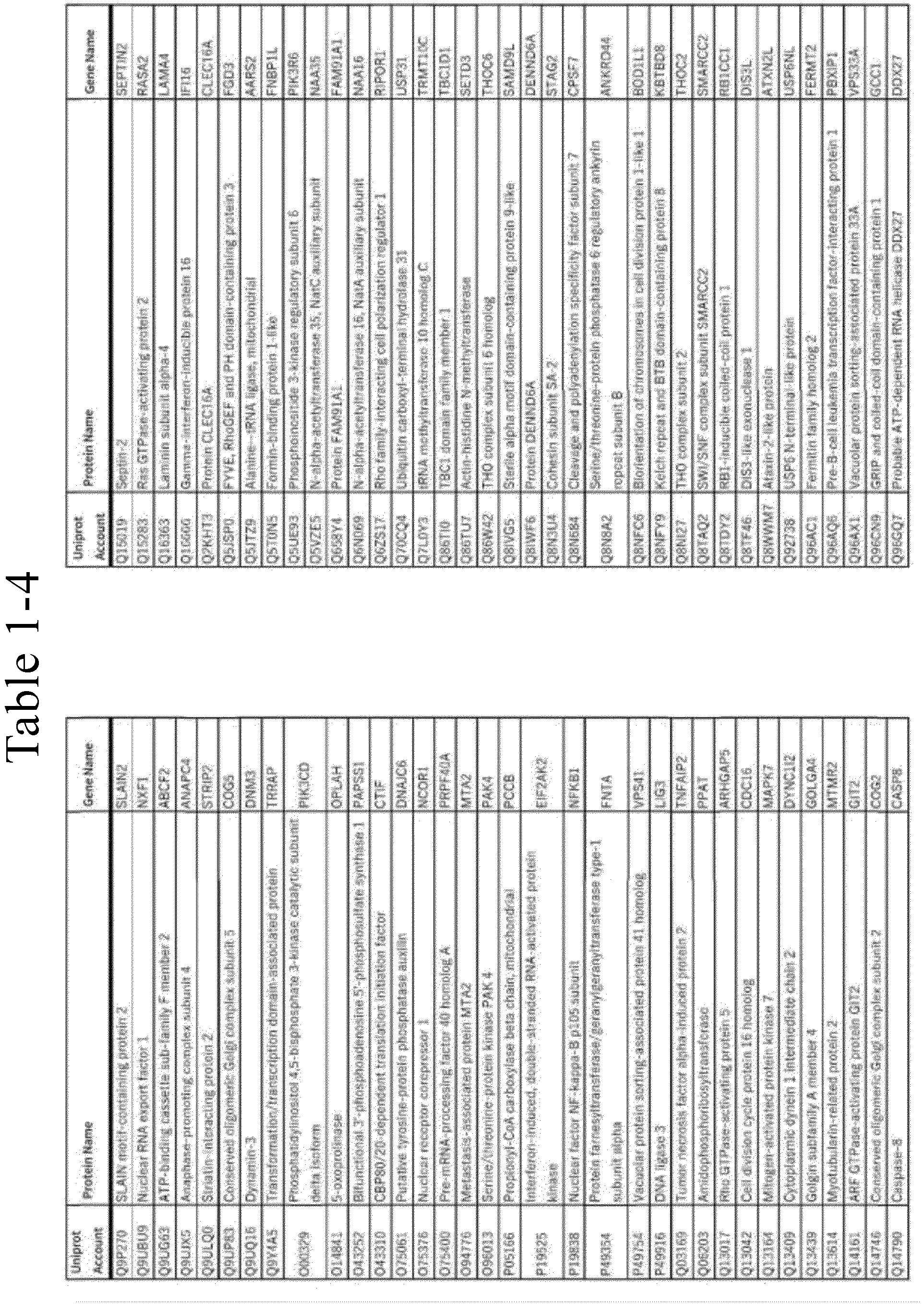

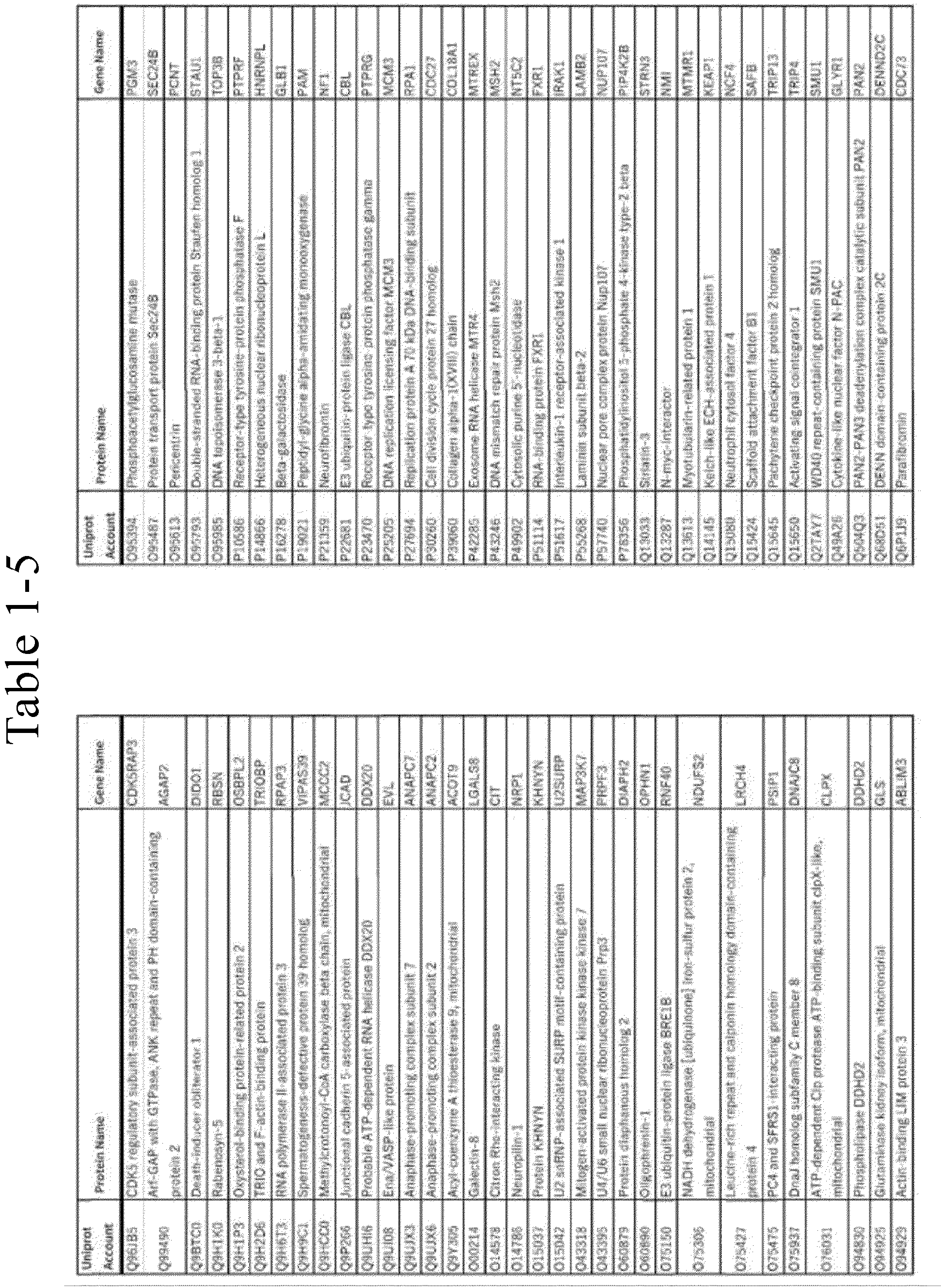

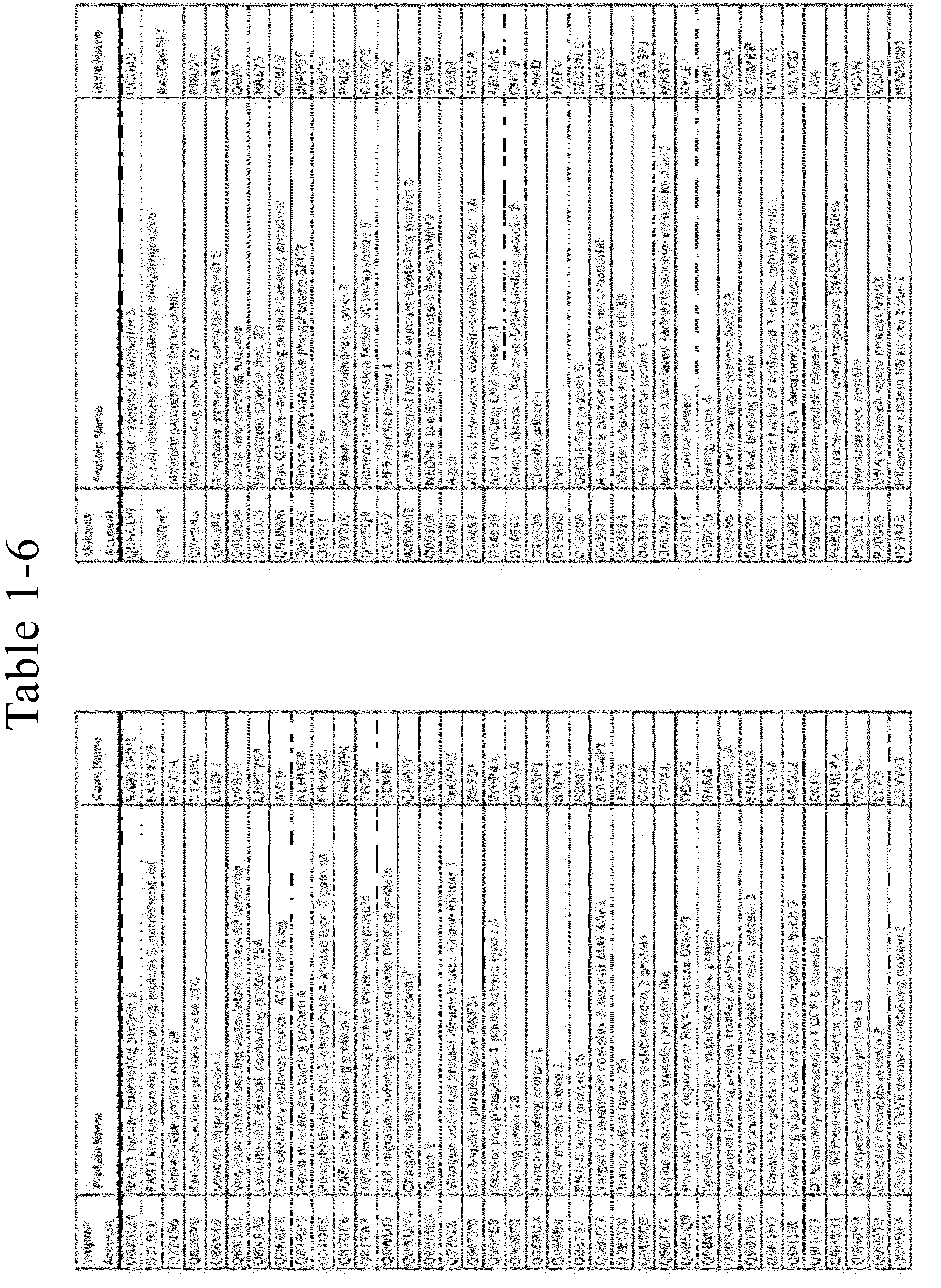

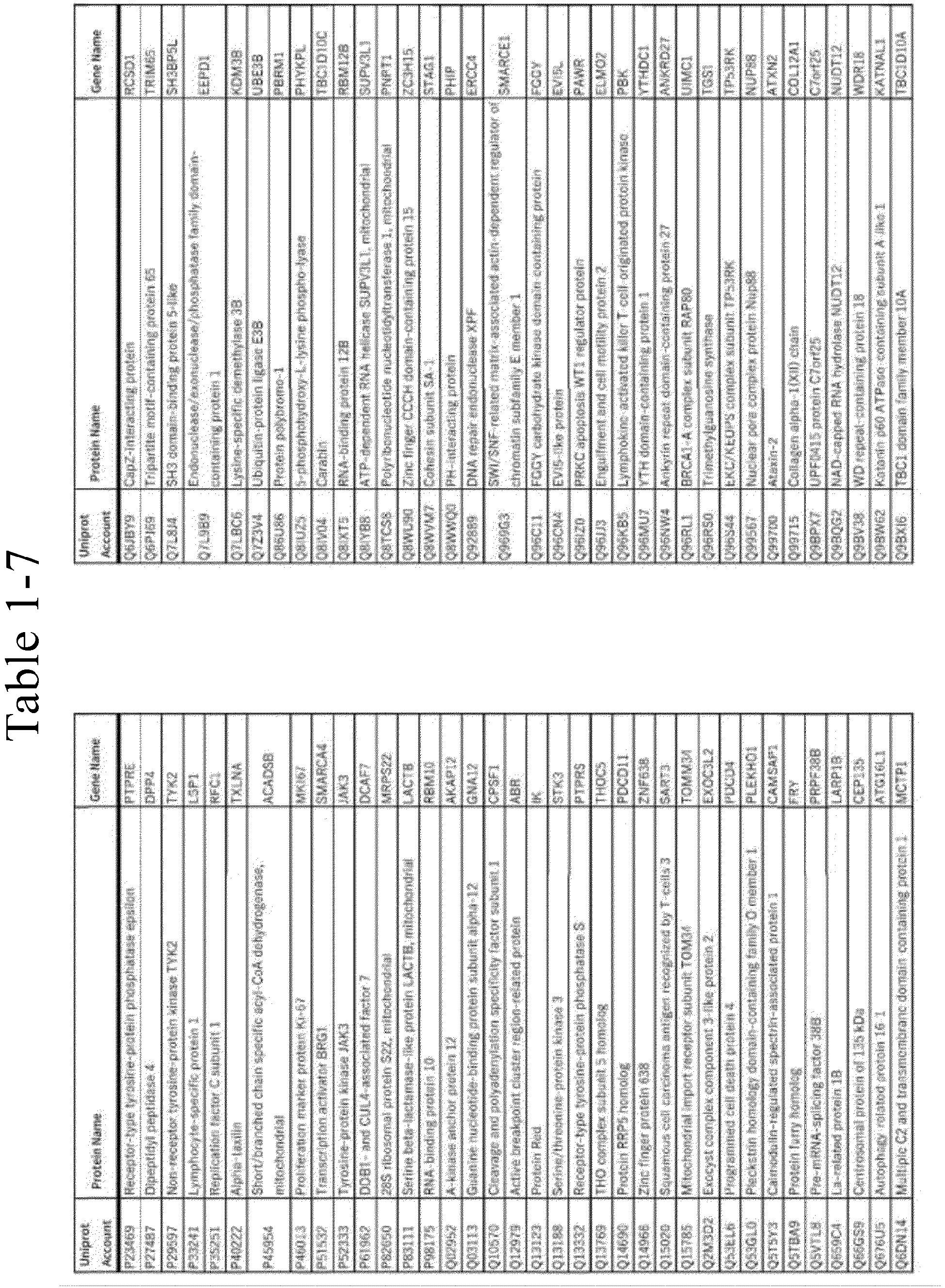

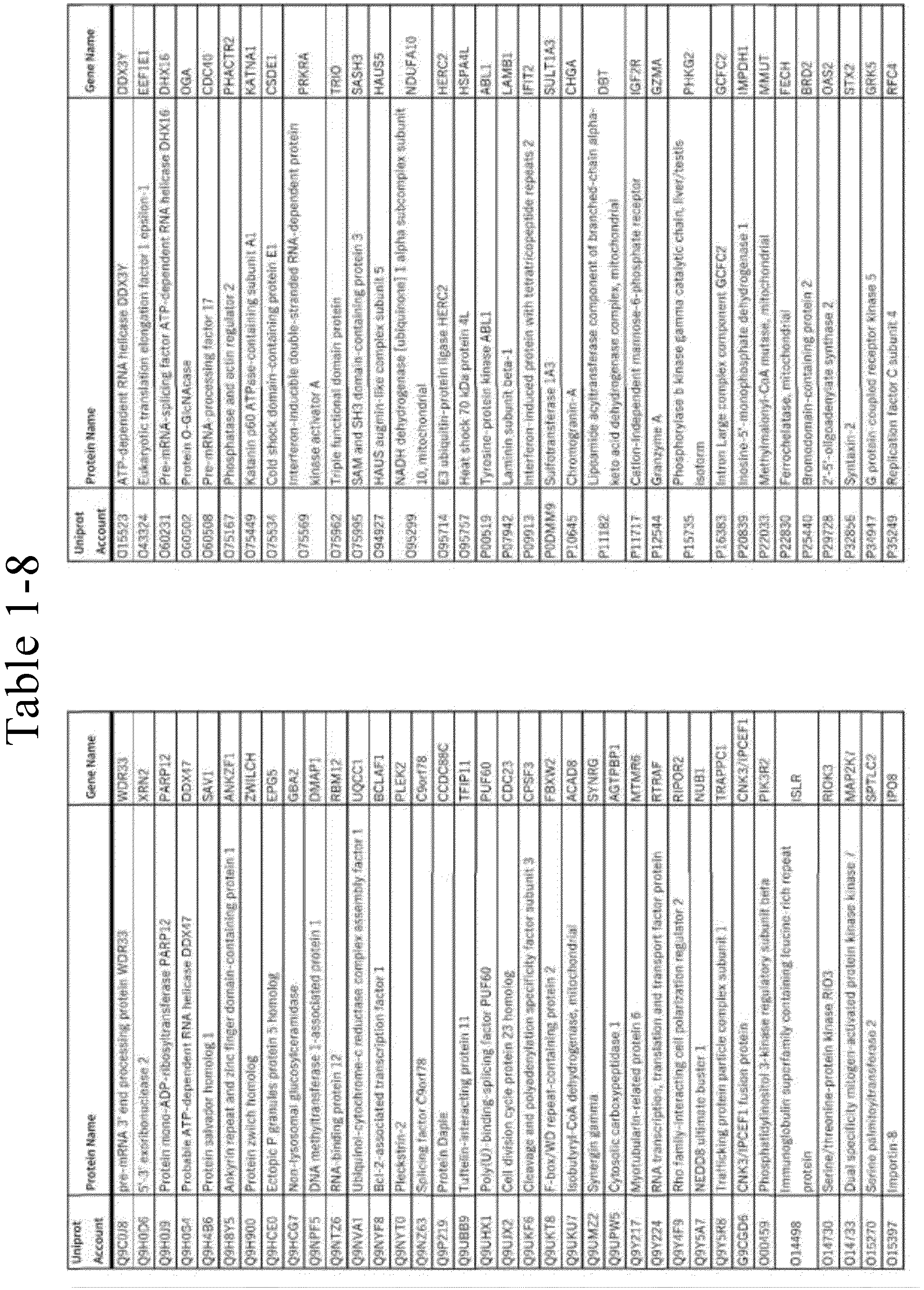

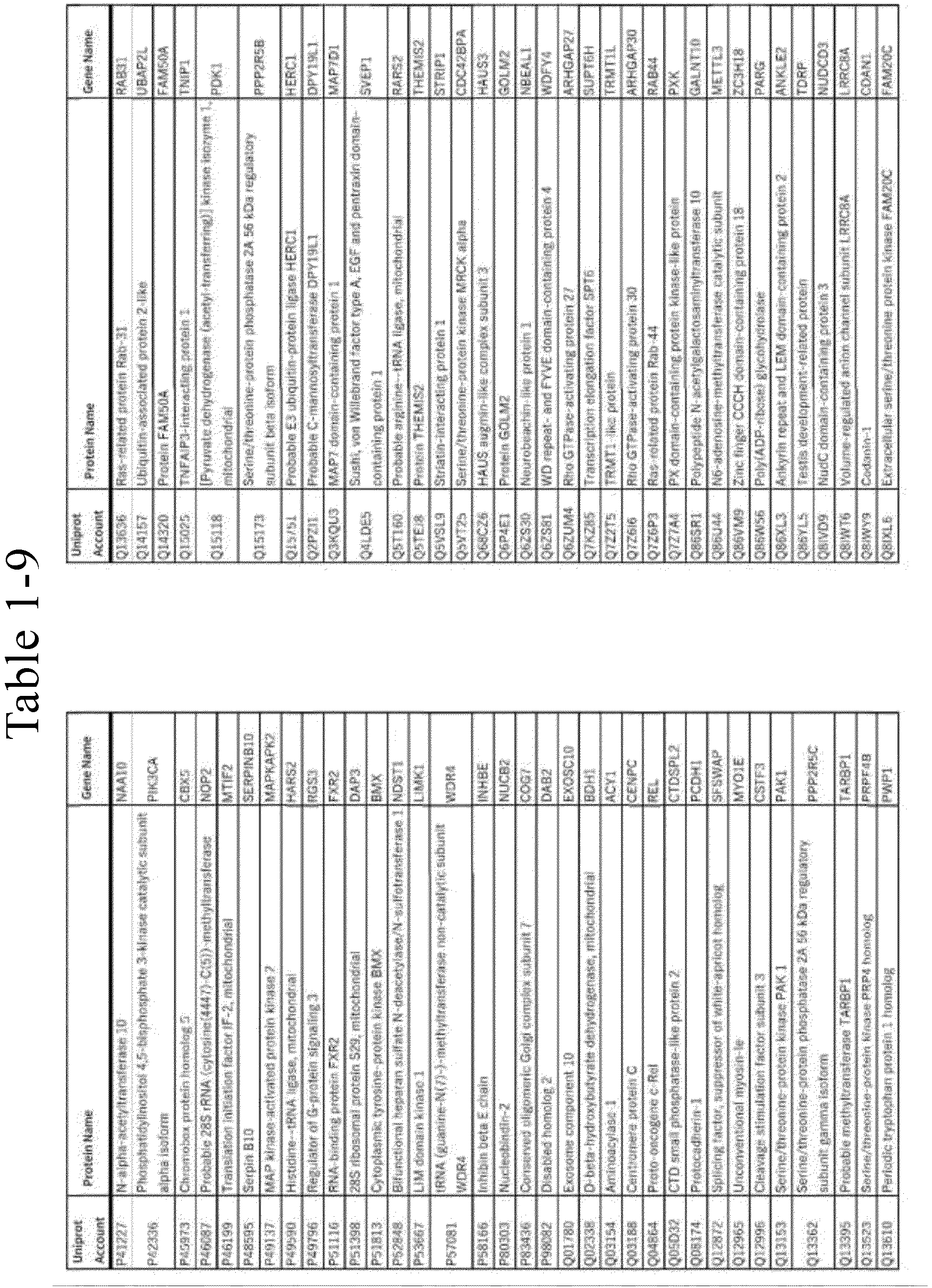

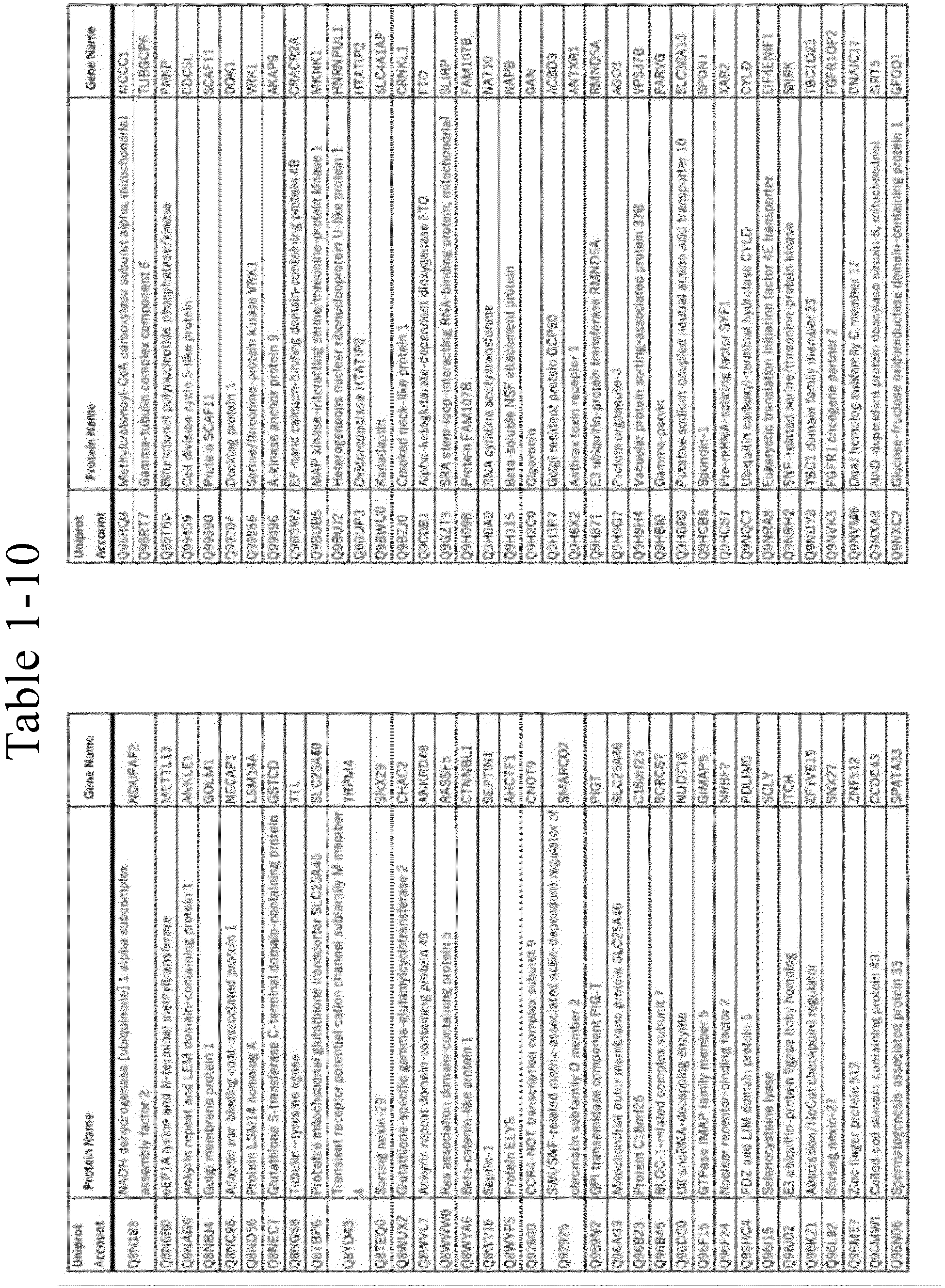

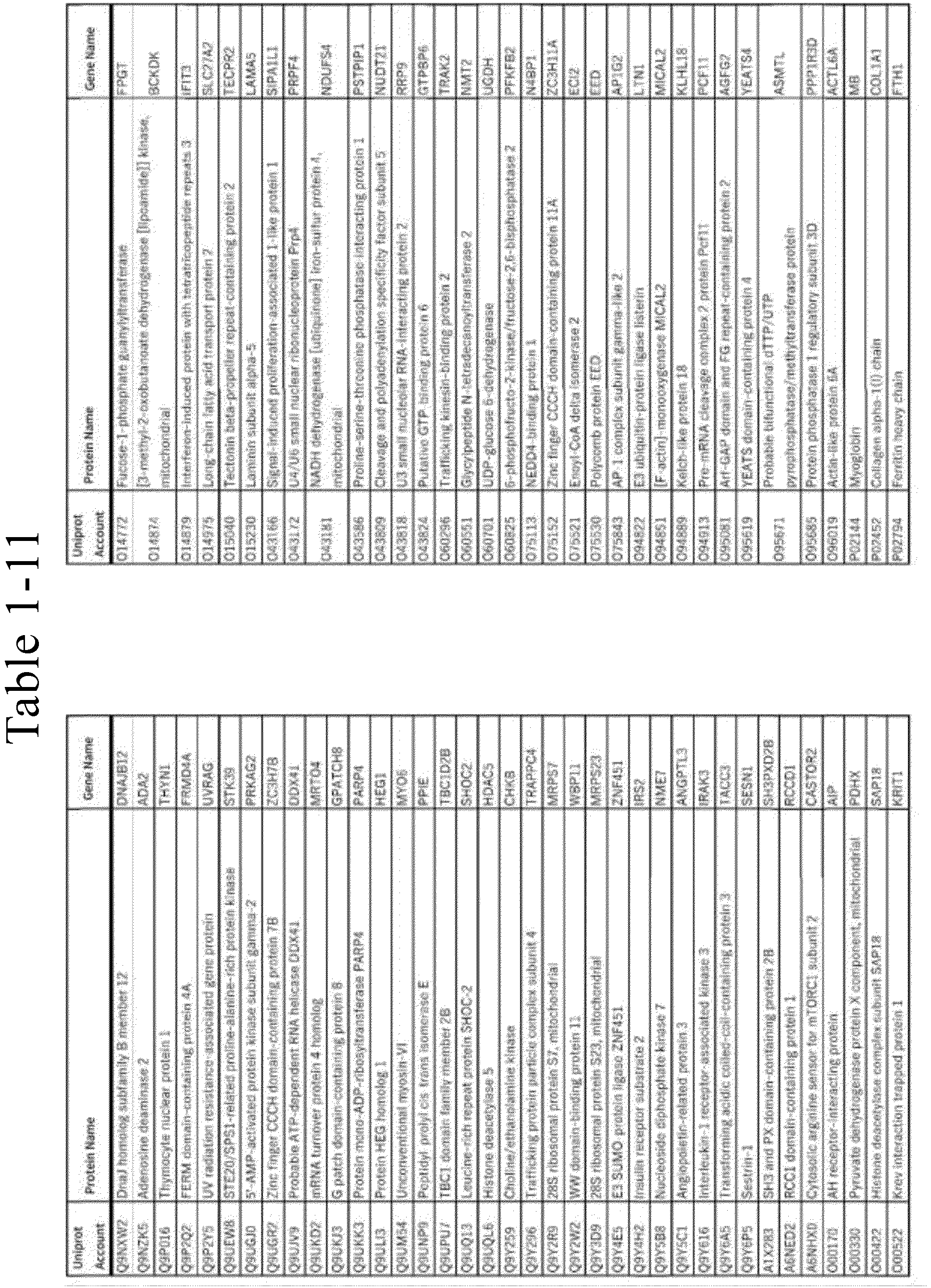

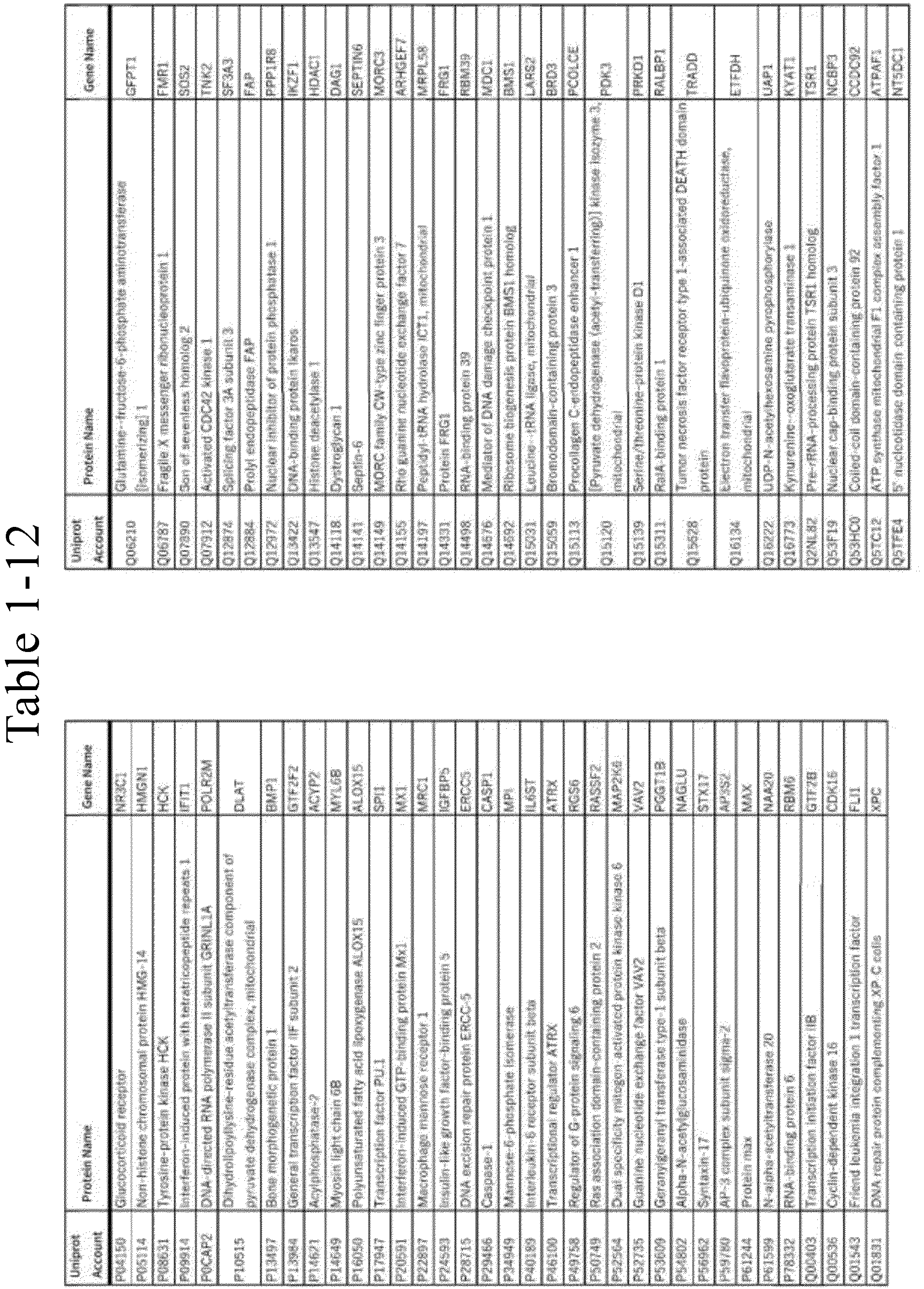

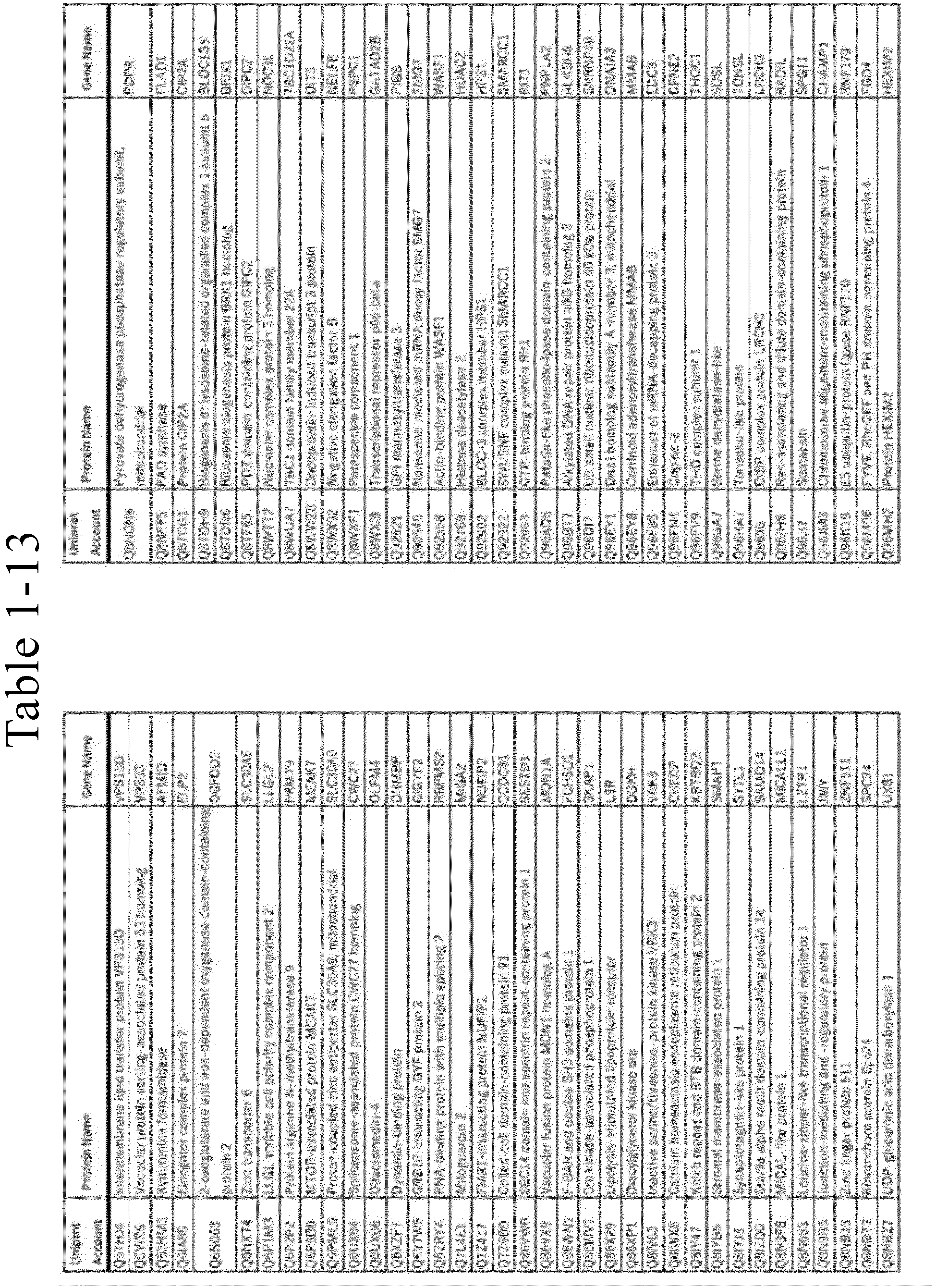

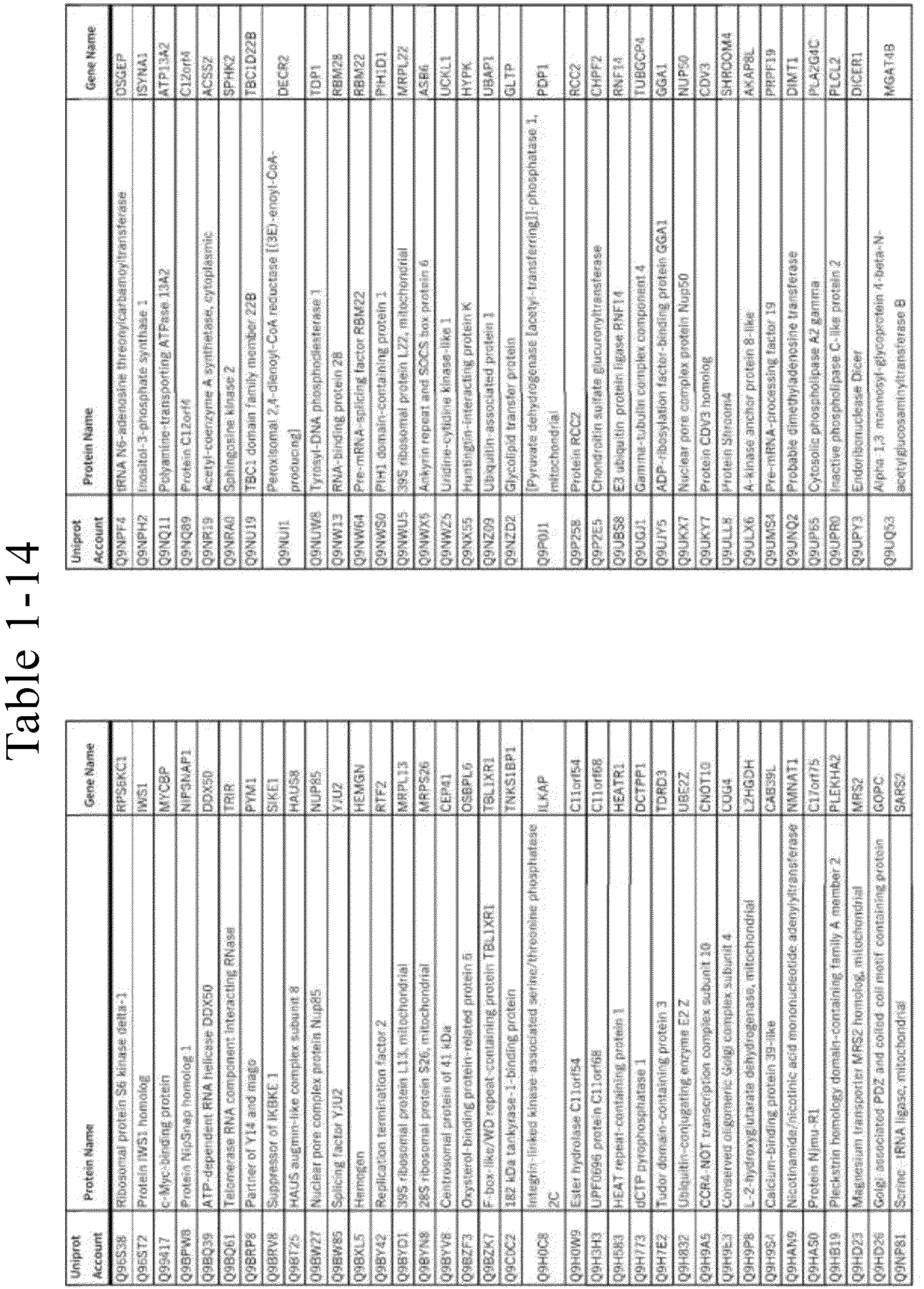

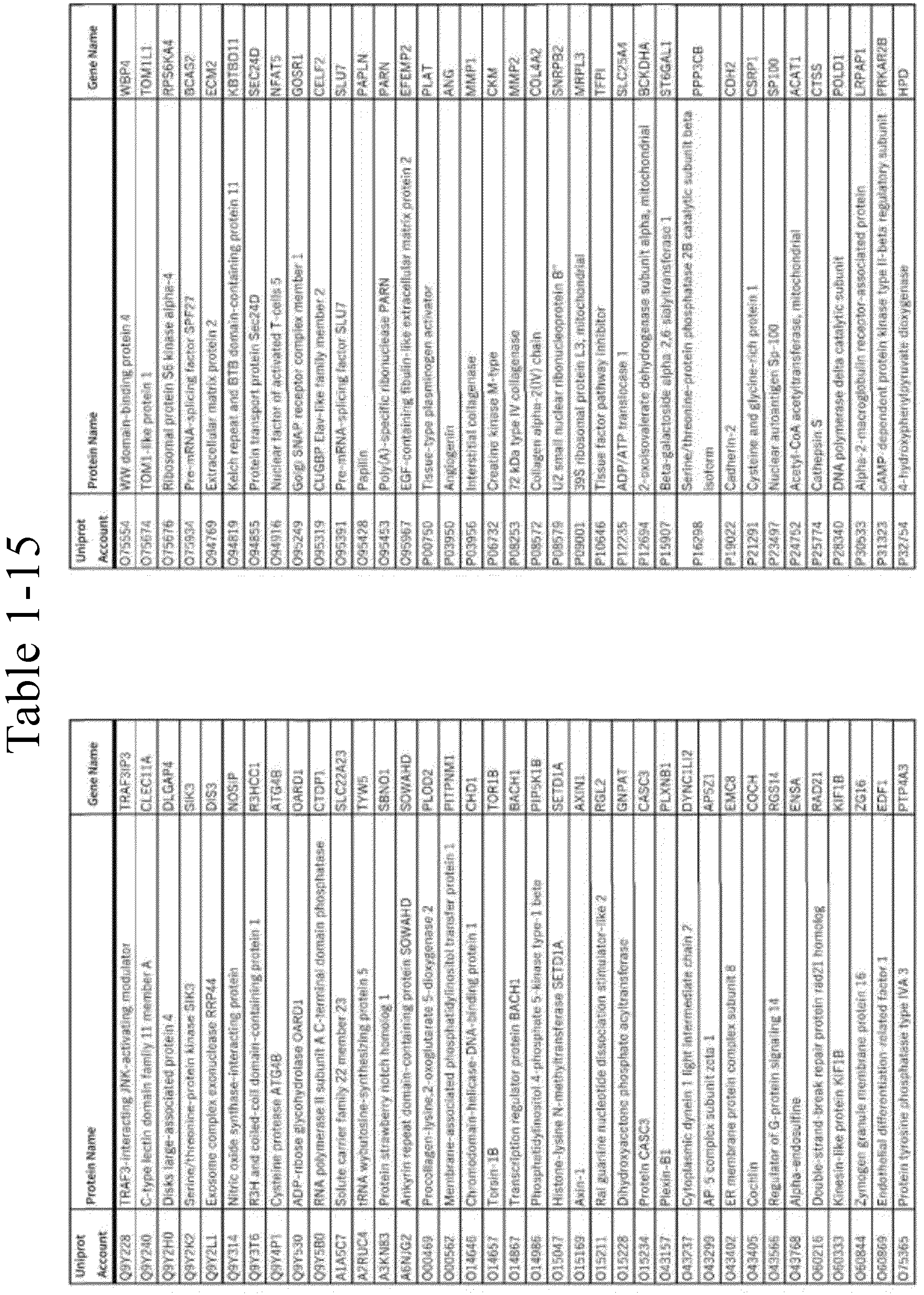

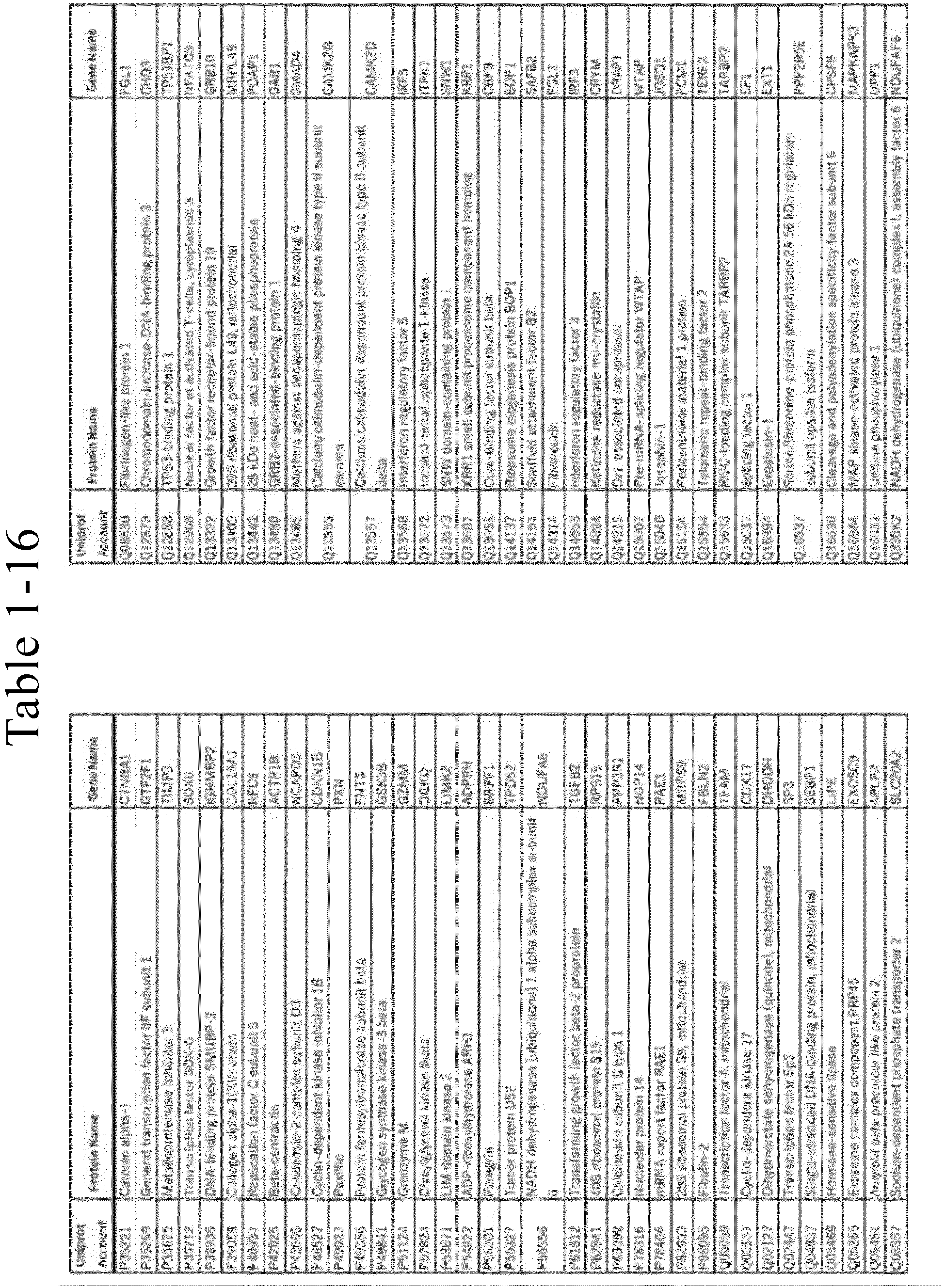

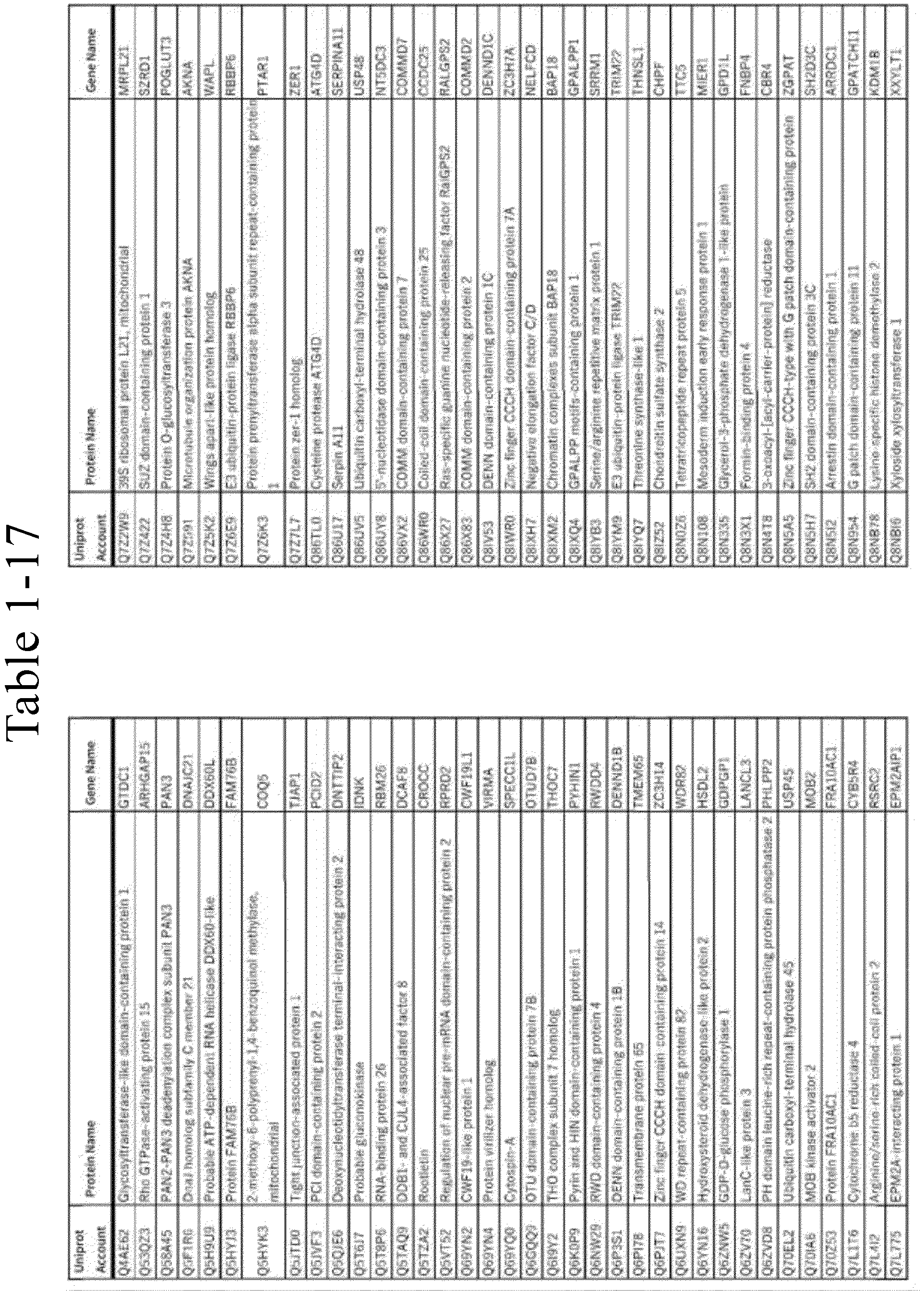

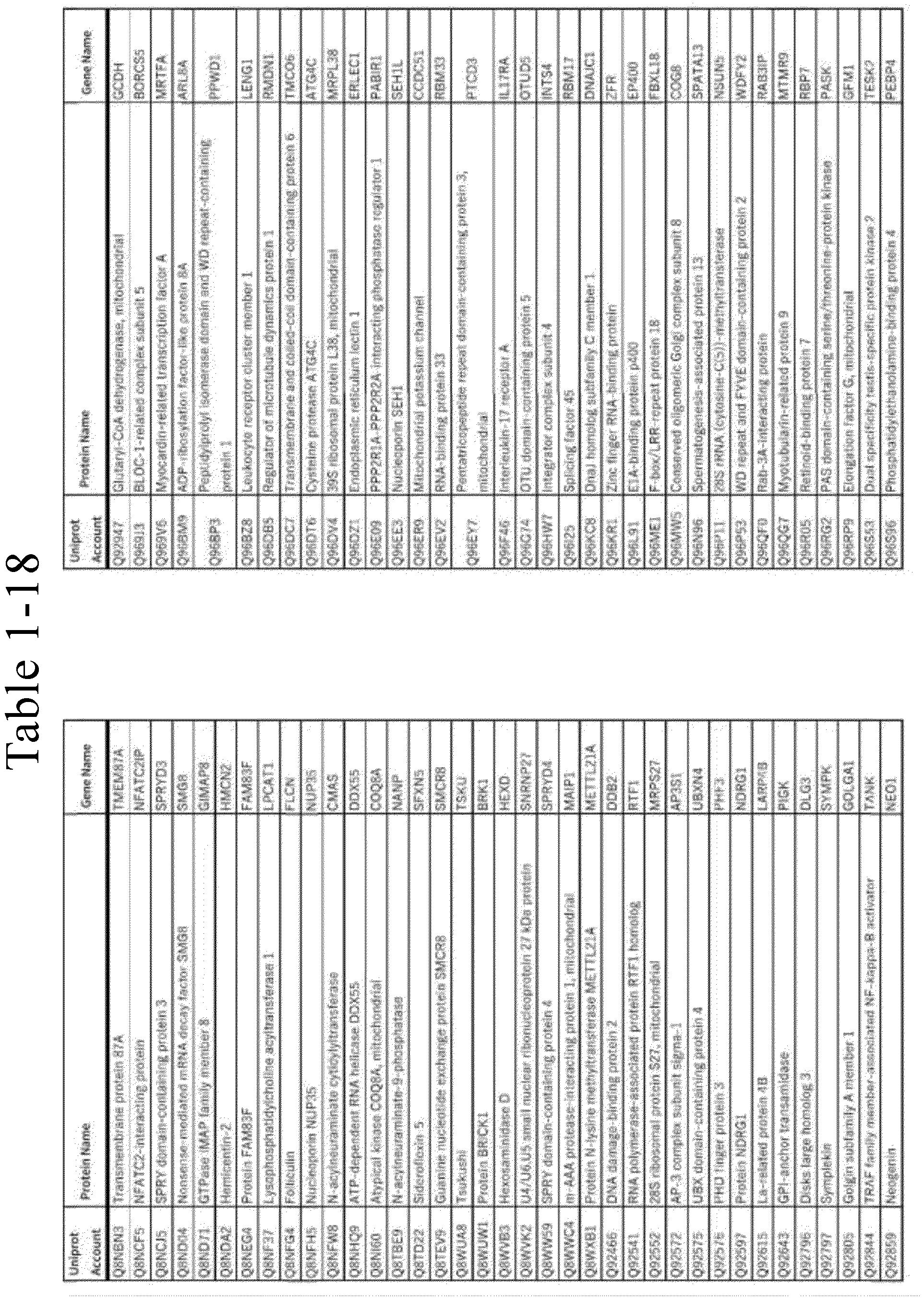

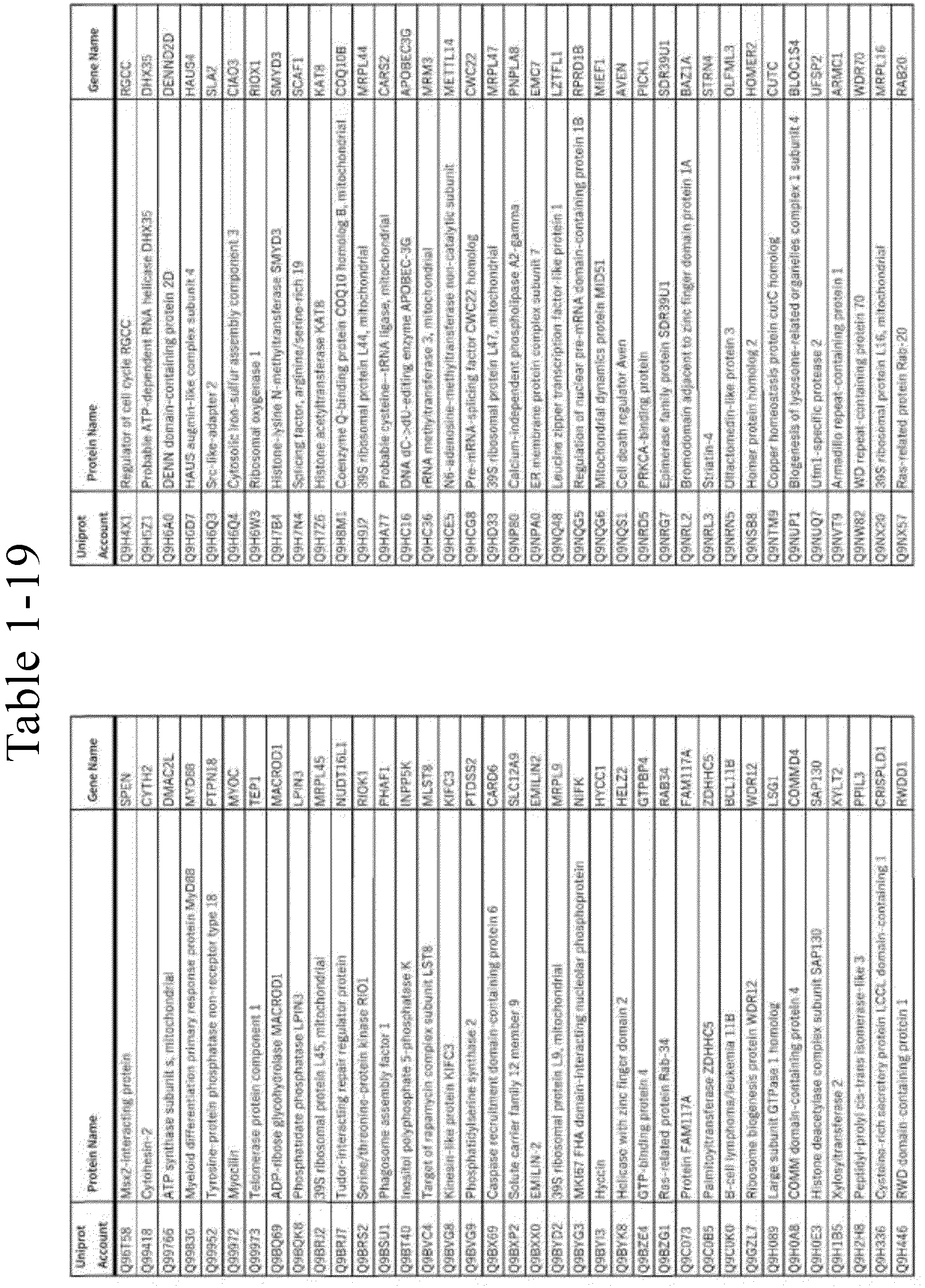

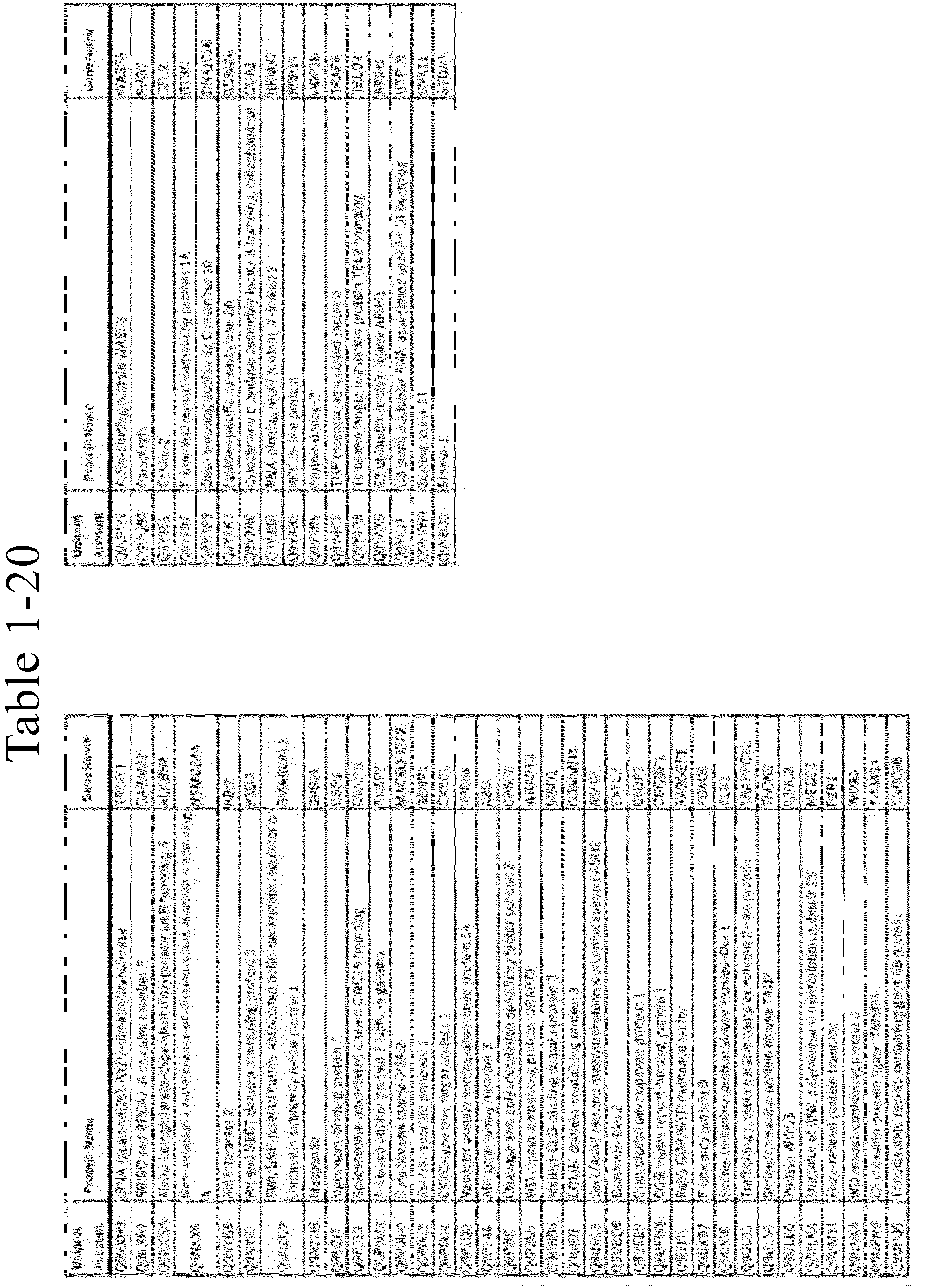

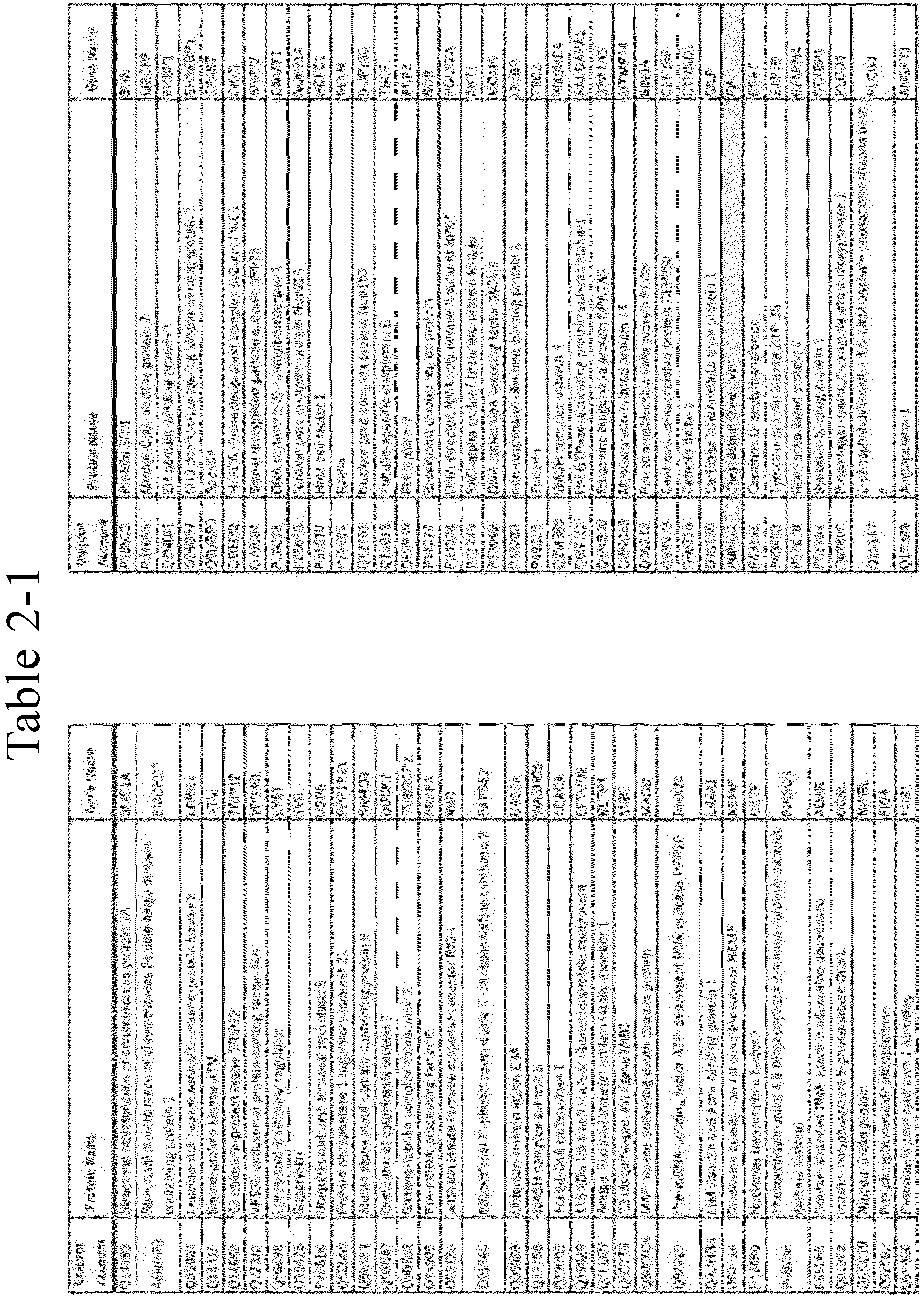

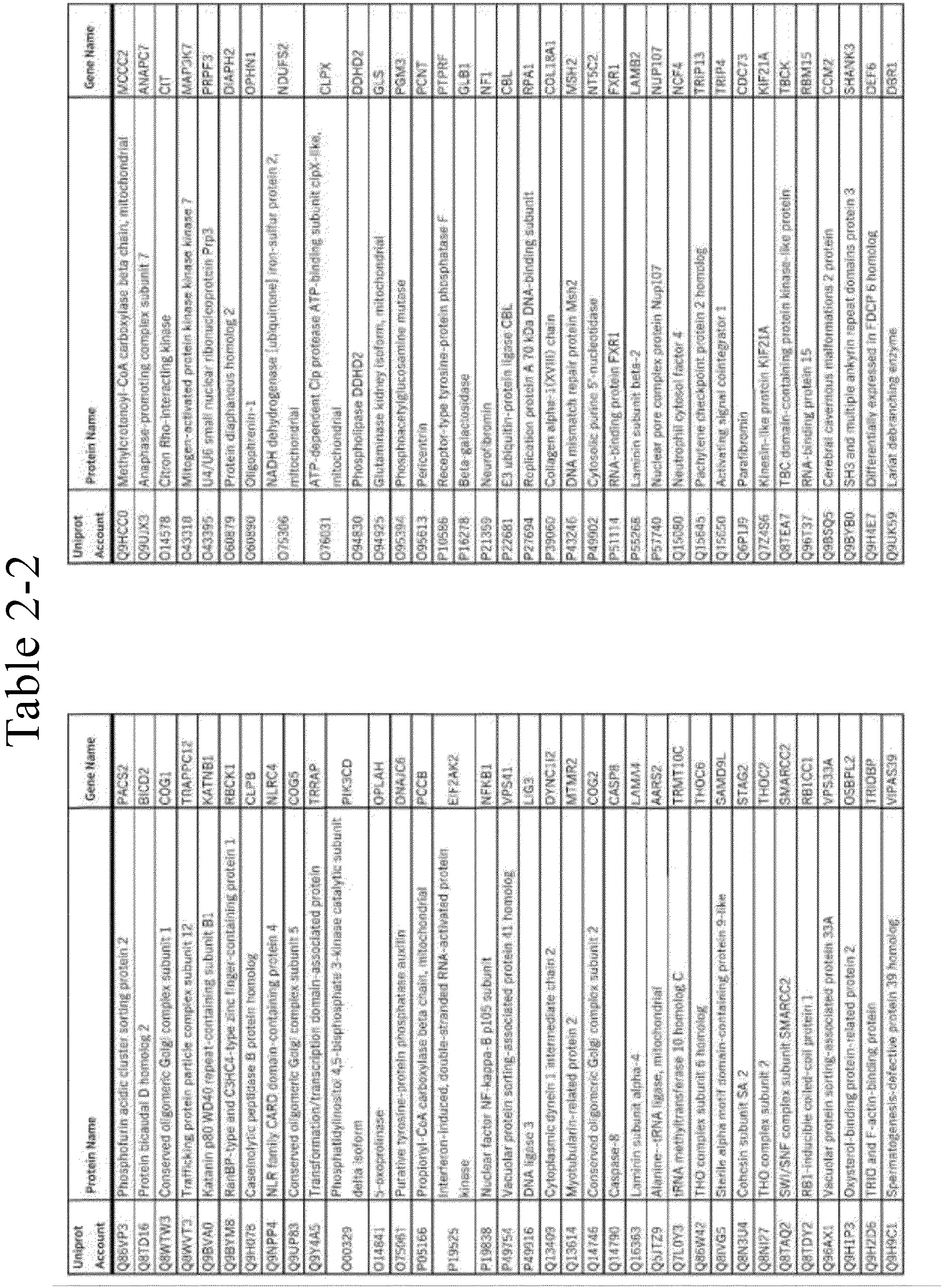

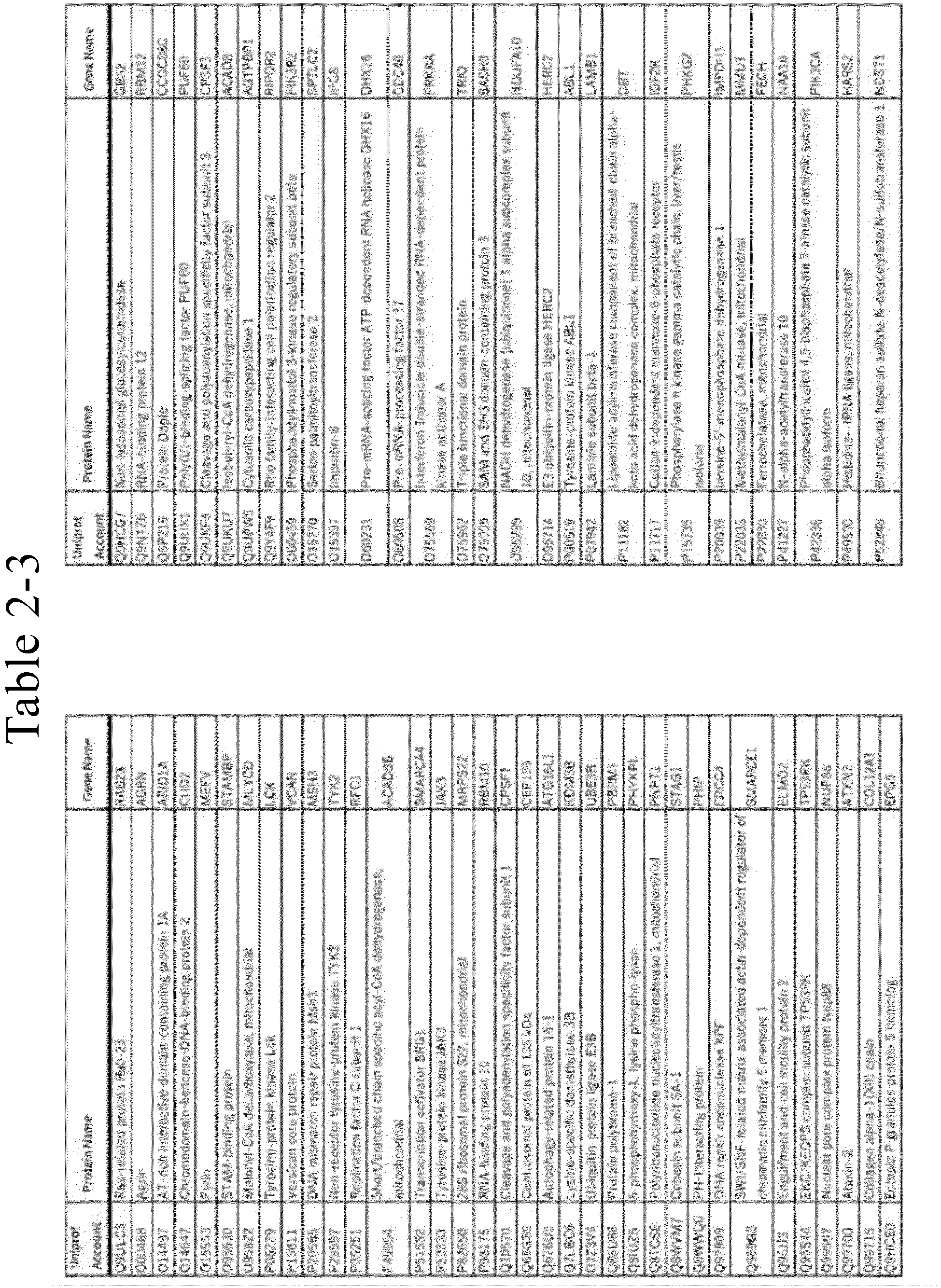

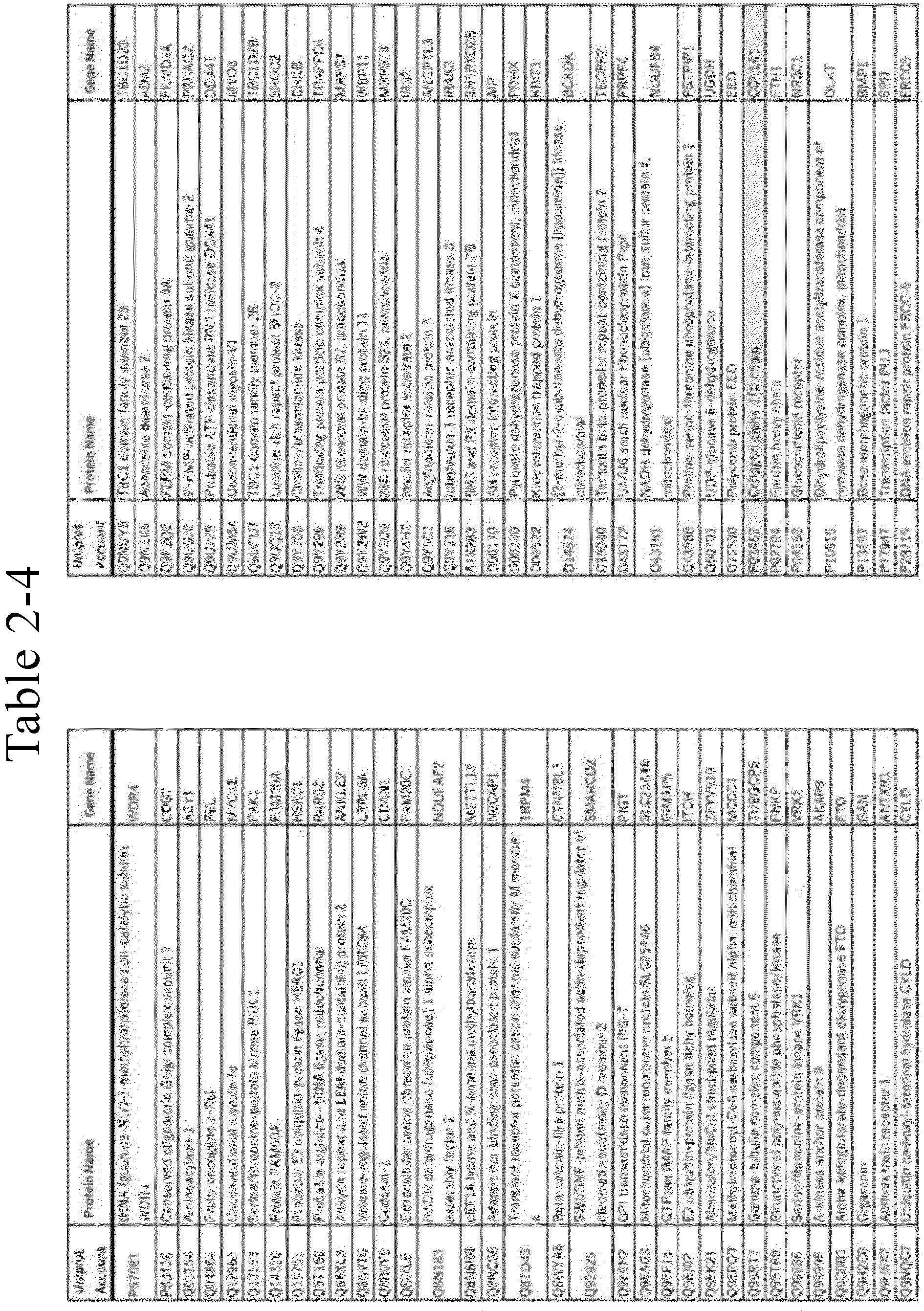

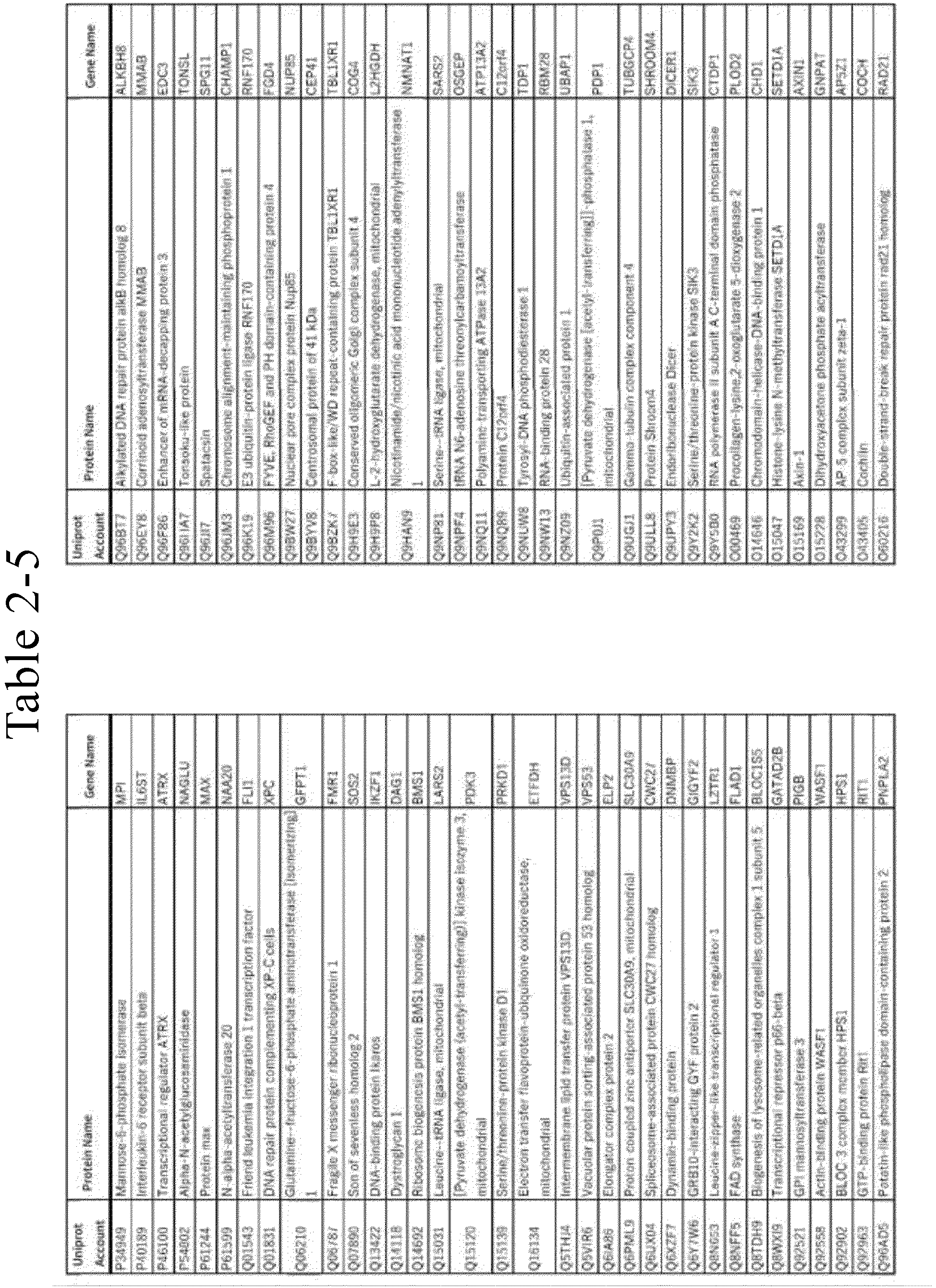

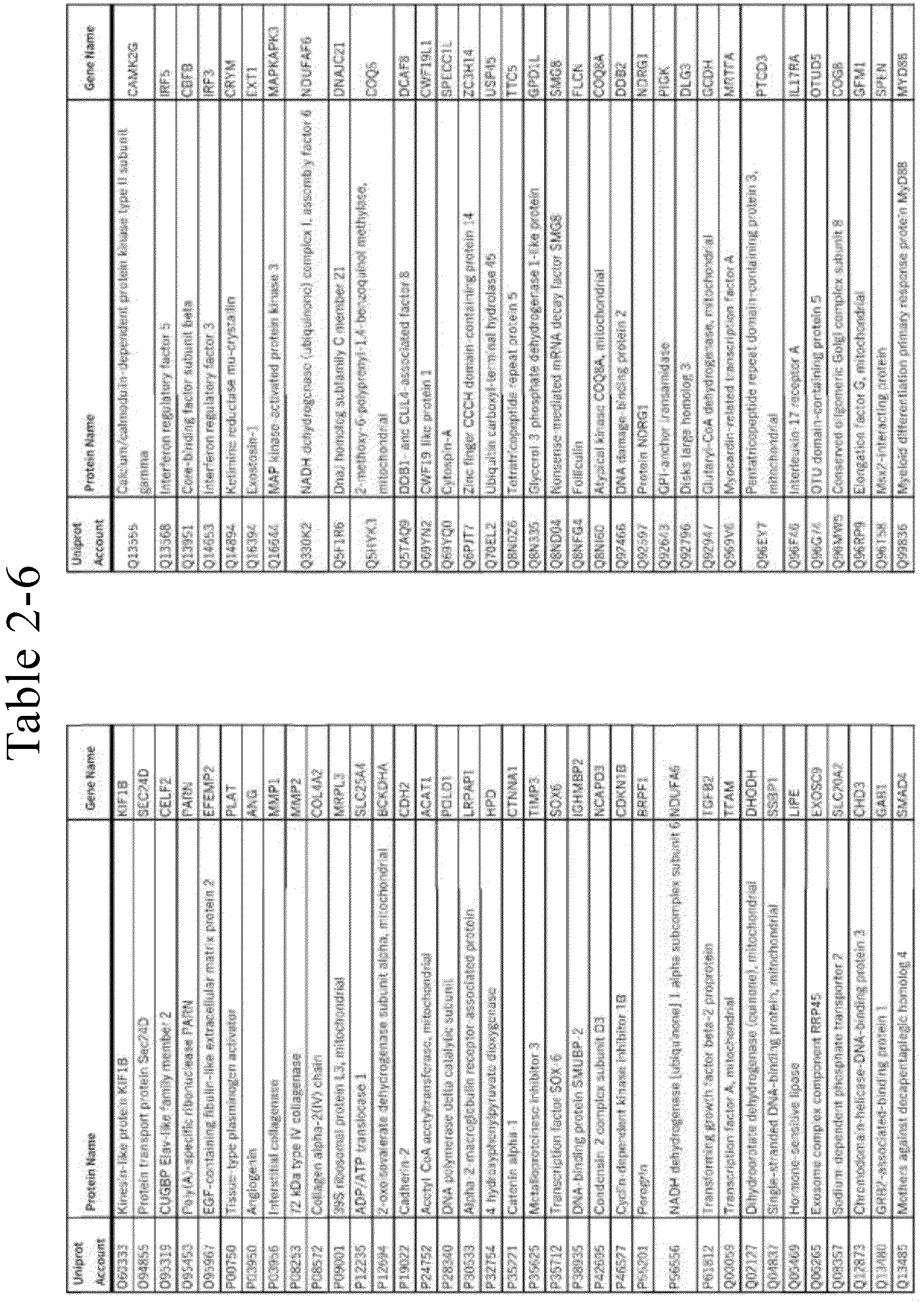

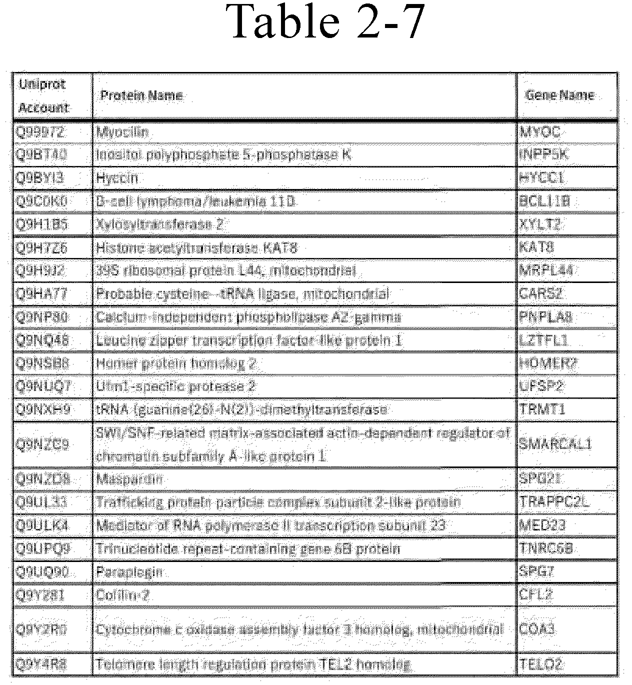

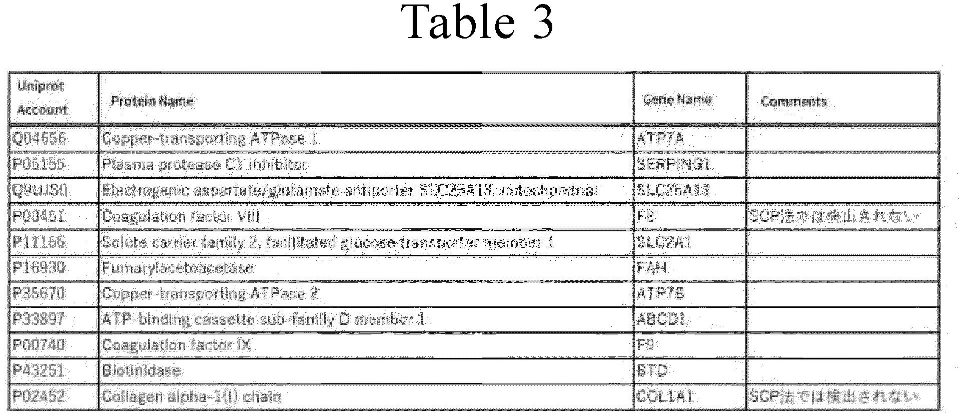

### [Example 2] Pretreatment of paper filter bound proteins for proteome analysis

The following samples and solutions were added to a deep-well 96-well plate for automated processing using the Maelstrom 8 Autostage (MS8) (manufactured by Taiwan Advanced Nanotech Inc.).
1^{st} Row: TBST (500 µl), iron powder (particle diameter 3-5 µm, 5 mg), blood-soaked dry filter paper (diameter 3.2 mm, 1.5 mg)
2^{nd} Row: TBST (500 µl)
3^{rd} Row: 50 mM Tris-HCl pH 8.0 (500 µl)
4^{th} Row: 50 mM Tris-HCl pH 8.0 (500 µl)
5^{th} Row: 50 mM Tris-HCl pH 8.0 (200 µl)

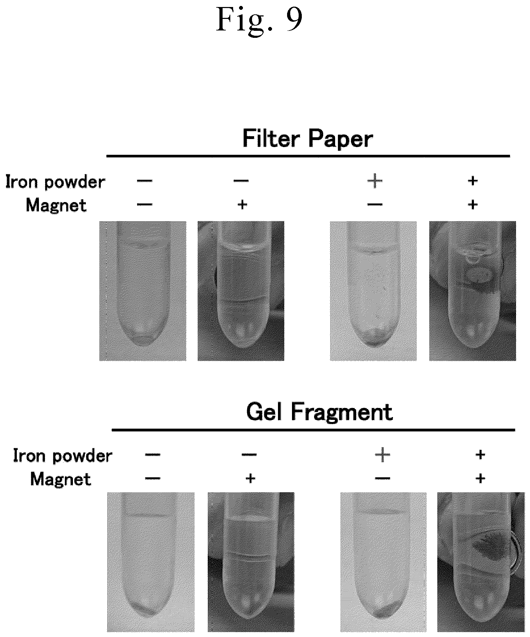

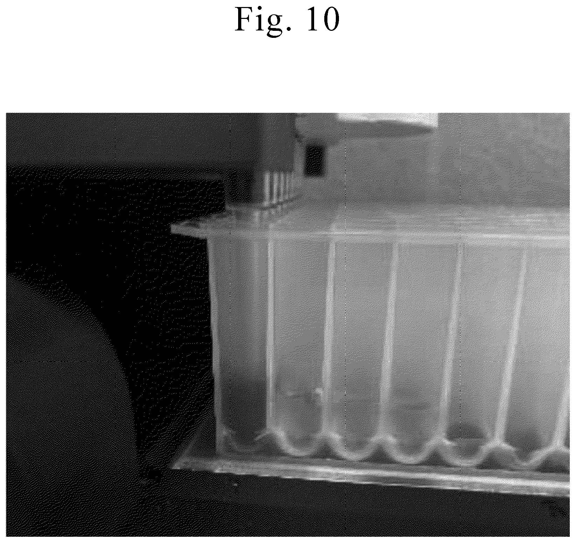

The 96-well plate was mounted on the autostage stand, and the pretreatment protocol shown on the right side of Figure 8 was carried out using the automated processing system of MS8. When iron powder and blood-soaked dry filter paper were added to TBST and agitated using the MS8, highly abundant components such as hemoglobin, albumin, and immunoglobulins, which are unnecessary for analysis, were removed into the TBST (Figure 9). The agitation caused the filter paper to swell and loosen its fibers, allowing iron powder to be incorporated into the gaps between the fibers. When the electromagnet of the MS8 was turned on, the filter paper having iron powder adhered was magnetically attracted to the electromagnets and separated from the liquid phase, moving to the next column along with the movement of the electromagnets (Figure 10). In contrast to conventional methods that required manual centrifugation and buffer exchange for repeated washing of filter paper (Figure 8, left), the addition of iron powder to the washing solution enabled automated pretreatment of filter paper using the MS8, which is designed for magnetic beads. Notably, the surface magnetic flux density of the electromagnets installed on the MS8 magnetic rods is 3000 gauss.

### [Experimental Example 3] Investigation of the adsorption and recovery efficiency of suspended filter paper

When using iron powder suspended in liquid phase (water) to collect filter paper in the liquid phase by magnetism, the number of filter paper sheets, the amount of iron powder added, and the particle diameter of the iron powder were varied, and the collection efficiency of the filter paper under each condition was evaluated. A cylindrical neodymium magnet (surface flux density (T) = 0.4 and attractive force (N) = 9.29) with a 5 mm diameter and a 5 mm height was used to collect the filter paper. The experimental conditions and evaluation results are summarized in Table 1.

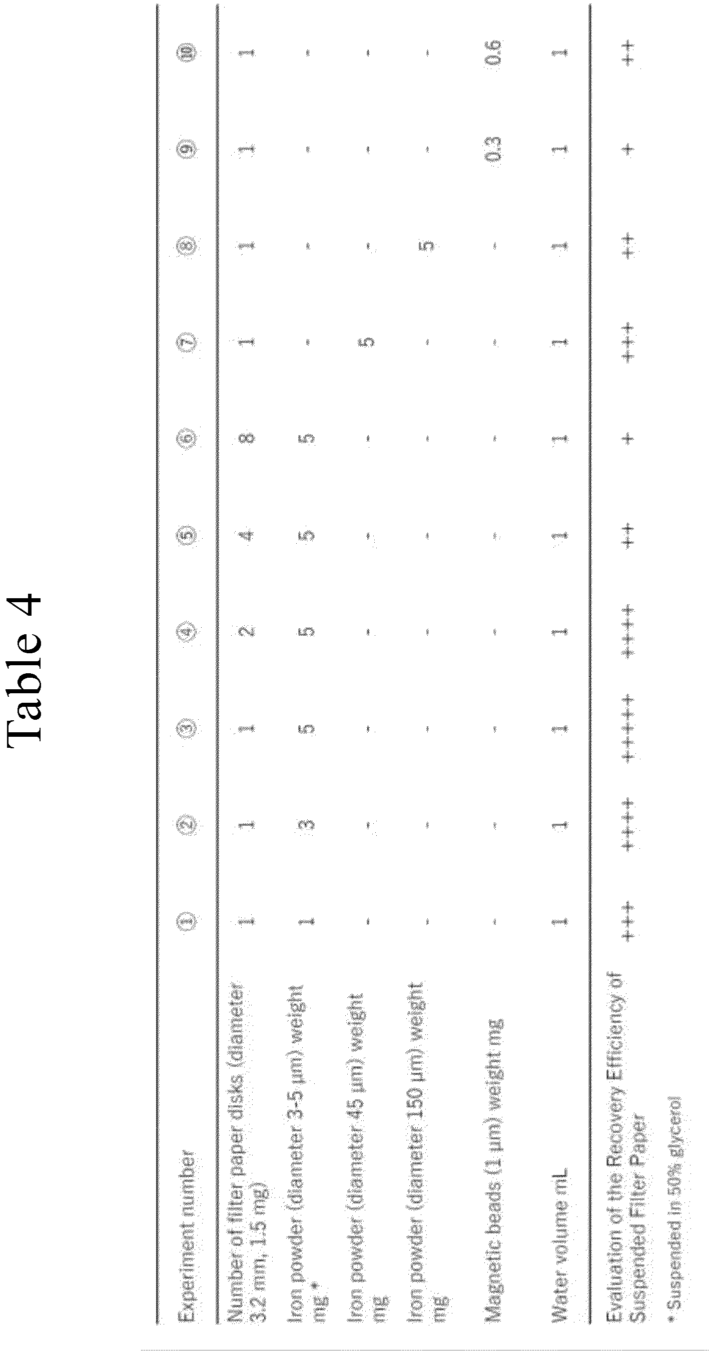

In experiments (1), (2), and (3), the amount of iron powder (magnetic material, 3-5 µm particle diameter) added to a single filter paper disc (non-magnetic material; 3.2 mm diameter, 1.5 mg) was varied as 1 mg, 3 mg, and 5 mg to evaluate how the adsorption of the filter paper having incorporated iron power to the magnet and its recovery efficiency changed. As a result, the filter paper could be recovered with the magnet under all tested conditions; however, the greater the amount of iron powder used, the higher the adsorption to the magnet and the recovery efficiency. At 5 mg, all filter paper discs were successfully recovered.

In experiments (3), (4), (5), and (6), the number of filter paper discs was increased to 1, 2, 4, and 8 while keeping the amount of iron powder constant at 5 mg, in order to evaluate how the adsorption to the magnet and recovery efficiency of the filter paper having incorporated iron powder would change. As a result, the adsorption and recovery efficiency of the filter paper decreased with the increase in the number of filter paper discs. These two sets of experiments indicate that a higher ratio of magnetic material to non-magnetic material leads to improved adsorption and recovery efficiency.

In experiments (3), (7), and (8), the particle diameter of the added iron powder was varied (3-5 µm, 45 µm, and 150 µm) while keeping the weight constant, to evaluate how changes in particle size affect the adsorption to and recovery efficiency of the filter paper having incorporated iron powder. As a result, it was observed that as the particle diameter increased, the sedimentation rate of the iron powder became faster, and the powder tended to accumulate at the bottom of the vessel rather than being uniformly adsorbed onto the filter paper. Therefore, it was suggested that magnetic materials with smaller particle diameters and slower sedimentation rates are more suitable for achieving uniform adsorption onto non-magnetic materials and improving recovery efficiency.

In experiments (9) and (10), instead of iron powder, carboxylate-modified magnetic beads (1 µm particle diameter, 0.3 or 0.6 mg) (Sera-Mag^{™} TMSpeedBeads and Sera-Mag^{™} Carboxylate-Modified Magnetic Particles, manufactured by Cytiva) were used to investigate whether filter paper could be adsorbed and recovered. As a result, it was found that the adsorption and recovery of the filter paper could also be successfully achieved using the carboxylate-modified magnetic beads.

Based on the results of these experiments, it was demonstrated that at least 0.3 mg/ml of magnetic particles (iron powder) for 1.5 mg of filter paper (at least 0.2 mg/ml of magnetic particles (iron powder) for 1.0 mg of non-magnetic carrier) is sufficient to enable magnetic adsorption and recovery of the filter paper.

Furthermore, when glycerol was added to the solution at a final concentration of 50% (v/v) during the suspension of iron powder, the sedimentation rate of the iron powder decreased, making sedimentation less likely. As a result, a more homogeneous suspension of iron powder could be obtained, thereby improving operability. The viscosity of a 50% (v/v) aqueous glycerol solution at 20°C is 6.00 mPa·s.

### INDUSTRIAL APPLICABILITY

According to the present invention, a peptide-containing sample in which the content of peptides derived from hydrophilic proteins such as albumin that are abundantly present in the biological sample is suppressed, and which is enriched in peptides derived from trace proteins can be prepared from a fiber aggregate having the biological sample adsorbed thereon in a simple manner by using commonly-used inexpensive reagents. By subjecting the peptide-containing sample obtained by the preparation method of the present invention to LC-MS analysis, it becomes possible to identify a large number of proteins including trace proteins.

According to the present invention, it becomes possible to separate a non-magnetic solid-phase carrier from a liquid in a magnetism-dependent manner. Therefore, the non-magnetic solid-phase carrier can be washed and recovered or removed without the use of centrifugation or the like. Conventionally, when performing washing, extraction, or other processing serially on a non-magnetic solid-phase carrier using a liquid, the non-magnetic solid-phase carrier is typically left inside a vessel and precipitated by centrifugation, followed by addition and removal of the liquid for washing or extraction. In such procedures, the precipitated non-magnetic solid-phase carrier may be unintentionally aspirated when the liquid is removed or recovered from the container, or the precipitate may float, resulting in loss of the non-magnetic solid-phase carrier. In contrast, in the method of the present invention, the non-magnetic solid-phase carrier is removed from the vessel while leaving the liquid for washing or extraction, rather than the non-magnetic solid-phase carrier, in the vessel. Therefore, the non-magnetic solid-phase carrier can be subjected to serial processing with a liquid such as washing or extraction while minimizing the loss of the non-magnetic solid-phase carrier. Since automated devices for handling magnetic solid-phase carriers such as magnetic beads are already widely available, combining the present invention with such devices enables automated processing of large amount of non-magnetic solid-phase carriers, which has been difficult to achieve by conventional means.

Furthermore, by applying this method for separating the non-magnetic solid-phase carrier using magnetic particles to the preparation of a peptide-containing sample from a fiber aggregate having the biological sample adsorbed thereon and allowing the magnetic particles to adhere to the fiber aggregate, operations such as washing, recovery, and buffer replacement of the fiber aggregate having the biological sample adsorbed thereon can be conveniently performed by magnetic adsorption. In addition, by employing automated processing using an automated nucleic acid extraction device and the like, a large number of samples can be processed in a short period of time, and the reproducibility of the results can be improved, thereby enabling application to mass screening for congenital diseases in newborns using dried blood spots.

This application is based on a patent application No. 2022-199020 (filing date: December 13, 2022) filed in Japan, the contents of which are incorporated in full herein.

## Claims

1. A method for preparing a peptide-containing sample for identifying, by mass spectrometry, proteins contained in a biological sample containing proteins from a fiber aggregate having the biological sample containing proteins adsorbed thereon, the method comprising the following steps:
1) washing the fiber aggregate having the biological sample containing proteins adsorbed thereon with an aqueous solvent to remove hydrophilic proteins contained in the biological sample from the fiber aggregate, and recovering the fiber aggregate having residual proteins bound thereto;
2) treating the recovered fiber aggregate having residual proteins bound thereto with a protease to digest the residual proteins bound to the fiber aggregate with the protease; and
3) removing the fiber aggregate from the digestion reaction mixture to recover a peptide mixture as a digestion product.

2. The preparation method according to claim 1, wherein the fiber aggregate is a cellulose fiber aggregate.

3. The preparation method according to claim 2, wherein the cellulose fiber aggregate is a filter paper.

4. The preparation method according to claim 1, wherein the fiber aggregate having the biological sample containing proteins adsorbed thereon is a dried blood spot.

5. The preparation method according to claim 1, further comprising subjecting the fiber aggregate having residual proteins bound thereto to a reductive alkylation treatment.

6. A method for separating non-magnetic solid-phase carriers from a liquid containing the non-magnetic solid-phase carriers, the method comprising the following steps:
1) providing a liquid containing magnetic particles and a non-magnetic solid-phase carrier;
2) agitating the liquid containing the magnetic particles and the non-magnetic solid-phase carriers to allow the magnetic particles to adhere to the non-magnetic solid-phase carrier;
3) bringing a magnet close to the liquid containing the magnetic particles and the non-magnetic solid-phase carriers to magnetically adsorb the non-magnetic solid-phase carrier to which the magnetic particles have adhered; and
4) separating the magnetically adsorbed non-magnetic solid-phase carrier from the liquid.

7. The method according to claim 6, wherein the non-magnetic solid-phase carrier has a porous structure, and the magnetic particles enter the voids in the porous structure by agitation.

8. The method of according to claim 7, wherein the non-magnetic solid-phase carrier comprises a fiber aggregate.

9. The method of according to claim 6, wherein the non-magnetic solid phase carrier is sheet-shaped.

10. The method according to claim 1, wherein the non-magnetic solid phase carriers are any one selected from the group consisting of paper, gel, fabric, resin, cotton and swab.

11. The method according to claim 6, wherein the liquid contains a thickening agent.

12. The method according to claim 6, wherein the magnetic particles are ferromagnetic metal particles or ferromagnetic metal oxide particles.

13. The method of according to claim 12, wherein the magnetic particles are an iron powder.

14. The method according to claim 6, wherein the average particle diameter of the magnetic particles is 150 µm or less.

15. The method according to claim 6, wherein 0.3 mg or more of magnetic particles are dispersed per 1 ml of liquid.

16. The method according to claim 6, wherein 0.2 mg or more of magnetic particles are dispersed in a liquid for 1 mg of non-magnetic solid phase carriers.

17. The preparation method according to claim 1, further comprising agitating the magnetic particles and the fiber aggregate having the biological sample containing protein adsorbed thereon in an aqueous solvent to allow the magnetic particles to adhere to the fibrous aggregate.

18. The preparation method according to claim 17, wherein the aqueous solvent used in Step 1 comprises magnetic particles, and Step 1 is performed by agitating, in the aqueous solvent containing the magnetic particles, the fiber aggregate having the biological sample containing proteins adsorbed thereon to wash the fiber aggregate with the aqueous solvent to remove hydrophilic proteins contained in the biological sample from the fiber aggregate and allow the magnetic particles to adhere to the fiber aggregate, thereby recovering a fiber aggregate having residual proteins bound thereto and the magnetic particles adhered thereto.

19. The preparation method according to claim 17, wherein the fiber aggregate having residual proteins bound thereto is recovered by magnetic adsorption in Step 1.

20. The preparation method according to claim 17, wherein the fiber aggregate is removed from the digestion reaction mixture by magnetic adsorption in Step 3.

21. The preparation method according to claim 17, wherein the magnetic particles are iron powder.

22. The preparation method according to claim 17, wherein the aqueous solvent comprises a thickening agent.

23. The preparation method according to claim 22, wherein the thickening agent is glycerol.
